(19) Europäisches Patentamt / European Patent Office / Office européen des brevets

(11) **EP 3 692 537 B1**

(12) # EUROPEAN PATENT SPECIFICATION

(45) Date of publication and mention of the grant of the patent:
**19.01.2022 Bulletin 2022/03**

(21) Application number: **18773486.8**

(22) Date of filing: **28.09.2018**

(51) International Patent Classification (IPC):
***G16B 30/00*** *(2019.01)* ***C12Q 1/6886*** *(2018.01)*
***G01N 33/68*** *(2006.01)*

(52) Cooperative Patent Classification (CPC):
**C12Q 1/6876; C12Q 1/6886; G01N 33/68; G16B 5/00;** C12Q 2600/158; G01N 2800/50; G01N 2800/52; G01N 2800/56; G16B 25/10; G16B 40/00

(86) International application number:
**PCT/EP2018/076488**

(87) International publication number:
**WO 2019/068585 (11.04.2019 Gazette 2019/15)**

(54) **ASSESSMENT OF NOTCH CELLULAR SIGNALING PATHWAY ACTIVITY USING MATHEMATICAL MODELLING OF TARGET GENE EXPRESSION**

BEURTEILUNG DER ZELLULÄREN NOTCH-SIGNALISIERUNGSPFADAKTIVITÄT MITTELS MATHEMATISCHER MODELLIERUNG DER ZIELGENEXPRESSION

ÉVALUATION DE L'ACTIVITÉ DE LA VOIE DE SIGNALISATION CELLULAIRE DE NOTCH À L'AIDE DE LA MODÉLISATION MATHÉMATIQUE DE L'EXPRESSION GÉNIQUE CIBLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.10.2017 EP 17194288**

(43) Date of publication of application:
**12.08.2020 Bulletin 2020/33**

(73) Proprietor: **Koninklijke Philips N.V.**
**5656 AE Eindhoven (NL)**

(72) Inventors:
- **VAN DE STOLPE, Anja**
  **5656 AE Eindhoven (NL)**
- **HOLTZER, Laurentius, Henricus, Franciscus, Maria**
  **5656 AE Eindhoven (NL)**
- **VERHAEGH, Wilhelmus, Franciscus, Johannes**
  **5656 AE Eindhoven (NL)**

(74) Representative: **Philips Intellectual Property & Standards**
**High Tech Campus 52**
**5656 AG Eindhoven (NL)**

(56) References cited:
**WO-A1-2016/062891 WO-A2-2013/011479**
**KR-A- 20160 086 775 US-A1- 2016 117 439**
**US-A1- 2016 125 127 US-A1- 2016 266 095**
**US-B1- 7 544 476**

- **S. S. RAO ET AL: "Inhibition of NOTCH Signaling by Gamma Secretase Inhibitor Engages the RB Pathway and Elicits Cell Cycle Exit in T-Cell Acute Lymphoblastic Leukemia Cells", CANCER RESEARCH, vol. 69, no. 7, 24 March 2009 (2009-03-24) , pages 3060-3068, XP055075643, ISSN: 0008-5472, DOI: 10.1158/0008-5472.CAN-08-4295**
- **WEIWEI LIU ET AL: "Blockage of Notch Signaling Inhibits the Migration and Proliferation of Retinal Pigment Epithelial Cells", THE SCIENTIFIC WORLD JOURNAL, vol. 2013, 1 January 2013 (2013-01-01), pages 1-6, XP055522472, DOI: 10.1155/2013/178708**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

SEQUENCE LISTING

<110> Koninklijke Philips N. V.

<120> Assessment of Notch cellular signaling pathway activity using mathematical modelling of target gene expression

<130> 2016PF01362

<160> 26

<170> PatentIn version 3.5

<210> 1
<211> 4900
<212> DNA
<213> Homo sapiens

<400> 1

```
taaagtcatc aaaacaacgt tatatcctgt gtgaaatgct gcagtcagga tgccttgtgg      60
tttgagtgcc ttgatcatgt gccctaaggg gatggtggcg gtggtggtgg ccgtggatga     120
cggagactct caggccttgg caggtgcgtc tttcagttcc cctcacactt cgggttcctc     180
ggggaggagg ggctggaacc ctagcccatc gtcaggacaa agatgctcag gctgctcttg     240
gctctcaact tattcccttc aattcaagta acaggaaaca agattttggt gaagcagtcg     300
cccatgcttg tagcgtacga caatgcggtc aaccttagct gcaagtattc ctacaatctc     360
ttctcaaggg agttccgggc atcccttcac aaaggactgg atagtgctgt ggaagtctgt     420
gttgtatatg ggaattactc ccagcagctt caggtttact caaaaacggg gttcaactgt     480
gatgggaaat tgggcaatga atcagtgaca ttctacctcc agaatttgta tgttaaccaa     540
acagatattt acttctgcaa aattgaagtt atgtatcctc ctccttacct agacaatgag     600
aagagcaatg gaaccattat ccatgtgaaa gggaaacacc tttgtccaag tcccctattt     660
cccggacctt ctaagccctt ttgggtgctg gtggtggttg gtggagtcct ggcttgctat     720
agcttgctag taacagtggc ctttattatt ttctgggtga ggagtaagag gagcaggctc     780
ctgcacagtg actacatgaa catgactccc cgccgccccg ggcccacccg caagcattac     840
cagccctatg ccccaccacg cgacttcgca gcctatcgct cctgacacgg acgcctatcc     900
agaagccagc cggctggcag cccccatctg ctcaatatca ctgctctgga taggaaatga     960
ccgccatctc cagccggcca cctcaggccc ctgttgggcc accaatgcca atttttctcg    1020
agtgactaga ccaaatatca agatcatttt gagactctga aatgaagtaa aagagatttc    1080
ctgtgacagg ccaagtctta cagtgccatg gcccacattc caacttacca tgtacttagt    1140
gacttgactg agaagttagg gtagaaaaca aaaagggagt ggattctggg agcctcttcc    1200
ctttctcact cacctgcaca tctcagtcaa gcaaagtgtg gtatccacag acattttagt    1260
tgcagaagaa aggctaggaa atcattcctt ttggttaaat gggtgtttaa tcttttggtt    1320
```

agtgggttaa acggggtaag ttagagtagg gggagggata ggaagacata tttaaaaacc 1380

attaaaacac tgtctcccac tcatgaaatg agccacgtag ttcctattta atgctgtttt 1440

cctttagttt agaaatacat agacattgtc ttttatgaat tctgatcata tttagtcatt 1500

ttgaccaaat gagggatttg gtcaaatgag ggattccctc aaagcaatat caggtaaacc 1560

aagttgcttt cctcactccc tgtcatgaga cttcagtgtt aatgttcaca atatactttc 1620

gaaagaataa aatagttctc ctacatgaag aaagaatatg tcaggaaata aggtcacttt 1680

atgtcaaaat tatttgagta ctatgggacc tggcgcagtg gctcatgctt gtaatcccag 1740

cactttggga ggccgaggtg ggcagatcac ttgagatcag gaccagcctg gtcaagatgg 1800

tgaaactccg tctgtactaa aaatacaaaa tttagcttgg cctggtggca ggcacctgta 1860

atcccagctg cccaagaggc tgaggcatga gaatcgcttg aacctggcag gcggaggttg 1920

cagtgagccg agatagtgcc acagctctcc agcctgggcg acagagtgag actccatctc 1980

aaacaacaac aacaacaaca acaacaacaa caaaccacaa aattatttga gtactgtgaa 2040

ggattatttg tctaacagtt cattccaatc agaccaggta ggagctttcc tgtttcatat 2100

gtttcagggt tgcacagttg gtctctttaa tgtcggtgtg gagatccaaa gtgggttgtg 2160

gaaagagcgt ccataggaga agtgagaata ctgtgaaaaa gggatgttag cattcattag 2220

agtatgagga tgagtcccaa gaaggttctt tggaaggagg acgaatagaa tggagtaatg 2280

aaattcttgc catgtgctga ggagatagcc agcattaggt gacaatcttc cagaagtggt 2340

caggcagaag gtgccctggt gagagctcct ttacagggac tttatgtggt ttagggctca 2400

gagctccaaa actctgggct cagctgctcc tgtaccttgg aggtccattc acatgggaaa 2460

gtattttgga atgtgtcttt tgaagagagc atcagagttc ttaagggact gggtaaggcc 2520

tgaccctgaa atgaccatgg atatttttct acctacagtt tgagtcaact agaatatgcc 2580

tggggacctt gaagaatggc ccttcagtgg ccctcaccat ttgttcatgc ttcagttaat 2640

tcaggtgttg aaggagctta ggttttagag gcacgtagac ttggttcaag tctcgttagt 2700

agttgaatag cctcaggcaa gtcactgccc acctaagatg atggttcttc aactataaaa 2760

tggagataat ggttacaaat gtctcttcct atagtataat ctccataagg gcatggccca 2820

agtctgtctt tgactctgcc tatccctgac atttagtagc atgcccgaca tacaatgtta 2880

gctattggta ttattgccat atagataaat tatgtataaa aattaaactg ggcaatagcc 2940

taagaagggg ggaatattgt aacacaaatt taaacccact acgcagggat gaggtgctat 3000

aatatgagga ccttttaact tccatcattt tcctgtttct tgaaatagtt tatcttgtaa 3060

tgaaatataa ggcacctccc acttttatgt atagaaagag gtcttttaat tttttttaa 3120

tgtgagaagg aagggaggag taggaatctt gagattccag atcgaaaata ctgtactttg 3180

gttgattttt aagtgggctt ccattccatg gatttaatca gtcccaagaa gatcaaactc 3240

agcagtactt gggtgctgaa gaactgttgg atttaccctg gcacgtgtgc cacttgccag 3300
cttcttgggc acacagagtt cttcaatcca agttatcaga ttgtatttga aaatgacaga 3360
gctggagagt tttttgaaat ggcagtggca aataaataaa tacttttttt taaatggaaa 3420
gacttgatct atggtaataa atgattttgt tttctgactg gaaaaatagg cctactaaag 3480
atgaatcaca cttgagatgt ttcttactca ctctgcacag aaacaaagaa gaaatgttat 3540
acagggaagt ccgttttcac tattagtatg aaccaagaaa tggttcaaaa acagtggtag 3600
gagcaatgct ttcatagttt cagatatggt agttatgaag aaaacaatgt catttgctgc 3660
tattattgta agagtcttat aattaatggt actcctataa tttttgattg tgagctcacc 3720
tatttgggtt aagcatgcca atttaaagag accaagtgta tgtacattat gttctacata 3780
ttcagtgata aaattactaa actactatat gtctgcttta aatttgtact ttaatattgt 3840
cttttggtat taagaaagat atgctttcag aatagatatg cttcgctttg gcaaggaatt 3900
tggatagaac ttgctattta aaagaggtgt ggggtaaatc cttgtataaa tctccagttt 3960
agcctttttt gaaaaagcta gactttcaaa tactaatttc acttcaagca gggtacgttt 4020
ctggtttgtt tgcttgactt cagtcacaat ttcttatcag accaatggct gacctctttg 4080
agatgtcagg ctaggcttac ctatgtgttc tgtgtcatgt gaatgctgag aagtttgaca 4140
gagatccaac ttcagccttg accccatcag tccctcgggt taactaactg agccaccggt 4200
cctcatggct attttaatga gggtattgat ggttaaatgc atgtctgatc ccttatccca 4260
gccatttgca ctgccagctg ggaactatac cagacctgga tactgatccc aaagtgttaa 4320
attcaactac atgctggaga ttagagatgg tgccaataaa ggacccagaa ccaggatctt 4380
gattgctata gacttattaa taatccaggt caaagagagt gacacacact ctctcaagac 4440
ctggggtgag ggagtctgtg ttatctgcaa ggccatttga ggctcagaaa gtctctcttt 4500
cctatagata tatgcatact ttctgacata taggaatgta tcaggaatac tcaaccatca 4560
caggcatgtt cctacctcag ggcctttaca tgtcctgttt actctgtcta gaatgtcctt 4620
ctgtagatga cctggcttgc ctcgtcaccc ttcaggtcct tgctcaagtg tcatcttctc 4680
ccctagttaa actaccccac accctgtctg ctttccttgc ttatttttct ccatagcatt 4740
ttaccatctc ttacattaga cattttctt atttatttgt agtttataag cttcatgagg 4800
caagtaactt tgctttgttt cttgctgtat ctccagtgcc cagagcagtg cctggtatat 4860
aataaatatt tattgactga gtgaaaaaaa aaaaaaaaaa 4900

<210> 2
<211> 5748
<212> DNA
<213> Homo sapiens

<400> 2

```
gagaagaaag ccagtgcgtc tctgggcgca ggggccagtg ggctcggag gcacaggcac      60
cccgcgacac tccaggttcc ccgacccacg tccctggcag ccccgattat ttacagcctc     120
agcagagcac ggggcggggg cagaggggcc cgcccgggag ggctgctact tcttaaaacc     180
tctgcgggct gcttagtcac agcccccctt gcttgggtgt gtccttcgct cgctccctcc     240
ctccgtctta ggtcactgtt ttcaacctcg aataaaaact gcagccaact tccgaggcag     300
cctcattgcc cagcggaccc cagcctctgc caggttcggt ccgccatcct cgtcccgtcc     360
tccgccggcc cctgccccgc gcccagggat cctccagctc ctttcgcccg cgccctccgt     420
tcgctccgga caccatggac aagttttggt ggcacgcagc ctggggactc tgcctcgtgc     480
cgctgagcct ggcgcagatc gatttgaata taacctgccg ctttgcaggt gtattccacg     540
tggagaaaaa tggtcgctac agcatctctc ggacggaggc cgctgacctc tgcaaggctt     600
tcaatagcac cttgcccaca atggcccaga tggagaaagc tctgagcatc ggatttgaga     660
cctgcaggta tgggttcata gaagggcacg tggtgattcc ccggatccac cccaactcca     720
tctgtgcagc aaacaacaca ggggtgtaca tcctcacatc caacacctcc cagtatgaca     780
catattgctt caatgcttca gctccacctg aagaagattg tacatcagtc acagacctgc     840
ccaatgcctt tgatggacca attaccataa ctattgttaa ccgtgatggc acccgctatg     900
tccagaaagg agaatacaga acgaatcctg aagacatcta ccccagcaac cctactgatg     960
atgacgtgag cagcggctcc tccagtgaaa ggagcagcac ttcaggaggt tacatctttt    1020
acacctttc tactgtacac cccatcccag acgaagacag tccctggatc accgacagca     1080
cagacagaat ccctgctacc actttgatga gcactagtgc tacagcaact gagacagcaa    1140
ccaagaggca agaaacctgg gattggtttt catggttgtt tctaccatca gagtcaaaga    1200
atcatcttca cacaacaaca caaatggctg gtacgtcttc aaataccatc tcagcaggct    1260
gggagccaaa tgaagaaaat gaagatgaaa gagacagaca cctcagtttt tctggatcag    1320
gcattgatga tgatgaagat tttatctcca gcaccatttc aaccacacca cgggcttttg    1380
accacacaaa acagaaccag gactggaccc agtggaaccc aagccattca aatccggaag    1440
tgctacttca gacaaccaca aggatgactg atgtagacag aaatggcacc actgcttatg    1500
aaggaaactg gaacccagaa gcacaccctc ccctcattca ccatgagcat catgaggaag    1560
aagagacccc acattctaca agcacaatcc aggcaactcc tagtagtaca acggaagaaa    1620
cagctaccca gaaggaacag tggtttggca acagatggca tgagggatat cgccaaacac    1680
ccaaagaaga ctcccattcg acaacaggga cagctgcagc ctcagctcat accagccatc    1740
caatgcaagg aaggacaaca ccaagcccag aggacagttc ctggactgat ttcttcaacc    1800
caatctcaca ccccatggga cgaggtcatc aagcaggaag aaggatggat atggactcca    1860
```

gtcatagtat aacgcttcag cctactgcaa atccaaacac aggtttggtg gaagatttgg 1920

acaggacagg acctctttca atgacaacgc agcagagtaa ttctcagagc ttctctacat 1980

cacatgaagg cttggaagaa gataaagacc atccaacaac ttctactctg acatcaagca 2040

ataggaatga tgtcacaggt ggaagaagag acccaaatca ttctgaaggc tcaactactt 2100

tactggaagg ttatacctct cattacccac acacgaagga aagcaggacc ttcatcccag 2160

tgacctcagc taagactggg tcctttggag ttactgcagt tactgttgga gattccaact 2220

ctaatgtcaa tcgttcctta tcaggagacc aagacacatt ccaccccagt gggggggtccc 2280

ataccactca tggatctgaa tcagatggac actcacatgg gagtcaagaa ggtggagcaa 2340

acacaacctc tggtcctata aggacacccc aaattccaga atggctgatc atcttggcat 2400

ccctcttggc cttggctttg attcttgcag tttgcattgc agtcaacagt cgaagaaggt 2460

gtgggcagaa gaaaaagcta gtgatcaaca gtggcaatgg agctgtggag gacagaaagc 2520

caagtggact caacggagag gccagcaagt ctcaggaaat ggtgcatttg gtgaacaagg 2580

agtcgtcaga aactccagac cagtttatga cagctgatga gacaaggaac ctgcagaatg 2640

tggacatgaa gattggggtg taacacctac accattatct tggaaagaaa caaccgttgg 2700

aaacataacc attacaggga gctgggacac ttaacagatg caatgtgcta ctgattgttt 2760

cattgcgaat ctttttttagc ataaaatttt ctactctttt tgttttttgt gttttgttct 2820

ttaaagtcag gtccaatttg taaaaacagc attgctttct gaaattaggg cccaattaat 2880

aatcagcaag aatttgatcg ttccagttcc cacttggagg cctttcatcc ctcgggtgtg 2940

ctatggatgg cttctaacaa aaactacaca tatgtattcc tgatcgccaa cctttccccc 3000

accagctaag gacatttccc agggttaata gggcctggtc cctgggagga aatttgaatg 3060

ggtccatttt gcccttccat agcctaatcc ctgggcattg ctttccactg aggttggggg 3120

ttggggtgta ctagttacac atcttcaaca gaccccctct agaaattttt cagatgcttc 3180

tgggagacac ccaaagggtg aagctattta tctgtagtaa actatttatc tgtgtttttg 3240

aaatattaaa ccctggatca gtcctttgat cagtataatt ttttaaagtt actttgtcag 3300

aggcacaaaa gggtttaaac tgattcataa taaatatctg tacttcttcg atcttcacct 3360

tttgtgctgt gattcttcag tttctaaacc agcactgtct gggtccctac aatgtatcag 3420

gaagagctga gaatggtaag gagactcttc taagtcttca tctcagagac cctgagttcc 3480

cactcagacc cactcagcca aatctcatgg aagaccaagg agggcagcac tgtttttgtt 3540

ttttgttttt tgtttttttt ttttgacact gtccaaaggt tttccatcct gtcctggaat 3600

cagagttgga agctgaggag cttcagcctc ttttatggtt taatggccac ctgttctctc 3660

ctgtgaaagg ctttgcaaag tcacattaag tttgcatgac ctgttatccc tggggcccta 3720

tttcatagag gctggcccta ttagtgattt ccaaaaacaa tatggaagtg ccttttgatg 3780

tcttacaata agagaagaag ccaatggaaa tgaaagagat tggcaaaggg gaaggatgat 3840
gccatgtaga tcctgtttga catttttatg gctgtatttg taaacttaaa cacaccagtg 3900
tctgttcttg atgcagttgc tatttaggat gagttaagtg cctggggagt ccctcaaaag 3960
gttaaaggga ttcccatcat tggaatctta tcaccagata ggcaagttta tgaccaaaca 4020
agagagtact ggctttatcc tctaacctca tattttctcc cacttggcaa gtcctttgtg 4080
gcatttattc atcagtcagg gtgtccgatt ggtcctagaa cttccaaagg ctgcttgtca 4140
tagaagccat tgcatctata aagcaacggc tcctgttaaa tggtatctcc tttctgaggc 4200
tcctactaaa agtcatttgt tacctaaact tatgtgctta acaggcaatg cttctcagac 4260
cacaaagcag aaagaagaag aaaagctcct gactaaatca gggctgggct tagacagagt 4320
tgatctgtag aatatcttta aaggagagat gtcaactttc tgcactattc ccagcctctg 4380
ctcctccctg tctaccctct cccctccctc tctccctcca cttcacccca caatcttgaa 4440
aaacttcctt tctcttctgt gaacatcatt ggccagatcc attttcagtg gtctggattt 4500
ctttttattt tcttttcaac ttgaaagaaa ctggacatta ggccactatg tgttgttact 4560
gccactagtg ttcaagtgcc tcttgttttc ccagagattt cctgggtctg ccagaggccc 4620
agacaggctc actcaagctc tttaactgaa aagcaacaag ccactccagg acaaggttca 4680
aaatggttac aacagcctct acctgtcgcc ccagggagaa aggggtagtg atacaagtct 4740
catagccaga gatggttttc cactccttct agatattccc aaaaagaggc tgagacagga 4800
ggttattttc aattttattt tggaattaaa tactttttc cctttattac tgttgtagtc 4860
cctcacttgg atatacctct gttttcacga tagaaataag ggaggtctag agcttctatt 4920
ccttggccat tgtcaacgga gagctggcca agtcttcaca aacccttgca acattgcctg 4980
aagtttatgg aataagatgt attctcactc ccttgatctc aagggcgtaa ctctggaagc 5040
acagcttgac tacacgtcat ttttaccaat gattttcagg tgacctgggc taagtcattt 5100
aaactgggtc tttataaaag taaaaggcca acatttaatt attttgcaaa gcaacctaag 5160
agctaaagat gtaatttttc ttgcaattgt aaatcttttg tgtctcctga agacttccct 5220
taaaattagc tctgagtgaa aaatcaaaag agacaaaaga catcttcgaa tccatatttc 5280
aagcctggta gaattggctt ttctagcaga acctttccaa aagttttata ttgagattca 5340
taacaacacc aagaattgat tttgtagcca acattcattc aatactgtta tatcagagga 5400
gtaggagaga ggaaacattt gacttatctg gaaaagcaaa atgtacttaa gaataagaat 5460
aacatggtcc attcaccttt atgttataga tatgtctttg tgtaaatcat ttgttttgag 5520
ttttcaaaga atagcccatt gttcattctt gtgctgtaca atgaccactg ttattgttac 5580
tttgactttt cagagcacac ccttcctctg gtttttgtat atttattgat ggatcaataa 5640

**taatgaggaa agcatgatat gtatattgct gagttgaaag cacttattgg aaaatattaa 5700**

**aaggctaaca ttaaaagact aaaggaaaca gaaaaaaaaa aaaaaaaa 5748**

<210> 3
<211> 2993
<212> DNA
<213> Homo sapiens

<400> 3

```
agcaaaccaa tcgcaagcct cgttgagtgg aaggggtggg atcttccccg gaagtgttgg      60
ttaaagcccc tccaatcagc ggctcggtgc ggcaagtttg aatttcgtgg aggctcgggt     120
tgtgagggtt cctgcttcgg agtcggcggt ggtcgtccag accgagtgtt ctttactttt     180
tgtttggttg aggtttcacg ctagaaggtg gctcaggatg tcttcatcac attttgccag     240
tcgacacagg aaggatataa gtactgaaat gattagaact aaaattgctc ataggaaatc     300
actgtctcag aaagaaaata gacataagga atacgaacga aatagacact ttggtttgaa     360
agatgtaaac attccaacct tggaaggtag aattcttgtt gaattagatg agacatctca     420
agggcttgtt ccagaaaaga ccaatgttaa gccaagggca atgaaaacta ttctaggtga     480
tcaacgaaaa cagatgctcc aaaaatacaa agaagaaaag caacttcaaa aattgaaaga     540
gcagagagag aaagctaaac gaggaatatt taaagtgggt cgttatagac ctgatatgcc     600
ttgttttctt ttatcaaacc agaatgctgt gaaagctgag ccaaaaaagg ctattccatc     660
ttctgtacgg attacaaggt caaaggccaa agaccaaatg gagcagacta agattgataa     720
cgagagtgat gttcgagcaa tccgacctgg tccaagacaa acttctgaaa agaaagtgtc     780
agacaaagag aaaaaagttg tgcagcctgt aatgcccacg tcgttgagaa tgactcgatc     840
agctactcaa gcagcaaagc aggttcccag aacagtctca tctaccacag caagaaagcc     900
agtcacaaga gctgctaatg aaaacgaacc agaaggaaag gtgccaagta aaggaagacc     960
tgccaaaaat gtagaaacaa aacccgacaa gggtatttct tgtaaagtcg atagtgaaga    1020
aaatactttg aattcacaaa ctaatgcaac aagtggaatg aatccagatg gagtcttatc    1080
aaaaatggaa aacttacctg agataaatac tgcaaaaata aaagggaaga attcctttgc    1140
acctaaggat tttatgtttc agccactgga tggtctgaag acctatcaag taacacctat    1200
gactcccaga agtgccaatg ctttttttgac acccagttac acctggactc ctttaaaaac    1260
agaagttgat gagtctcaag caacaaaaga aattttggca caaaaatgta aaacttactc    1320
taccaagaca atacagcaag attcaaataa attgccatgt cctttgggtc ctctaactgt    1380
ttggcatgaa gaacatgttt taaataaaaa tgaagctact actaaaaatt taaatggcct    1440
tccaataaaa gaagtcccat cacttgaaag aaatgaaggt cgaattgctc agccccacca    1500
tggtgtgcca tatttcagaa atatcctcca gtcagaaact gagaaattaa cttcacattg    1560
```

cttcgagtgg gacaggaaac ttgaattgga cattccagat gatgctaaag atcttattcg 1620
cacagcagtt ggtcaaacaa gactccttat gaaggaaagg tttaaacagt ttgaaggact 1680
ggttgatgat tgtgaatata aacgaggtat aaaggagact acctgtacag atctggatgg 1740
attttgggat atggttagtt ttcagataga agatgtaatc cacaaattca acaatctgat 1800
caaacttgag gaatctgggt ggcaagtcaa taataatatg aatcataata tgaacaaaaa 1860
tgtctttagg aaaaaagttg tctcaggtat agcaagtaaa ccaaaacagg atgatgctgg 1920
aagaattgca gcgagaaatc gcctagctgc cataaaaaat gcaatgagag agagaattag 1980
gcaggaagaa tgtgctgaaa cagcagtttc tgtgatacca aaggaagttg ataaaatagt 2040
gttcgatgct ggattttca gagttgaaag tcctgttaaa ttattctcag gactttctgt 2100
ctcttctgaa ggcccttctc aaagacttgg aacacctaag tctgtcaaca aagctgtatc 2160
tcagagtaga aatgagatgg gcattccaca acaaactaca tcaccagaaa atgccggtcc 2220
tcagaatacg aaaagtgaac atgtgaagaa gactttgttt ttgagtattc ctgaaagcag 2280
gagcagcata gaagatgctc agtgtcctgg attaccagat ttaattgaag aaaatcatgt 2340
tgtaaataag acagacttga aggtggattg tttatccagt gagagaatga gtttgcctct 2400
tcttgctggt ggagtagcag atgatattaa tactaacaaa aaagaaggaa tttcagatgt 2460
tgtggaagga atggaactga attcttcaat tacatcacag gatgttttga tgagtagccc 2520
tgaaaaaaat acagcttcac aaaatagcat cttagaagaa ggggaaacta aaatttctca 2580
gtcagaacta tttgataata aaagtctcac tactgaatgc caccttcttg attcaccagg 2640
tctaaactgc agtaatccat ttactcagct ggagaggaga catcaagaac atgccagaca 2700
catttctttt ggtggtaacc tgattacttt ttcacctcta caaccaggag aattttgaat 2760
ttaaaaataa atccaaacat tttccttcat attatcaatg cttatatatt ccttagacta 2820
ttgaaatttt ggagaaaatg tatttgtgtt cacttctata gcatataatg ttttaatatt 2880
ctgtgttcat caaagtgtat tttagatata ctctttctca agggaagtgg ggatattttg 2940
tacattttca acacagaata aaaaatgtac tgtgccttgc ctctcttgtt taa 2993

<210> 4
<211> 3317
<212> DNA
<213> Homo sapiens

<400> 4

```
cgaacagggg cggctgcctc actccctacc tgagccagcc gaggggccca aggactttag        60
agctgtttcc tccggcataa gagagacact tgctttccag ggcagcaccc tttatcggag       120
aaggctctac agggaagggg tctttgcagc ctggatggcc atcccacatt cctttaacgg       180
aggtctctag gcctcagaga gaacccagag ttagaaagga ggccagacgg tccttgctgt       240
```

```
cccccctgggg agagaggaag ttgccgcctg ctgccaggcc caggaggagc tgggcctgca      300
atagtggggg acctggcccc tgaggcagtg gcggccatgt cacggccagg ccacggtggg      360
ctgatgcctg tgaatggtct gggcttccca ccgcagaacg tggcccgggt ggtggtgtgg      420
gagtggctga atgagcacag ccgctggcgg ccctacacgg ccaccgtgtg ccaccacatt      480
gagaacgtgc tgaaggagga cgctcgcggt tccgtggtcc tggggcaggt ggacgcccag      540
cttgtgccct acatcatcga cctgcagtcc atgcaccagt ttcgccagga cacaggcacc      600
atgcggcccg tgcggcgcaa cttctacgac ccgtcgtcgg cgccgggcaa gggcatcgtg      660
tgggagtggg agaacgacgg cggcgcatgg acggcctacg atatggacat ctgcatcacc      720
atccagaacg cctacgagaa gcagcacccg tggctcgacc tctcatcgct aggcttctgc      780
tacctcatct acttcaacag catgtcgcag atgaaccgcc agacgcgccg gcgccgccgc      840
ctgcgccgcc gcctggacct cgcctacccg ctcaccgtgg gctccatccc taagtcgcag      900
tcgtggcccg tgggcgccag ctcgggccag ccctgctcct gccagcagtg cctgctggtc      960
aacagcacgc gcgccgcctc caacgccatc ctggcctcgc agcgccgcaa ggcgcccccc     1020
gcgcccccgc tgccgccgcc gccgccacct ggagggcctc caggcgcgct tgccgtgcgc     1080
cccagcgcca ccttcacagg cgccgcgctc tgggcagcgc ccgccgccgg ccccgccgag     1140
cccgcgccgc ctcccggggc gcccccacgg agcccgggcg cccccggcgg agcgcgcacc     1200
ccggggcaga acaacctcaa ccggcccggg ccccagcgca ccaccagcgt gagcgcgcgc     1260
gcctccatcc cgccgggggt ccccgcactc ccggtgaaga acttgaatgg tactgggccg     1320
gtccatccgg ccctggcagg gatgaccggg atactgctgt gcgcggccgg gctgcccgtg     1380
tgcctgacgc gggcccccaa gcccatcctg cacccgccgc ccgtgagcaa gagcgacgtg     1440
aagcccgtgc ctggcgtgcc cggggtgtgc cgcaagacca agaagaagca ccttaaaaag     1500
agtaagaatc ccgaggatgt ggttcgaaga tacatgcaga aggtgaaaaa cccacctgat     1560
gaggactgca ccatctgcat ggagcgactg gtcacagcat caggctacga gggcgtgctt     1620
cggcacaagg gcgtgcggcc tgagctcgtg ggccgcctgg gccgctgtgg ccacatgtac     1680
cacctgctgt gcctcgtggc catgtactcc aatggcaaca aggatggcag cctgcagtgc     1740
cccacctgca aggccatcta cggggagaag acgggtacgc agccgcctgg gaagatggag     1800
ttccacctca tcccccactc gctgcccggc ttccctgata cccagaccat ccgcatcgtc     1860
tatgacatcc ccacaggcat ccagggccct gagcacccca accccgggaa gaagttcacc     1920
gcaagaggat tccctcgcca ctgctatcta cccaacaacg agaaaggccg gaaggtgctg     1980
cggctgctca tcacggcctg ggagagaaga ctcatcttca ctatcggcac gtccaacacc     2040
acgggcgagt cggacaccgt ggtgtggaac gagatccacc acaagaccga gtttggatcc     2100
aacctcacgg gccacggcta cccggacgct agctacctag acaacgtgct ggctgagctc     2160
```

```
acagcccagg gcgtatccga ggctgcagcc aaggcttgag gcccaaggct gcccaccttc      2220
cctcctgctt tgcccctggt ccggcaaatg cctccttcgc caggtgtgtc ctggtagccc      2280
aggttcaggg ctggggagga gcctgcggaa ggggccgcag ccattcaggg gacctgcctg      2340
gtggcagctg ggatgaagag agatggcatg tcaggctggc cccgaatcat agctccctga      2400
gagggccaag cagagagtac tggaaacctc cctaccaaaa agacagagac ccgccccctc      2460
acacacaaac acacatgtcc tgttgaactc atgcacgcac acccacgtgc ctgtacttgc      2520
ccccaggctg gaagagaaga gacagaaaga ccccatgacc cccccatgtg gatccccatc      2580
tgtgtctcag ttgcatctgt acagccttgt ctgcaaactg gaggatgcgg ggcaagccct      2640
taggggcctg ccagggctcg gggggcaaag agggactcgg gaaactcagt gtaccccaga      2700
tgcctcaccc attccgtgtc atcacccatg tctgccaccc actgattggg caattgtggg      2760
cccatggggt ggaagccccc agatgactga gcagttctac aaaagaatgg ccagcacgag      2820
cggggactag agggtcctga ttttgtgtct gtgcctcttc atctctctgg actctgatct      2880
ccttctccct tcccatctcc aggccttctg tctgtcccag ataaaggcgc tgttctccca      2940
tcctccctac cccatcctct ccaccaaatc gctcccaatt ttgagagcca aaggctggcg      3000
cttctgactt caggagcgaa aggaggaggc ctagtttggg ccgatgtatt ttaaagcaga      3060
gtggacagca gagagtcaat ttccctttcg ttgggagtgg gcagtggggt ggctaattgt      3120
cttcggccaa ccaggggcct gttgcccagg caactcacca gctccgcctc tgctgattgg      3180
ctgccacggt gggagtcagc caagatttaa agggatgcca gcgattgctc ttttcaaaac      3240
ctaccagtcc cactgtgggt ggagaaataa atggtctttc tcctcctcaa aaaaaaaaaa      3300
aaaaaaaaaa aaaaaaa                                                     3317
```

<210> 5
<211> 4234
<212> DNA
<213> Homo sapiens

<400> 5

```
cgtgagcggc gcagcaagat cccagctcgg accccggacg gcgcgcgccc ccgaagcccc      60
ggatcccagt cgggcccgca gctgaccgcc agattactgt gcatcccgaa tcacgaccac     120
ctgcaccctc ctgccccggc ccgcccccca agtcctcagg cacccagctc cccggcgccc     180
cggatcctcc tggaccggtc cgtccagatt cccgcgggac cgacctgtcc gcatccccag     240
gaccgccggg ctcggtgcac cgcctcggtc ccggagccgc ccgcctggat tgcattccct     300
cctctcctgg atctcctggg acccgacgcg agcctgcccc ggagcccgcc gagcgcaccc     360
tctctcgggt gcctgcagcc ccgccggcgc ggcccggccc ggcgcggccc ggctcggctc     420
ctagagctgc cacggccatg gccagagccc gcccgccgcc gccgccgtcg ccgccgccgg     480
```

ggcttctgcc gctgctccct ccgctgctgc tgctgccgct gctgctgctg cccgccggct 540
gccgggcgct ggaagagacc ctcatggaca caaaatgggt aacatctgag ttggcgtgga 600
catctcatcc agaaagtggg tgggaagagg tgagtggcta cgatgaggcc atgaatccca 660
tccgcacata ccaggtgtgt aatgtgcgcg agtcaagcca gaacaactgg cttcgcacgg 720
ggttcatctg gcggcgggat gtgcagcggg tctacgtgga gctcaagttc actgtgcgtg 780
actgcaacag catccccaac atccccggct cctgcaagga gaccttcaac ctcttctact 840
acgaggctga cagcgatgtg gcctcagcct cctccccctt ctggatggag aacccctacg 900
tgaaagtgga caccattgca cccgatgaga gcttctcgcg gctggatgcc ggccgtgtca 960
acaccaaggt gcgcagcttt gggccacttt ccaaggctgg cttctacctg gccttccagg 1020
accagggcgc ctgcatgtcg ctcatctccg tgcgcgcctt ctacaagaag tgtgcatcca 1080
ccaccgcagg cttcgcactc ttccccgaga ccctcactgg ggcggagccc acctcgctgg 1140
tcattgctcc tggcacctgc atccctaacg ccgtggaggt gtcggtgcca ctcaagctct 1200
actgcaacgg cgatggggag tggatggtgc ctgtgggtgc ctgcacctgt gccaccggcc 1260
atgagccagc tgccaaggag tcccagtgcc gcccctgtcc ccctgggagc tacaaggcga 1320
agcagggaga ggggccctgc ctcccatgtc cccccaacag ccgtaccacc tccccagccg 1380
ccagcatctg cacctgccac aataacttct accgtgcaga ctcggactct gcggacagtg 1440
cctgtaccac cgtgccatct ccaccccgag gtgtgatctc caatgtgaat gaaacctcac 1500
tgatcctcga gtggagtgag ccccgggacc tgggtggccg ggatgacctc ctgtacaatg 1560
tcatctgcaa gaagtgccat ggggctggag gggcctcagc ctgctcacgc tgtgatgaca 1620
acgtggagtt tgtgcctcgg cagctgggcc tgacggagcg ccgggtccac atcagccatc 1680
tgctggccca cacgcgctac acctttgagg tgcaggcggt caacggtgtc tcgggcaaga 1740
gccctctgcc gcctcgttat gcggccgtga atatcaccac aaaccaggct gccccgtctg 1800
aagtgcccac actacgcctg cacagcagct caggcagcag cctcacccta tcctgggcac 1860
ccccagagcg gcccaacgga gtcatcctgg actacgagat gaagtacttt gagaagagcg 1920
agggcatcgc ctccacagtg accagccaga tgaactccgt gcagctggac gggcttcggc 1980
ctgacgcccg ctatgtggtc caggtccgtg cccgcacagt agctggctat gggcagtaca 2040
gccgccctgc cgagtttgag accacaagtg agagaggctc tggggcccag cagctccagg 2100
agcagcttcc cctcatcgtg ggctccgcta cagctgggct tgtcttcgtg gtggctgtcg 2160
tggtcatcgc tatcgtctgc ctcaggaagc agcgacacgg ctctgattcg gagtacacgg 2220
agaagctgca gcagtacatt gctcctggaa tgaaggttta tattgaccct tttacctacg 2280
aggaccctaa tgaggctgtt cgggagtttg ccaaggagat cgacgtgtcc tgcgtcaaga 2340

tcgaggaggt gatcggagct ggggaatttg gggaagtgtg ccgtggtcga ctgaaacagc 2400
ctggccgccg agaggtgttt gtggccatca agacgctgaa ggtgggctac accgagaggc 2460
agcggcggga cttcctaagc gaggcctcca tcatgggtca gtttgatcac ccccaatataa 2520
tccggctcga gggcgtggtc accaaaagtc ggccagttat gatcctcact gagttcatgg 2580
aaaactgcgc cctggactcc ttcctccggc tcaacgatgg gcagttcacg gtcatccagc 2640
tggtgggcat gttgcggggc attgctgccg gcatgaagta cctgtccgag atgaactatg 2700
tgcaccgcga cctggctgct cgcaacatcc ttgtcaacag caacctggtc tgcaaagtct 2760
cagactttgg cctctcccgc ttcctggagg atgacccctc cgatcctacc tacaccagtt 2820
ccctgggcgg gaagatcccc atccgctgga ctgccccaga ggccatagcc tatcggaagt 2880
tcacttctgc tagtgatgtc tggagctacg gaattgtcat gtgggaggtc atgagctatg 2940
gagagcgacc ctactgggac atgagcaacc aggatgtcat caatgccgtg gagcaggatt 3000
accggctgcc accacccatg gactgtccca cagcactgca ccagctcatg ctggactgct 3060
gggtgcggga ccggaacctc aggcccaaat tctcccagat tgtcaatacc ctggacaagc 3120
tcatccgcaa tgctgccagc ctcaaggtca ttgccagcgc tcagtctggc atgtcacagc 3180
ccctcctgga ccgcacggtc ccagattaca caaccttcac gacagttggt gattggctgg 3240
atgccatcaa gatggggcgg tacaaggaga gcttcgtcag tgcggggttt gcatcttttg 3300
acctggtggc ccagatgacg gcagaagacc tgctccgtat tggggtcacc ctggccggcc 3360
accagaagaa gatcctgagc agtatccagg acatgcggct gcagatgaac cagacgctgc 3420
ctgtgcaggt ctgacaccgg ctcccacggg gaccctgagg accgtgcagg gatgccaagc 3480
agccggctgg actttcggac tcttggactt ttggatgcct ggccttaggc tgtggcccag 3540
aagctggaag tttgggaaag gcccaagctg ggacttctcc aggcctgtgt tccctcccca 3600
ggaagtgcgc cccaaacctc ttcatattga agatggatta ggagaggggg tgatgacccc 3660
tccccaagcc cctcagggcc cagaccttcc tgctctccag caggggatcc ccacaacctc 3720
acacttgtct gttcttcagt gctggaggtc ctggcagggt caggctgggg taagccgggg 3780
ttccacaggg cccagccctg gcaggggtct ggcccccag gtaggcggag agcagtccct 3840
ccctcaggaa ctggaggagg ggactccagg aatggggaaa tgtgacacca ccatcctgaa 3900
gccagcttgc acctccagtt tgcacaggga tttgttctgg gggctgaggg ccctgtcccc 3960
accccccgccc ttggtgctgt cataaaaggg caggcagggg caggctgagg agttgccctt 4020
tgccccccag agactgactc tcagagccag agatgggatg tgtgagtgtg tgtgtgtgtg 4080
tgtgtgtgtg cgcgcgcgcg cgcgtgtgtg tgtgcacgca ctggcctgca cagagagcat 4140
gggtgagcgt gtaaaagctt ggccctgtgc cctacaatgg ggccagctgg gccgacagca 4200
gaataaaggc aataagatga aaaaaaaaa aaaa 4234

<210> 6
<211> 1047
<212> DNA
<213> Homo sapiens

<400> 6

```
tttctcaggc ataagggctg tagtgtgagg attgggagga actcgaccta ctccgctaac      60
ccagtggcct gagccaatca caaagaggat tggagcctca ctcgagcgct ccttcccttc     120
tcctctctct gtgacagcct cttggaaaga gggacactgg aggggtgtgt ttgcaattta     180
aatcactgga tttttgccca ccctctttcc aaataagaag gcaggagctg cttgctgagg     240
tgtaaagggt cttctgagct gcagtggcaa ttagaccaga agatccccgc tcctgtctct     300
aaagagggga aagggcaagg atggtggagg ctttctgtgc tacctggaag ctgaccaaca     360
gtcagaactt tgatgagtac atgaaggctc taggcgtggg ctttgccact aggcaggtgg     420
gaaatgtgac caaaccaacg gtaattatca gtcaagaagg agacaaagtg gtcatcagga     480
ctctcagcac attcaagaac acggagatta gtttccagct gggagaagag tttgatgaaa     540
ccactgcaga tgatagaaac tgtaagtctg ttgttagcct ggatggagac aaacttgttc     600
acatacagaa atgggatggc aaagaaacaa attttgtaag agaaattaag gatggcaaaa     660
tggttatgac ccttactttt ggtgatgtgg ttgctgttcg ccactatgag aaggcataaa     720
aatgttcctg gtcggggctt ggaagagctc ttcagttttt ctgtttcctc aagtctcagt     780
gctatcctat tacaacatgg ctgatcatta attagaaggt tatccttggt gtggaggtgg     840
aaaatggtga tttaaaaact tgttactcca agcaacttgc ccaattttaa tctgaaaatt     900
tatcatgttt tataatttga attaaagttt tgtccccccc cccctttttt ttataaacaa     960
gtgaatacat tttataattt cttttggaat gtaaatcaaa tttgaataaa aatcttacac    1020
gtgaaattta aaaaaaaaaa aaaaaaa                                        1047
```

<210> 7
<211> 3097
<212> DNA
<213> Homo sapiens

<400> 7

```
atcgccagtc tagcccactc cttcataaag ccctcgcatc ccaggagcga gcagagccag      60
agcaggatgg agaggagacg catcacctcc gctgctcgcc gctcctacgt ctcctcaggg     120
gagatgatgg tgggggggcct ggctcctggc cgccgtctgg gtcctggcac ccgcctctcc     180
ctggctcgaa tgccccctcc actcccgacc cgggtggatt tctccctggc tggggcactc     240
aatgctggct tcaaggagac ccgggccagt gagcgggcag agatgatgga gctcaatgac     300
cgctttgcca gctacatcga gaaggttcgc ttcctggaac agcaaaacaa ggcgctggct     360
```

```
gctgagctga accagctgcg ggccaaggag cccaccaagc tggcagacgt ctaccaggct      420
gagctgcgag agctgcggct gcggctcgat caactcaccg ccaacagcgc ccggctggag      480
gttgagaggg acaatctggc acaggacctg gccactgtga ggcagaagct ccaggatgaa      540
accaacctga ggctggaagc cgagaacaac ctggctgcct atagacagga agcagatgaa      600
gccaccctgg cccgtctgga tctggagagg aagattgagt cgctggagga ggagatccgg      660
ttcttgagga agatccacga ggaggaggtt cgggaactcc aggagcagct ggcccgacag      720
caggtccatg tggagcttga cgtggccaag ccagacctca ccgcagccct gaaagagatc      780
cgcacgcagt atgaggcaat ggcgtccagc aacatgcatg aagccgaaga gtggtaccgc      840
tccaagtttg cagacctgac agacgctgct gcccgcaacg cggagctgct ccgccaggcc      900
aagcacgaag ccaacgacta ccggcgccag ttgcagtcct tgacctgcga cctggagtct      960
ctgcgcggca cgaacgagtc cctggagagg cagatgcgcg agcaggagga gcggcacgtg     1020
cgggaggcgg ccagttatca ggaggcgctg gcgcggctgg aggaagaggg gcagagcctc     1080
aaggacgaga tggcccgcca cttgcaggag taccaggacc tgctcaatgt caagctggcc     1140
ctggacatcg agatcgccac ctacaggaag ctgctagagg gcgaggagaa ccggatcacc     1200
attcccgtgc agaccttctc caacctgcag attcgagaaa ccagcctgga caccaagtct     1260
gtgtcagaag gccacctcaa gaggaacatc gtggtgaaga ccgtggagat gcgggatgga     1320
gaggtcatta aggagtccaa gcaggagcac aaggatgtga tgtgaggcag gacccacctg     1380
gtggcctctg ccccgtctca tgaggggccc gagcagaagc aggatagttg ctccgcctct     1440
gctggcacat ttccccagac ctgagctccc caccacccca gctgctcccc tccctcctct     1500
gtccctaggt cagcttgctg ccctaggctc cgtcagtatc aggcctgcca gacggcaccc     1560
acccagcacc cagcaactcc aactaacaag aaactcaccc ccaaggggca gtctggaggg     1620
gcatggccag cagcttgcgt tagaatgagg aggaaggaga gaaggggagg agggcggggg     1680
gcacctacta catcgccctc cacatccctg attcctgttg ttatggaaac tgttgccaga     1740
gatggaggtt ctctcggagt atctgggaac tgtgcctttg agtttcctca ggctgctgga     1800
ggaaaactga gactcagaca ggaaagggaa ggccccacag acaaggtagc cctggccaga     1860
ggcttgtttt gtcttttggt ttttatgagg tgggatatcc ctatgctgcc taggctgacc     1920
ttgaactcct gggctcaagc agtctaccca cctcagcctc ctgtgtagct gggattatag     1980
attggagcca ccatgcccag ctcagagggt tgttctccta gactgaccct gatcagtcta     2040
agatgggtgg ggacgtcctg ccacctgggg cagtcacctg cccagatccc agaaggacct     2100
cctgagcgat gactcaagtg tctcagtcca cctgagctgc catccaggga tgccatctgt     2160
gggcacgctg tgggcaggtg ggagcttgat tctcagcact tgggggatct gttgtgtacg     2220
tggagaggga tgaggtgctg ggagggatag agggggctg cctggccccc agctgtgggt      2280
```

acagagaggt caagcccagg aggactgccc cgtgcagact ggaggggacg ctggtagaga 2340

tggaggagga ggcaattggg atggcgctag gcatacaagt aggggttgtg ggtgaccagt 2400

tgcacttggc ctctggattg tgggaattaa ggaagtgact catcctcttg aagatgctga 2460

aacaggagag aaaggggatg tatccatggg ggcagggcat gactttgtcc catttctaaa 2520

ggcctcttcc ttgctgtgtc ataccaggcc gccccagcct ctgagcccct gggactgctg 2580

cttcttaacc ccagtaagcc actgccacac gtctgaccct ctccacccca tagtgaccgg 2640

ctgcttttcc ctaagccaag ggcctcttgc ggtcccttct tactcacaca caaaatgtac 2700

ccagtattct aggtagtgcc ctattttaca attgtaaaac tgaggcacga gcaaagtgaa 2760

gacactggct catattcctg cagcctggag gccgggtgct cagggctgac acgtccaccc 2820

cagtgcaccc actctgcttt gactgagcag actggtgagc agactggtgg gatctgtgcc 2880

cagagatggg actgggaggg cccacttcag ggttctcctc tcccctctaa ggccgaagaa 2940

gggtccttcc ctctccccaa gacttggtgt cctttccctc cactccttcc tgccacctgc 3000

tgctgctgct gctgctaatc ttcagggcac tgctgctgcc tttagtcgct gaggaaaaat 3060

aaagacaaat gctgcgccct tccccaaaaa aaaaaaa 3097

<210> 8
<211> 1895
<212> DNA
<213> Homo sapiens

<400> 8

atgacaggaa gtgacccgtt aaggaagcag cacatcgctg cattcggctg gttttcaggg 60

tcttgttccc aatcagtttc cagccaacac cagggtgtcc tagtccgcag aggtgtgggg 120

gacacactcc ataatctcta cttttctttt tgtgcagctg agtcatggag ctttcagccc 180

cagcacatgg ctcctcctta actgcgtctg ctcaacctcc ctcagccctg tgaacagcat 240

ccccgcacac agacgcagag caggactctc tctgctgcca cttcaccttc ctgagagagg 300

accagcggcc agagcctcag tgactgccac cctggaggac agggcacaac aaccgtttct 360

ggagagaatg ggaggattcc agaggggcaa atatggaact atggctgaag gtagatcaga 420

agataacttg tctgcaacac caccggcatt gaggattatc ctagtgggca aaacaggctg 480

cgggaaaagt gccacaggga acagcatcct tggccagccc gtgtttgagt ccaagctgag 540

ggcccagtca gtgaccagga cgtgccaggt gaaaacagga acatggaacg ggaggaaagt 600

cctggtggtt gacacgccct ccatctttga gtcacaggcc gatacccaag agctgtacaa 660

gaacatcggg gactgctacc tgctctctgc cccggggccc cacgtcctgc ttctggtgat 720

ccagctgggg cgtttcactg ctcaggacac agtggccatc aggaaggtga aagaggtctt 780

tgggacaggg gccatgagac atgtggtcat cctcttcacc cacaaagagg acttaggggg 840

ccaggccctg gatgactatg tagcaaacac ggacaactgc agcctgaaag acctggtgcg 900

ggagtgtgag agaaggtact gtgccttcaa caactggggc tctgtggagg agcagaggca 960

gcagcaggca gagctcctgg ctgtgattga gaggctgggg agggagcgag agggctcctt 1020

ccacagcaat gacctcttct tggatgccca gctgctccaa agaactggag ctggggcctg 1080

ccaggaagac tacaggcagt accaggccaa agtggaatgg caggtggaga agcacaagca 1140

agagctgagg gagaacgaga gtaactgggc atacaaggcg ctcctcagag tcaaacactt 1200

gatgcttttg cattatgaga tttttgtttt tctattgttg tgcagcatac ttttttttcat 1260

tatttttctg ttcatctttc attacattta aatctctgga ccctggagca cttctaatgt 1320

atcaccccat ggagtcattg ttctaataat caccaattca gactcagatc ctcgtggtct 1380

atggagcatg ctgcttgctg tctgtgcagc tcccatttcc ccttcttcct gatagacttg 1440

gagctgtgtg cctccactcc aaggctgcct gcctgctgta aacactattc cactctgtct 1500

gccaacaact gcttcaggaa tgggcctgag atcccatgca ggtccctgag aagtgagtaa 1560

aagtccgcag aggtggggat ggaagatctc tccttagata gaacctgtct tcctccctgg 1620

cattgtgggg tctgggcgtg acactgggac tctcagcagc tttgtgctgc caacctgaga 1680

ttgaaggcag tgcctcagag cagcacagag agttggggcc ccctgagccc tgagccacca 1740

gccctgcagc ctgccctatc tccgcatttc cagttgtatt agccaataga tttcctactt 1800

atttaagcta tttgagctcc gggtctcttc tacctgcatt ctaaaacatt caaagtaata 1860

aaaatttctc cacattcaaa aaaaaaaaaa aaaaa 1895

<210> 9
<211> 1475
<212> DNA
<213> Homo sapiens

<400> 9

```
gggatcacac aggatccgga gctggtgctg ataacagcgg aatcccccgt ctacctctct      60
ccttggtcct ggaacagcgc tactgatcac caagtagcca caaaatataa taaaccctca     120
gcacttgctc agtagttttg tgaaagtctc aagtaaaaga gacacaaaca aaaaattctt     180
tttcgtgaag aactccaaaa ataaaattct ctagagataa aaaaaaaaaa aaaaggaaaa     240
tgccagctga tataatggag aaaaattcct cgtccccggt ggctgctacc ccagccagtg     300
tcaacacgac accggataaa ccaaagacag catctgagca cagaaagtca tcaaagccta     360
ttatggagaa aagacgaaga gcaagaataa atgaaagtct gagccagctg aaaacactga     420
ttttggatgc tctgaagaaa gatagctcgc ggcattccaa gctggagaag gcggacattc     480
tggaaatgac agtgaagcac ctccggaacc tgcagcgggc gcagatgacg gctgcgctga     540
gcacagaccc aagtgtgctg gggaagtacc gagccggctt cagcgagtgc atgaacgagg     600
tgacccgctt cctgtccacg tgcgagggcg ttaataccga ggtgcgcact cggctgctcg     660
gccacctggc caactgcatg acccagatca atgccatgac ctaccccggg cagccgcacc     720
ccgccttgca ggcgccgcca ccgcccccac cgggacccgg cggcccccag cacgcgccgt     780
tcgcgccgcc gccgccactc gtgcccatcc ccgggggcgc ggcgcccccct cccggcggcg     840
ccccctgcaa gctgggcagc caggctggag aggcggctaa ggtgtttgga ggcttccagg     900
tggtaccggc tcccgatggc cagtttgctt tcctcattcc caacggggcc ttcgcgcaca     960
gcggccctgt catccccgtc tacaccagca acagcggcac ctccgtgggc cccaacgcag    1020
tgtcaccttc cagcggcccc tcgcttacgg cggactccat gtggaggccg tggcggaact    1080
gaggggggctc aggccacccc tcctcctaaa ctccccaacc cacctctctt ccctccggac    1140
tctaaacagg aacttgaata ctgggagaga agaggacttt tttgattaag tggttacttt    1200
gtgttttttt aatttctaag aagttacttt ttgtagagag agctgtatta agtgactgac    1260
catgcactat atttgtatat attttatatg ttcatattgg attgcgcctt tgtattataa    1320
aagctcagat gacatttcgt tttttacacg agatttcttt tttatgtgat gccaaagatg    1380
tttgaaaatg ctcttaaaat atcttccttt ggggaagttt atttgagaaa atataataaa    1440
agaaaaaagt aaaggctttt aaaaaaaaaa aaaaa                                1475
```

<210> 10
<211> 962
<212> DNA
<213> Homo sapiens

<400> 10

gggaaagaat gcggagccgg gttcacacac cccgcggcgg cgaggcctta aatagggaaa 60
cggcctgagg cgcgcgcggg cctggagccg ggatccgccc taggggctcg gatcgccgcg 120
cgctcgccgc tcgcccgcca gcccgcccgt ggtccgtggc ggcgcgctcc acccggcacg 180
gggaggcgcg gggcgcacca tggccgcaga cacgccgggg aaaccgagcg cctcgccgat 240
ggcaggagcg ccggccagcg ccagccggac cccagacaag ccccggagcg cggccgagca 300
ccgcaagtcc tccaagccgg tcatggagaa gcggcgccga gcgcgtatta acgagagcct 360
cgctcagctc aaaaccctca tcctggacgc cctcagaaaa gagagctccc gccactcgaa 420
gctggagaag gcggacatcc tggagatgac cgtgagacac ctgcggagcc tgcgtcgcgt 480
gcaggtgacg gccgcgctca gcgccgaccc cgccgttctg ggcaagtacc gcgccggctt 540
ccacgagtgt ctggcggagg tgaaccgctt cctggccggc tgcgagggcg tcccggccga 600
cgtgcgctcc cgcctgctgg gccacctggc agcctgcctg cgccagctgg gaccctcccg 660
ccgcccggcc tcgctgtccc cggctgcccc cgcagaggcc ccagcgcccg aggtctacgc 720
gggccgcccg ctgctgccat cgctcggcgg ccccttccct ctgctcgcgc cgccgctgct 780
gccgggtctg acccgggcgc tgcccgccgc cccagggcg gggccgcagg gcccgggtgg 840
gccctggagg ccgtggctgc gctgaggctg tggccctgag actgcatcgg aggcggcgcc 900
ccgttctagg gccgtggcct ttgccgagac tgtagcagag aaaacgtatt tattattcca 960
ga 962

<210> 11
<211> 1319
<212> DNA
<213> Homo sapiens

<400> 11

```
cgcgcttggc cttgcccgcg cccgctcgcc tcgtctcgcc cggcctcccc gcgtcgcctc      60
gtcgcctgtt ccgcgccagg catggccccc agcactgtgg ccgtggagct gctcagcccc     120
aaagagaaaa accgactgcg gaagccggtg gtggagaaga tgcgccgcga ccgcatcaac     180
agcagcatcg agcagctgaa gctgctgctg gagcaggagt tcgcgcggca ccagcccaac     240
tccaagctgg agaaggccga catcctggag atggctgtca gctacctgaa gcacagcaaa     300
gccttcgtcg ccgccgccgg ccccaagagc ctgcaccagg actacagcga aggctactcg     360
tggtgcctgc aggaggccgt gcagttcctg acgctccacg ccgccagcga cacgcagatg     420
aagctgctgt accacttcca gcggcccccg gccgcgcccg ccgcgcccgc caaggagccc     480
aaggcgccgg gcgccgcgcc cccgcccgcg ctctccgcca aggccaccgc cgccgccgcc     540
gccgcgcacc agcccgcctg cggcctctgg cggccctggt gacccggcgg gacctgcggg     600
cgcgcggccc gacgaccaga gggcgagcct gctcctctcg cctgtaggga agcgccttcc     660
cgccgtcgtc cgccccgggc ttggacgcgc ccttctccgg aaggctctgg ccccaagctg     720
gccggcccgc aggagcccca ttctcagaga atgtgtgtgc agagtccctg ccgttttagg     780
acaatcaggg cccatcttct gccaagtgtc tgaccccatg gggttgttct gtgtttgcat     840
ttaagcaagt gacttctggg aagtccccgg ccgcccgggg ttctatgata tttgtagtgc     900
cggggctcgc acactgctgc ccccagcctg tagaggactt tcttcagggc ccgtagctgc     960
tgggcgtacc cctggcaggc gggctgtgcc gcgggcacat ttgccttttg tgaaggccga    1020
actcgagctg tatcctcata ggaaacagtg atcaccccgg acgggcgtcc aggaccctga    1080
gggccatggc caaaaggctc ctgagtgtgc ctggtggtct ggctggggct cacggtgggc    1140
tgtctgggga gggtgggtgc ctccactatg atccttaaag gattcctctg tgtgggtgga    1200
tgcgtgtggg cacgactttg tactcagaaa ttgaactctc agtcacgtgg aagccacggg    1260
actgctccga agccgccata ataaaatctg attgttcagc ccccaaaaaa aaaaaaaaa     1319
```

<210> 12
<211> 1684
<212> DNA
<213> Homo sapiens

<400> 12

gaggagcaat ggtcacccgg gatcgagctg agaataggga cggccccaag atgctcaagc 60
cgcttgtgga gaagcggcgc cgggaccgca tcaaccgcag cctggaagag ctgaggctgc 120
tgctgctgga gcggacccgg gaccagaacc tccggaaccc gaagctggag aaagcggaga 180
tattggagtt cgccgtgggc tacttgaggg agcgaagccg ggtggagccc ccggggggttc 240
cccggtcccc agtccaggac gccgaggcgc tcgccagctg ctacttgtcc ggtttccgcg 300
agtgcctgct tcgcttggcg gccttcgcgc acgacgccag cccggccgcc cgcgcccagc 360
tcttctccgc gctgcacggc tatctgcgcc ccaaaccgcc ccggcccaag ccggtagatc 420
cgaggcctcc agcgccgcgc ccatccctgg accccgccgc accggccctt ggccctgcgc 480
tgcaccagcg ccccccagtg caccagggcc accctagccc gcgctgcgca tggtccccat 540
ccctctgctc cccgcgcgcc ggggattctg gcgcgccggc gcccctcacc ggactgctgc 600
cgccgccacc gccgcctcac agacaagacg ggcgcccaa ggccccgctg cccccgccgc 660
ccgctttctg gagaccttgg ccctgagcct tggggggtgg tgggggcggg gtctaggggt 720
ggggtagaga ctccagcccg agggcagcag agggacccgg gcgtccgggc gagcaggtgt 780
tggggagggc agtggggcgc gcgggctcag cgcgcgggtg agatgtggtc tatattagag 840
tatctatata aatatatatt tccctggttc ctgtcccttt tccctgcccc aacttctccc 900
ttgcgtctag gattgtactc tctctgcccc tcagcccagt cccagtccct tcccgagtcc 960
ctagtgcatg gaataaagtg gttattaaat ccccgtgtgt ccccgagcca ggggcctgcc 1020
tttatctcga cgtccacgcc cactttccct tcccttctgt ctcccaccct cagtcctgct 1080
ctccatggcc caagccccgg ggcagacagg taagtaaaga agagagcaga gcgggaactg 1140
agatcgaaat tgaaaccagg tggaaagaga gagatagggt agggggagaa gggatggggg 1200
cctttaagaa aaaaacggat aaaaaggaaa aattgaaata aaatcgactc tggtgggatt 1260
cgaacccaca acctttgaat tgctctattc gtcactagaa gtccaatgcg ctatccattg 1320
cgccacagag ccacccgacg aacggcggcg tcttgtagct tacgggtact agagtgggaa 1380
tggggcaggg ttggggagcg gggctaaggg acttgggcgg gacatgccag gagggcgcgg 1440
tttggatctc agaggccaag ccaggtagag gtagcgggcg caaagcatgt tagccaggtg 1500
agagagaggg cgcacatggg tcgaaaaaac agggagggag agcaaccgaa aatggctgag 1560
cgagcgagtg cagagctccg gctgcccgct tggggggtgt ttccggctca ggcgctcccc 1620
actcccagat atagtcccac ccaaataaac tagttttgtt gtaaattaaa aaaaaaaaaa 1680
aaaa 1684

<210> 13
<211> 2319
<212> DNA

<213> Homo sapiens

<400> 13

```
ttccccactc ccccgccctc cccagggccc tgggaagggg ctcagcgtgg gaaaggatgg      60
ttgagtttta accagaggca aagcgtgagc gggatcagtg tgtgcggaac gcaagcagcc     120
gagagcggag aggcgccgct gtagttaact cctccctgcc cgccgcgccg accctcccca     180
ggaaccccca gggagccagc atgaagcgag ctcaccccga gtacagctcc tcggacagcg     240
agctggacga gaccatcgag gtggagaagg agagtgcgga cgagaatgga aacttgagtt     300
cggctctagg ttccatgtcc ccaactacat cttcccagat tttggccaga aaaagacgga     360
gaggaataat tgagaagcgc cgacgagacc ggatcaataa cagtttgtct gagctgagaa     420
ggctggtacc cagtgctttt gagaagcagg gatctgctaa gctagaaaaa gccgagatcc     480
tgcagatgac cgtggatcac ctgaaaatgc tgcatacggc aggagggaaa ggttactttg     540
acgcgcacgc ccttgctatg gactatcgga gtttgggatt tcgggaatgc ctggcagaag     600
ttgcgcgtta tctgagcatc attgaaggac tagatgcctc tgacccgctt cgagttcgac     660
tggtttcgca tctcaacaac tacgcttccc agcgggaagc cgcgagcggc gcccacgcgg     720
gcctcggaca cattccctgg gggaccgtct tcggacatca cccgcacatc gcgcacccgc     780
tgttgctgcc ccagaacggc cacgggaacg cgggcaccac ggcctcaccc acggaaccgc     840
accaccaggg caggctgggc tcggcacatc cggaggcgcc tgctttgcga gcgcccccta     900
gcggcagcct cggaccggtg ctccctgtgg tcacctccgc ctccaaactg tcgccgcctc     960
tgctctcctc agtggcctcc ctgtcggcct tccccttctc tttcggctcc ttccacttac    1020
tgtctcccaa tgcactgagc ccttcagcac ccacgcaggc tgcaaacctt ggcaagccct    1080
atagaccttg gggacggag atcggagctt tttaaagaac tgatgtagaa tgagggaggg    1140
gaaagtttaa aatcccagct gggctggact gttgccaaca tcaccttaaa gtcgtcagta    1200
aaagtaaaaa ggaaaaaggt acactttcag ataatttttt ttttaaagac taaaggtttg    1260
ttggtttact tttatctttt ttaatgtttt tttcatcatg tcatgtatta gcagttttta    1320
aaaactagtt gttaaatttt gttcaagaca ttaaattgaa atagtgagta taagccaaca    1380
ctttgtgata ggtttgtact gtgcctaatt tactttgtaa accagaatga ttccgttttt    1440
gcctcaaaat ttggggaatc ttaacattta gtatttttgg tctgttttc tccttgtata    1500
gttatggtct gtttttagaa ttaattttcc aaaccactat gcttaatgtt aacatgattc    1560
tgtttgttaa tattttgaca gattaaggtg ttgtataaat aatattcttt tgggggggagg    1620
ggaactatat tgaattttat atttctgagc aaagcgttga caaatcagat gatcagcttt    1680
atccaagaaa gaagactagt aaattgtctg cctcctatag cagaaaggtg aatgtacaaa    1740
```

```
ctgttggtgg ccctgaatcc atctgaccag ctgctggtat ctgccaggac tggcagttct      1800
gatttagtta ggagagagcc gctgataggt taggtctcat ttggagtgtt ggtggaaagg      1860
aaactgaagg taattgaata gaatacgcct gcatttacca gccccagcaa cacaaagaat      1920
ttttaatcac acggatctca aattcacaaa tgttaacatg gataagtgat catggtgtgc      1980
gagtggtcaa ttgagtagta cagtggaaac tgttaaatgc ataacctaat tttcctggga      2040
ctgccatatt ttcttttaac tggaaatttt tatgtgagtt ttccttttgg tgcatggaac      2100
tgtggttgcc aaggtattta aaagggcttt cctgcctcct tctctttgat ttatttaatt      2160
tgatttgggc tataaaatat catttttcag gtttattctt ttagcaggtg tagttaaacg      2220
acctccactg aactgggttt gacctctgtt gtactgatgt gttgtgacta aataaaaaag      2280
aaagaacaaa gtaaaaaaaa aaaaaaaaaa aaaaaaaaa                             2319
```

<210> 14
<211> 2672
<212> DNA
<213> Homo sapiens

<400> 14

gcgtggccgg cgccggctct tgcggccgag cagagttgcg gcgtgggaaa gagccgctag 60

gagcagaccg cgccgccgcc ggagccgcgc ctgcccaggc ccggggaggg aggaggcggg 120

cgtcagggtg ctgcgccccg ctcggcgtcc gagcttccgg ccgggctgtg ccccgcgcgg 180

tcttcgccgg gatgaagcgc ccctgcgagg agacgacctc cgagagcgac atggacgaga 240

ccatcgacgt ggggagcgag aacaattact cggggcaaag tactagctct gtgattagat 300

tgaattctcc aacaacaaca tctcagatta tggcaagaaa gaaaaggaga gggattatag 360

agaaaaggcg tcgggatcgg ataaataaca gtttatctga gttgagaaga cttgtgccaa 420

ctgcttttga aaaacaagga tctgcaaagt tagaaaaagc tgaaatattg caaatgacag 480

tggatcattt gaagatgctt caggcaacag gggtaaagg ctactttgac gcacacgctc 540

ttgccatgga cttcatgagc ataggattcc gagagtgcct aacagaagtt gcgcggtacc 600

tgagctccgt ggaaggcctg gactcctcgg atccgctgcg ggtgcggctt gtgtctcatc 660

tcagcacttg cgccacccag cgggaggcgg cggccatgac atcctccatg gcccaccacc 720

atcatccgct ccacccgcat cactgggccg ccgccttcca ccacctgccc gcagccctgc 780

tccagcccaa cggcctccat gcctcagagt caacccccttg tcgcctctcc acaacttcag 840

aagtgcctcc tgcccacggc tctgctctcc tcacggccac gtttgcccat gcggattcag 900

ccctccgaat gccatccacg ggcagcgtcg ccccctgcgt gccacctctc tccacctctc 960

tcttgtccct ctctgccacc gtccacgccg cagccgcagc agccaccgcg gctgcacaca 1020

gcttccctct gtccttcgcg ggggcattcc ccatgcttcc cccaaacgca gcagcagcag 1080

tggccgcggc cacagccatc agcccgccct tgtcagtatc agccacgtcc agtcctcagc 1140

agaccagcag tggaacaaac aataaacctt accgaccctg ggggacagaa gttggagctt 1200

tttaaatttt tcttgaactt cttgcaatag taactgaatg tcctccattt cagagtcagc 1260

ttaaaacctc tgcaccctga aggtagccat acagatgccg acagatccac aaaggaacaa 1320

taaagctatt tgagacacaa acctcacgag tggaaatgtg gtattctctt tttttctct 1380

ccctttttttg tttggttcaa ggcagctcgg taactgacat cagcaacttt tgaaaacttc 1440

acacttgtta ccatttagaa gtttcctgga aaatatatgg accgtaccat ccagcagtgc 1500

atcagtatgt ctgaattggg gaagtaaaat gccctgactg aattctcttg agactagatg 1560

ggacatacat atatagagag agagtgagag agtcgtgttt cgtaagtgcc tgagcttagg 1620

aagttttctt ctggatatat aacattgcac aagggaagac gagtgtggag gataggttaa 1680

gaaaggaaag ggacagaagt cttgcaatag gctgcagaca ttttaatacc atgccagaga 1740

agagtattct gctgaaacca acaggtttta ctggtcaaaa tgactgctga aaataatttt 1800

caagttgaaa gatctagttt tatcttagtt tgccttcttt gtacagacat gccaagaggt 1860

gacatttagc agtgcattgg tataagcaat tatttcatca gttctcagat taacaagcat 1920

ttctgctctg cctgcaggcc cccaggcact ttttttttttg gatggctcaa aatatggtgc 1980

tgctttatat aaaccttaca tttatatagt gcacctatga gcagttgcct accatgtgtc 2040

caccagaggc tatttaattc atgccaactt gaaaactctc cagtttgtag gagtttggtt 2100

taatttattc agtttcatta ggactatttt tatatattta tcctcttcat tttctcctaa 2160

tgatgcaaca tctattcttg tcaccctttg ggagaagtta catttctgga ggtgatgaag 2220

caaggaggga gcactaggaa gagaaaagct acaattttta aagctctttg tcaagttagt 2280

gattgcattt gatcccaaaa caagatgaat gtatgcaatg ggatgtacat aagttatttt 2340

tgcccatgcc taaactagtg ctatgtaatg ggttgtggt tttgtttttt tcgatttcgt 2400

ttaatgacaa aataatctct taatatgctg aaatcaagca cgtgagagtt tttgtttaaa 2460

agataagaga cacagcatgt attatgcact tcatttctct actgtgtgga gaaagcaata 2520

aacattatga gaatgttaaa cgttatgcaa aattatactt ttaaatattt gttttgaaat 2580

tactgtacct agtctttttt gcattacttt gtaacctttt tctatgcaag agtctttaca 2640

taccactaat taaatgaagt ccttttttgac ta 2672

<210> 15
<211> 4131
<212> DNA
<213> Homo sapiens

<400> 15

ccgactggga gccttagccg cggggctgag accaggcagc ctgcgttcgc catgaagcga 60

cccaaggagc cgagcggctc cgacggggag tccgacggac ccatcgacgt gggccaagag 120
ggccagctga gccagatggc caggccgctg tccaccccca gctcttcgca gatgcaagcc 180
aggaagaaac gcagagggat catagagaaa cggcgtcgag accgcatcaa cagtagcctt 240
tctgaattgc gacgcttggt ccccactgcc tttgagaaac agggctcttc caagctggag 300
aaagccgagg tcttgcagat gacggtggat cacttgaaaa tgctccatgc cactggtggg 360
acaggattct ttgatgcccg agccctggca gttgacttcc ggagcattgg ttttcgggag 420
tgcctcactg aggtcatcag gtacctgggg gtccttgaag ggcccagcag ccgtgcagac 480
cccgtccgga ttcgccttct ctcccacctc aacagctacg cagccgagat ggagccttcg 540
cccacgccca ctggcccttt ggccttccct gcctggccct ggtctttctt ccatagctgt 600
ccagggctgc cagccctgag caaccagctc gccatcctgg gaagagtgcc cagccctgtc 660
ctccccggtg tctcctctcc tgcttacccc atcccagccc tccgaaccgc tccccttcgc 720
agagccacag gcatcatcct gccagcccgg aggaatgtgc tgcccagtcg aggggcatct 780
tccacccgga gggcccgccc cctagagagg ccagcgaccc ctgtgcctgt cgcccccagc 840
agcagggctg ccaggagcag ccacatcgct cccctcctgc agtcttcctc cccaacaccc 900
cctggtccta cagggtcggc tgcttacgtg gctgttccca cccccaactc atcctcccca 960
gggccagctg ggaggccagc gggagccatg ctctaccact cctgggtctc tgaaatcact 1020
gaaatcgggg ctttctgagc tgccccttca ccaccccgcc ccaaggaata aggaaggttc 1080
ttttaccagg agcccaaaaa agggcactgc cttttctgct ttgcttcatg gactggctca 1140
tatgtgaagg cacgttctcc agccatcaga ggcccctcc tcctccaacc catctctcct 1200
tctcactgtt atcccagctt atccacccag ctctcctgga gctgttctgg tctcagaggc 1260
ttggttccat ttctcacctg aacagatgag tcctgggaga gaccctcaga gatccgccca 1320
gacccctctc ctgccctctg cacaccagca gcaggcatga accttgggtc tgggaaaaag 1380
ctttaacctg cagggcacca ggacccaagg caggctgttc cttggggcgg tcagacccca 1440
gtcaggagca atgactgact ggctgcagcc ttcccacgcc aagaggctgg aacatagtgt 1500
ctgcctcgct tcctggagat agtaactgag caggggctac aaagaggtct cctgggaacc 1560
ctgtctgccc cttcccacct gtccttgggc cacaccatca cactgaacca caggacagac 1620
cctttctcca ccacagccaa ggcctggaga ctgggggccc agcagagcct gctcccaccc 1680
tcctcccagc agcagacacc caccctctca ctgactaaca ggtccctgca cacagctggc 1740
ctggtaaacc cagctgggag gtttctaggc agcagcaaaa ctctgtgaca gggtgtcctc 1800
acaccaggcc ttggacagct ctcccagaca ggagccaggg ttgagcaatg gagagcccag 1860
cccccacgtc ttacagtcgc catcctccag gcgtgtggtc cctccccatt gggtgcacag 1920

tgcagagggg ccgtggcccc atgtgatggt gcgcagagag gaacctcttg ggattcagca 1980
ccagacgtct gtgctgcctg gtttgcatcc ggctcacaga gcccagactg ctggaacagc 2040
caaggactgt caggctggac aaaaataact gcaaggaggg gcaagagaaa ggatgattcg 2100
aggcaccttg gcccttcaag gtcatgcagt gggtcgagcg cctgagatcc tgttcaccag 2160
gactccacag agctggctct gctcagaagc catttcattc cccggctcca ccctaggcca 2220
ctttttctaa cagaggaaac aaatggtcca gcagtcgttc ccagcagaac agcggagcct 2280
ggactgacac ccagtgggac cagtgttgcc acaccagttg ataaaatgca gaaacccttc 2340
tgtactcgtt ggtaaatatc tactccccca agtgactcca ggtgcccccc accgcctggc 2400
acttccccca ggactcctac gatctggtta ctgcctggcc gatccaaggc tgtggagtcc 2460
cagagccagc agttcactgg tgctcattcc acactggtta gatacttcag ttgtcacccc 2520
tgggaagatt ctcccacctc ctccctttga tggaaccacc ctccccagag gctgcattga 2580
ggagactcca cagactgaaa agtgagtttg cagaaacctt ggggaaaagg gccctttcaa 2640
agaagtggat aagagggagg agatcattga gtgacccaga aagctctttt gaaaagacag 2700
actcctcaag gagagataaa gaggaaagca cctctttcat tttttagtgt gagctaattc 2760
catcagactg ctgtcctcct ggacccatct gagatgtgca gtagcaagga gagggggat 2820
cattttagag agtgggtcat tggcagggag tgctccggag ggaggcagag gggagactgt 2880
ggtagaagga agacagaact cacacatgct cccaggattg gggacaggga cagaggaggt 2940
aacagaaggc aaaggccagt ttccccgtta tcatgaaggg gcccactcag gacaggaaca 3000
aggacaactc ctcctcctcc tcctcctctc ctgctgctcc tgggatacca ggtcagtgat 3060
gtagtcttgc agtttggcaa cttcctagcc tgagaatccc tagtggggct gtgggaaaca 3120
catttccacg ttgcaagcat gcaactccaa agaatctgtg atgccactga aatgagatgg 3180
gaatgatcca gctctttcag catcttggtt gaacttgctt tcattgtccc tgggatattg 3240
tggaaggaaa ggtgactgtg tgatctgatt ctgtggtcaa ggacttgcat cttgtgtttc 3300
tatccccaag ccttcctggt gtctccaact cctaccccat tgcatgggtt gttgcggaca 3360
tccaataaag atttttttag tgcttctgga aacttccagt agattctact tctaaactat 3420
ctctggagtc catccacttc tgtctgcacc cacagccatc ctggccaggc cacatcacct 3480
cccccagatc actgccctgg cctcagaaag gtcttccctc ttgctttgtc aatcagttct 3540
cagtagcagc agagagaaat tgaaagctgc aggtcatatc gtatcatctt tagtttgaaa 3600
acctcactct cttaccctat tgttctaaag gtcttctttt ggtcccaacc tcatttccag 3660
cctcatttct tgccagtccc agacttgctc cctgagcttc tgccacctgc ccttccttca 3720
tttcctcgac attccagcct tgttcccacc tccagcactt tgcatatgct gttccctttg 3780
ccaagaatgc tcttccccta ccctgtgcat ggctgagttc tgcagaccct caggccttgg 3840

cttcaacgtt gcctcgtcca agaggccttc ctcgactact ttacttgtgg agttcctcta 3900

tcacaaggcc tctgttcttt cccttcatgg agaatttgcc actgcatatc catttgtgta 3960

atttacttgt tggttgactg tgcctcccac tcgagtgtaa gctcatgagg ccaggtgcca 4020

tgcctggttc agtctccact ctgtacccag cattgagcac agggcctggt ccatagttgg 4080

cgttcaataa atacttgttg aagaagtgaa ctgaaaaaaa aaaaaaaaaa a 4131

<210> 16
<211> 2969
<212> DNA
<213> Homo sapiens

<400> 16

actttagctc agacctttct tttaaccttg cctatcatgt ttcgagtcag aatttaaata 60
ctgtgcagtt taagctacaa tacgcttggc ctataacttg gttccaggca tttatattta 120
tgtcactttt gtctacttat tatactaaca aggtggaaaa agcaatccca gtctctccaa 180
aagacaagat gtgaaatgga gaagtatctg acacctcagc ttcctccagt tcctataatt 240
ccagagcata aaaagtatag acgagacagt gcctcagtcg tagaccagtt cttcactgac 300
actgaagggt taccttacag tatcaacatg aacgtcttcc tccctgacat cactcacctg 360
agaactggcc tctacaaatc ccagagaccg tgcgtaacac acatcaagac agaacctgtt 420
gccattttca gccaccagag tgaaacgact gcccctcctc cggccccgac ccaggccctc 480
cctgagttca ccagtatatt cagctcacac cagaccgcag ctccagaggt gaacaatatt 540
ttcatcaaac aagaacttcc tacaccagat cttcatcttt ctgtccctac ccagcagggc 600
cacctgtacc agctactgaa tacaccggat ctagatatgc ccagttctac aaatcagaca 660
gcagcaatgg acactcttaa tgtttctatg tcagctgcca tggcaggcct taacacacac 720
acctctgctg ttccgcagac tgcagtgaaa caattccagg gcatgccccc ttgcacatac 780
acaatgccaa gtcagtttct tccacaacag gccacttact ttcccccgtc accaccaagc 840
tcagagcctg gaagtccaga tagacaagca gagatgctcc agaatttaac cccacctcca 900
tcctatgctg ctacaattgc ttctaaactg gcaattcaca atccaaattt acccaccacc 960
ctgccagtta actcacaaaa catccaacct gtcagataca atagaaggag taaccccgat 1020
ttggagaaac gacgcatcca ctactgcgat taccctggtt gcacaaaagt ttataccaag 1080
tcttctcatt taaaagctca cctgaggact cacactggtg aaaagccata caagtgtacc 1140
tgggaaggct gcgactggag gttcgcgcga tcggatgagc tgacccgcca ctaccggaag 1200
cacacaggcg ccaagccctt ccagtgcggg gtgtgcaacc gcagcttctc gcgctctgac 1260
cacctggccc tgcatatgaa gaggcaccag aactgagcac tgcccgtgtg acccgttcca 1320
ggtcccctgg gctccctcaa atgacagacc taactattcc tgtgtaaaaa caacaaaaac 1380

aaacaaaagc aagaaaacca caactaaaac tggaaatgta tattttgtat atttgagaaa 1440

acagggaata cattgtatta ataccaaagt gtttggtcat tttaagaatc tggaatgctt 1500

gctgtaatgt atatggcttt actcaagcag atctcatctc atgacaggca gccacgtctc 1560

aacatgggta aggggtgggg gtggagggga gtgtgtgcag cgtttttacc taggcaccat 1620

catttaatgt gacagtgttc agtaaacaaa tcagttggca ggcaccagaa gaagaatgga 1680

ttgtatgtca agattttact tggcattgag tagttttttt caatagtagg taattcctta 1740

gagatacagt atacctggca attcacaaat agccattgaa caaatgtgtg ggtttttaaa 1800

aattatatac atatatgagt tgcctatatt tgctattcaa aattttgtaa atatgcaaat 1860

cagctttata ggtttattac aagtttttta ggattctttt ggggaagagt cataattctt 1920

ttgaaaataa ccatgaatac acttacagtt aggatttgtg gtaaggtacc tctcaacatt 1980

accaaaatca tttctttaga gggaaggaat aatcattcaa atgaacttta aaaaagcaaa 2040

tttcatgcac tgattaaaat aggattattt taaatacaaa aggcatttta tatgaattat 2100

aaactgaaga gcttaaagat agttacaaaa tacaaaagtt caacctctta caataagcta 2160

aacgcaatgt catttttaaa aagaaggact tagggtgtcg ttttcacata tgacaatgtt 2220

gcatttatga tgcagtttca agtaccaaaa cgttgaattg atgatgcagt tttcatatat 2280

cgagatgttc gctcgtgcag tactgttggt taaatgacaa tttatgtgga ttttgcatgt 2340

aatacacagt gagacacagt aattttatct aaattacagt gcagtttagt taatctatta 2400

atactgactc agtgtctgcc tttaaatata aatgatatgt tgaaaactta aggaagcaaa 2460

tgctacatat atgcaatata aaatagtaat gtgatgctga tgctgttaac caaagggcag 2520

aataaataag caaaatgcca aaaggggtct taattgaaat gaaaatttaa ttttgttttt 2580

aaaatattgt ttatctttat ttattttgtg gtaatatagt aagttttttt agaagacaat 2640

tttcataact tgataaatta tagttttgtt tgttagaaaa gttgctctta aaagatgtaa 2700

atagatgaca aacgatgtaa ataattttgt aagaggcttc aaaatgttta tacgtggaaa 2760

cacacctaca tgaaaagcag aaatcggttg ctgttttgct tctttttccc tcttattttt 2820

gtattgtggt catttcctat gcaaataatg gagcaaacag ctgtatagtt gtagaatttt 2880

ttgagagaat gagatgttta tatattaacg acaatttttt ttttggaaaa taaaaagtgc 2940

ctaaaagatg taaaaaaaaa aaaaaaaaa 2969

<210> 17
<211> 4518
<212> DNA
<213> Homo sapiens

<400> 17

ggagtttatt cataacgcgc tctccaagta tacgtggcaa tgcgttgctg ggttatttta 60

atcattctag gcatcgtttt cctccttatg cctctatcat tcctccctat ctacactaac 120

atcccacgct ctgaacgcgc gcccattaat acccttcttt cctccactct ccctgggact 180

cttgatcaaa gcgcggccct ttccccagcc ttagcgaggc gccctgcagc ctggtacgcg 240

cgtggcgtgg cggtgggcgc gcagtgcgtt ctcggtgtgg agggcagctg ttccgcctgc 300

gatgatttat actcacagga caaggatgcg gtttgtcaaa cagtactgct acggaggagc 360

agcagagaaa gggagagggt ttgagaggga gcaaaagaaa atggtaggcg cgcgtagtta 420

attcatgcgg ctctcttact ctgtttacat cctagagcta gagtgctcgg ctgcccggct 480

gagtctcctc cccaccttcc ccaccctccc caccctcccc ataagcgccc ctcccgggtt 540

cccaaagcag agggcgtggg ggaaaagaaa aaagatcctc tctcgctaat ctccgcccac 600

cggcccttta taatgcgagg gtctggacgg ctgaggaccc ccgagctgtg ctgctcgcgg 660

ccgccaccgc cgggccccgg ccgtccctgg ctcccctcct gcctcgagaa gggcagggct 720

tctcagaggc ttggcgggaa aaagaacgga gggagggatc gcgctgagta taaaagccgg 780

ttttcggggc tttatctaac tcgctgtagt aattccagcg agaggcagag ggagcgagcg 840

ggcggccggc tagggtggaa gagccgggcg agcagagctg cgctgcgggc gtcctgggaa 900

gggagatccg gagcgaatag ggggcttcgc ctctggccca gccctcccgc tgatccccca 960

gccagcggtc cgcaaccctt gccgcatcca cgaaactttg cccatagcag cgggcgggca 1020

ctttgcactg gaacttacaa cacccgagca aggacgcgac tctcccgacg cggggaggct 1080

attctgccca tttggggaca cttccccgcc gctgccagga cccgcttctc tgaaaggctc 1140

tccttgcagc tgcttagacg ctggattttt ttcgggtagt ggaaaaccag cagcctcccg 1200

cgacgatgcc cctcaacgtt agcttcacca acaggaacta tgacctcgac tacgactcgg 1260

tgcagccgta tttctactgc gacgaggagg agaacttcta ccagcagcag cagcagagcg 1320

agctgcagcc cccggcgccc agcgaggata tctggaagaa attcgagctg ctgcccaccc 1380

cgcccctgtc ccctagccgc cgctccgggc tctgctcgcc ctcctacgtt gcggtcacac 1440

ccttctccct tcggggagac aacgacggcg gtggcgggag cttctccacg gccgaccagc 1500

tggagatggt gaccgagctg ctgggaggag acatggtgaa ccagagtttc atctgcgacc 1560

cggacgacga gaccttcatc aaaaacatca tcatccagga ctgtatgtgg agcggcttct 1620

cggccgccgc caagctcgtc tcagagaagc tggcctccta ccaggctgcg cgcaaagaca 1680

gcggcagccc gaaccccgcc cgcggccaca gcgtctgctc cacctccagc ttgtacctgc 1740

aggatctgag cgccgccgcc tcagagtgca tcgacccctc ggtggtcttc ccctaccctc 1800

tcaacgacag cagctcgccc aagtcctgcg cctcgcaaga ctccagcgcc ttctctccgt 1860

cctcggattc tctgctctcc tcgacggagt cctccccgca gggcagcccc gagcccctgg 1920

tgctccatga ggagacaccg cccaccacca gcagcgactc tgaggaggaa caagaagatg 1980
aggaagaaat cgatgttgtt tctgtggaaa agaggcaggc tcctggcaaa aggtcagagt 2040
ctggatcacc ttctgctgga ggccacagca aacctcctca cagcccactg gtcctcaaga 2100
ggtgccacgt ctccacacat cagcacaact acgcagcgcc tccctccact cggaaggact 2160
atcctgctgc caagagggtc aagttggaca gtgtcagagt cctgagacag atcagcaaca 2220
accgaaaatg caccagcccc aggtcctcgg acaccgagga gaatgtcaag aggcgaacac 2280
acaacgtctt ggagcgccag aggaggaacg agctaaaacg gagctttttt gccctgcgtg 2340
accagatccc ggagttggaa aacaatgaaa aggcccccaa ggtagttatc cttaaaaaag 2400
ccacagcata catcctgtcc gtccaagcag aggagcaaaa gctcatttct gaagaggact 2460
tgttgcggaa acgacgagaa cagttgaaac acaaacttga acagctacgg aactcttgtg 2520
cgtaaggaaa agtaaggaaa acgattcctt ctaacagaaa tgtcctgagc aatcacctat 2580
gaacttgttt caaatgcatg atcaaatgca acctcacaac cttggctgag tcttgagact 2640
gaaagattta gccataatgt aaactgcctc aaattggact ttgggcataa aagaactttt 2700
ttatgcttac catctttttt ttttctttaa cagatttgta tttaagaatt gtttttaaaa 2760
aattttaaga tttacacaat gtttctctgt aaatattgcc attaaatgta aataacttta 2820
ataaaacgtt tatagcagtt acacagaatt tcaatcctag tatatagtac ctagtattat 2880
aggtactata aaccctaatt ttttttattt aagtacattt tgctttttaa agttgatttt 2940
tttctattgt ttttagaaaa aataaaataa ctggcaaata tatcattgag ccaaatctta 3000
agttgtgaat gttttgtttc gtttcttccc cctcccaacc accaccatcc ctgtttgttt 3060
tcatcaattg ccccttcaga gggtggtctt aagaaaggca agagttttcc tctgttgaaa 3120
tgggtctggg ggccttaagg tctttaagtt cttggaggtt ctaagatgct tcctggagac 3180
tatgataaca gccagagttg acagttagaa ggaatggcag aaggcaggtg agaaggtgag 3240
aggtaggcaa aggagataca agaggtcaaa ggtagcagtt aagtacacaa agaggcataa 3300
ggactgggga gttgggagga aggtgaggaa gaaactcctg ttactttagt taaccagtgc 3360
cagtcccctg ctcactccaa acccaggaat tctgcccagt tgatggggac acggtgggaa 3420
ccagcttctg ctgccttcac aaccaggcgc cagtcctgtc catgggttat ctcgcaaacc 3480
ccagaggatc tctgggagga atgctactat taaccctatt tcacaaacaa ggaaatagaa 3540
gagctcaaag aggttatgta acttatctgt agccacgcag ataatacaaa gcagcaatct 3600
ggacccattc tgttcaaaac acttaaccct tcgctatcat gccttggttc atctgggtct 3660
aatgtgctga gatcaagaag gtttaggacc taatggacag actcaagtca taacaatgct 3720
aagctctatt tgtgtcccaa gcactcctaa gcattttatc cctaactcta catcaacccc 3780
atgaaggaga tactgttgat ttccccatat tagaagtaga gagggaagct gaggcacaca 3840

```
aagactcatc cacatgccca agattcactg atagggaaaa gtggaagcga gatttgaacc     3900
caggctgttt actcctaacc tgtccaagcc acctctcaga cgacggtagg aatcagctgg     3960
ctgcttgtga gtacaggagt tacagtccag tgggttatgt tttttaagtc tcaacatcta     4020
agcctggtca ggcatcagtt cccctttttt tgtgatttat tttgttttta ttttgttgtt     4080
cattgtttaa tttttccttt tacaatgaga aggtcaccat cttgactcct accttagcca     4140
tttgttgaat cagactcatg acggctcctg ggaagaagcc agttcagatc ataaaataaa     4200
acatatttat tctttgtcat gggagtcatt attttagaaa ctacaaactc tccttgcttc     4260
catccttttt tacatactca tgacacatgc tcatcctgag tccttgaaaa ggtatttttg     4320
aacatgtgta ttaattataa gcctctgaaa acctatggcc caaaccagaa atgatgttga     4380
ttatataggt aaatgaagga tgctattgct gttctaatta cctcattgtc tcagtctcaa     4440
agtaggtctt cagctccctg tactttggga ttttaatcta ccaccaccca taaatcaata     4500
aataattact ttctttga                                                   4518
```

<210> 18
<211> 3125
<212> DNA
<213> Homo sapiens

<400> 18

```
ccgcaaccag agccgccgcc acggtgagtg gctggattca gacccctggg tggccgggac      60
aagagaaaag agggaggagg gcctttagcg gacagcgcct ggggctggag agcagcagct     120
gcacacagcc ggaaagggcg cgcaggcgac gacactcgga tccacgtcga caccgttgta     180
caaagatacg cggacccgcg ggcgtctaaa attctgggaa gcagaacctg gccggagcca     240
ctagacagag ccgggcctag cccagagaca tggagagttg ctacaaccca ggtctggatg     300
gtattattga atatgatgat ttcaaattga actcctccat tgtggaaccc aaggagccag     360
ccccagaaac agctgatggc ccctacctgg tgatcgtgga acagcctaag cagagaggct     420
tccgatttcg atatggctgt gaaggcccct cccatggagg actgcccggt gcctccagtg     480
agaagggccg aaagacctat cccactgtca agatctgtaa ctacgaggga ccagccaaga     540
tcgaggtgga cctggtaaca cacagtgacc cacctcgtgc tcatgcccac agtctggtgg     600
gcaagcaatg ctcggagctg gggatctgcg ccgtttctgt ggggcccaag gacatgactg     660
cccaatttaa caacctgggt gtcctgcatg tgactaagaa gaacatgatg gggactatga     720
tacaaaaact tcagaggcag cggctccgct ctaggcccca gggccttacg gaggccgagc     780
agcgggagct ggagcaagag gccaaagaac tgaagaaggt gatggatctg agtatagtgc     840
ggctgcgctt ctctgccttc cttagagcca gtgatggctc cttctccctg cccctgaagc     900
cagtcatctc ccagcccatc catgacagca aatctccggg ggcatcaaac ctgaagattt     960
```

ctcgaatgga caagacagca ggctctgtgc ggggtggaga tgaagtttat ctgctttgtg 1020
acaaggtgca gaaagatgac attgaggttc ggttctatga ggatgatgag aatggatggc 1080
aggcctttgg ggacttctct cccacagatg tgcataaaca gtatgccatt gtgttccgga 1140
caccccccta tcacaagatg aagattgagc ggcctgtaac agtgtttctg caactgaaac 1200
gcaagcgagg aggggacgtg tctgattcca aacagttcac ctattaccct ctggtggaag 1260
acaaggaaga ggtgcagcgg aagcggagga aggccttgcc caccttctcc cagcccttcg 1320
ggggtggctc ccacatgggt ggaggctctg gggtgcagc cgggggctac ggaggagctg 1380
gaggaggtgg cagcctcggt ttcttcccct cctccctggc ctacagcccc taccagtccg 1440
gcgcgggccc catgggctgc tacccgggag gcgggggcgg ggcgcagatg gccgccacgg 1500
tgcccagcag gactccggg gaggaagccg cggagccgag cgccccctcc aggaccccc 1560
agtgcgagcc gcaggccccg gagatgctgc agcgagctcg agagtacaac gcgcgcctgt 1620
tcggcctggc gcagcgcagc gcccgagccc tactcgacta cggcgtcacc gcggacgcgc 1680
gcgcgctgct ggcgggacag cgccacctgc tgacggcgca ggacgagaac ggagacacac 1740
cactgcacct agccatcatc cacgggcaga ccagtgtcat tgagcagata gtctatgtca 1800
tccaccacgc ccaggacctc ggcgttgtca acctcaccaa ccacctgcac cagacgcccc 1860
tgcacctggc ggtgatcacg gggcagacga gtgtggtgag ctttctgctg cgggtaggtg 1920
cagacccagc tctgctggat cggcatggag actcagccat gcatctggcg ctgcgggcag 1980
gcgctggtgc tcctgagctg ctgcgtgcac tgcttcagag tggagctcct gctgtgcccc 2040
agctgttgca tatgcctgac tttgagggac tgtatccagt acacctggcg gtccgagccc 2100
gaagccctga gtgcctggat ctgctggtgg acagtggggc tgaagtggag gccacagagc 2160
ggcagggggg acgaacagcc ttgcatctag ccacagagat ggaggagctg gggttggtca 2220
cccatctggt caccaagctc cgggccaacg tgaacgctcg cacctttgcg ggaaacacac 2280
ccctgcacct ggcagctgga ctggggtacc cgaccctcac ccgcctcctt ctgaaggctg 2340
gtgctgacat ccatgctgaa aacgaggagc ccctgtgccc actgccttca ccccctacct 2400
ctgatagcga ctcggactct gaagggcctg agaaggacac ccgaagcagc ttccggggcc 2460
acacgcctct tgacctcact tgcagcacca aggtgaagac cttgctgcta aatgctgctc 2520
agaacaccat ggagccaccc ctgaccccgc ccagcccagc agggccggga ctgtcacttg 2580
gtgatacagc tctgcagaac ctggagcagc tgctagacgg gccagaagcc cagggcagct 2640
gggcagagct ggcagagcgt ctggggctgc gcagcctggt agacacgtac cgacagacaa 2700
cctcacccag tggcagcctc ctgcgcagct acgagctggc tggcggggac ctggcaggtc 2760
tactggaggc cctgtctgac atgggcctag aggagggagt gaggctgctg aggggtccag 2820

```
aaacccgaga caagctgccc agcacagcag aggtgaagga agacagtgcg tacgggagcc      2880
agtcagtgga gcaggaggca gagaagctgg gcccaccccc tgagccacca ggagggctct      2940
gccacgggca cccccagcct caggtgcact gacctgctgc ctgcccccag ccccccttccc      3000
ggaccccctg tacagcgtcc ccacctattt caaatcttat ttaacacccc acacccaccc      3060
ctcagttggg acaaataaag gattctcatg ggaaggggag gacccctcct tcccaactta      3120
tggca                                                                  3125
```

<210> 19
<211> 2529
<212> DNA
<213> Homo sapiens

<400> 19

gctgatagca cagttctgtc cagagaagga aggcagaata aacttattca ttcccaggaa 60
ctcttggggt aggtgtgtgt ttttcacatc ttaaaggctc acagaccctg cgctggacaa 120
atgttccatt cctgaaggac ctctccagaa tccggattgc tgaatcttcc ctgttgccta 180
gaagggctcc aaaccacctc ttgacaatgg gaaactgggt ggttaaccac tggttttcag 240
ttttgtttct ggttgtttgg ttagggctga atgttttcct gtttgtggat gccttcctga 300
aatatgagaa ggccgacaaa tactactaca caagaaaaat ccttgggtca acattggcct 360
gtgcccgagc gtctgctctc tgcttgaatt ttaacagcac gctgatcctg cttcctgtgt 420
gtcgcaatct gctgtccttc ctgaggggca cctgctcatt ttgcagccgc acactgagaa 480
agcaattgga tcacaacctc accttccaca agctggtggc ctatatgatc tgcctacata 540
cagctattca catcattgca cacctgttta actttgactg ctatagcaga agccgacagg 600
ccacagatgg ctcccttgcc tccattctct ccagcctatc tcatgatgag aaaaaggggg 660
gttcttggct aaatcccatc cagtcccgaa acacgacagt ggagtatgtg acattcacca 720
gcattgctgg tctcactgga gtgatcatga caatagcctt gattctcatg gtaacttcag 780
ctactgagtt catccggagg agttattttg aagtcttctg gtatactcac caccttttta 840
tcttctatat ccttggctta gggattcacg gcattggtgg aattgtccgg ggtcaaacag 900
aggagagcat gaatgagagt catcctcgca agtgtgcaga gtcttttgag atgtgggatg 960
atcgtgactc ccactgtagg cgccctaagt ttgaagggca tccccctgag tcttggaagt 1020
ggatccttgc accggtcatt ctttatatct gtgaaaggat cctccggttt taccgctccc 1080
agcagaaggt tgtgattacc aaggttgtta tgcacccatc caaagttttg gaattgcaga 1140
tgaacaagcg tggcttcagc atggaagtgg ggcagtatat ctttgttaat tgcccctcaa 1200
tctctctcct ggaatggcat ccttttactt tgacctctgc tccagaggaa gatttcttct 1260
ccattcatat ccgagcagca ggggactgga cagaaaatct cataagggct ttcgaacaac 1320

aatattcacc aattcccagg attgaagtgg atggtccctt tggcacagcc agtgaggatg 1380
ttttccagta tgaagtggct gtgctggttg gagcaggaat tggggtcacc ccctttgctt 1440
ctatcttgaa atccatctgg tacaaattcc agtgtgcaga ccacaacctc aaaacaaaaa 1500
agatctattt ctactggatc tgcagggaga caggtgcctt ttcctggttc aacaacctgt 1560
tgacttccct ggaacaggag atggaggaat taggcaaagt gggttttcta aactaccgtc 1620
tcttcctcac cggatgggac agcaatattg ttggtcatgc agcattaaac tttgacaagg 1680
ccactgacat cgtgacaggt ctgaaacaga aaacctcctt tgggagacca atgtgggaca 1740
atgagttttc tacaatagct acctcccacc ccaagtctgt agtgggagtt ttcttatgtg 1800
gccctcggac tttggcaaag agcctgcgca aatgctgtca ccgatattcc agtctggatc 1860
ctagaaaggt tcaattctac ttcaacaaag aaaatttttg agttatagga ataaggacgg 1920
taatctgcat tttgtctctt tgtatcttca gtaatttact tggtctcgtc aggtttgagc 1980
agtcacttta ggataagaat gtgcctctca agccttgact ccctggtatt ctttttttga 2040
ttgcattcaa cttcgttact tgagcttcag caacttaaga acttctgaag ttcttaaagt 2100
tctgaagttc ttaaagccca tggatccttt ctcagaaaaa taactgtaaa tctttctgga 2160
cagccatgac tgtagcaagg cttgatagca gaggtttggt ggttcagagt tatacaacta 2220
atcccaggtg attttatcaa ttccagtgtt accatctcct gagttttggt ttgtaatctt 2280
ttgtccctcc cacccccaca gaagatttct aagtagggtg actttttaaa taaaaattta 2340
ttgaataatt aatgataaaa cataataata aacataaata ataaacaaaa ttaccgagaa 2400
ccccatcccc atataacacc aacagtgtac atgtttactg tcacttttga tatggtctta 2460
tccagtgtga acagcaattt attcttattt ttgctcatca aaaaataaag gattttcttc 2520
ttcacttga 2529

<210> 20
<211> 2639
<212> DNA
<213> Homo sapiens

<400> 20

```
ggcgccttgg gaccgcgtgg gagccgcagc cgaaccgagt agggaccggg accgcgcggc      60
gccgccgtcc ccggccgggc ccggcccccg cgagccgagc gcgcgccccc gtcgcccacc     120
cgggcgcggc tggatgcggc ggggtccccg cggcggcgac ccccggcccc gagcgcccgg     180
agcgcccaga ggcggcgtgc ggggcccggg gacgccgcgc cctccatgcg ccgaggcgcg     240
ccccgagaca gccgggggcc cgcgccgcag ccgccgcccg cgctgagccc cggcccggcc     300
cgcggcccgc gcccggcggc agcatgagcc aggccgagct gtccacctgc tccgcgccgc     360
agacgcagcg catcttccag gaggctgtgc gcaaggcaa cacgcaggag ctgcagtcgc      420
```

tgctgcagaa catgaccaac tgcgagttca acgtgaactc gttcgggccc gagggccaga 480

cggcgctgca ccagtcggtc atcgacggca acctggagct cgtgaagctg ctggtcaagt 540

tcggcgccga catccgcctg gccaaccgcg acggctggag cgcgctgcac atcgccgcgt 600

tcggtggcca ccaggacatc gtgctctatc tcatcaccaa ggcgaagtac gcggccagcg 660

gccggtgatg cccgccggga ccccggaccc cggccctgcg cccgcgtcgt ctctgctgta 720

ccttcccgcc aactacctcg gtgcgcgccc ggctcgcagg ccccgccaga aggcccgtgg 780

ccacggcgaa tacggcgcgt gcgtcccggc cccagggtcc ggcagccccg ccggccgagc 840

gcctccctgc ggcctagccg ggcccggccg ggccggagca gcttcccacg gcccccaccc 900

gctcgcctgc ccgccgcctc gcgggtgggg gcggggcgcg ggctccagcc ccttttgaaa 960

tttgagtctc gcaaccagca agttcggaat cccgagatac cggatcctct gcgcaaaatg 1020

ttttctcccg aaggtgaaag gcgggcggcg ggagccgaag gcggactcgg agcgctccgc 1080

cgccgccttc aggacccgcc cgcaggcccg ggacgcgccg atgccggctg cagccgagga 1140

gcagccccga ggtccgaggt ccgcgccgct ggcgcgcggc cgaggagacg ctcggctgtt 1200

cgctgttgct ggtgttctaa actatttatc ttgtgtgtgt acatttgtgg gtggagtttg 1260

tgcgcctggt ttttttgttt ggaaaacact gcgtggtcaa tgtggttatg ggggggagtg 1320

atgcattttt ttctagtctt aaaactaaaa acttgagtct accatttctt ggttgcactg 1380

aaaataccgc ccagcctgat ggtgttcccg tgctgtccct ccccccttccc ttctccccgc 1440

gtctacctcc ccaccccgtt ctgttccccc tccctccttc tccctctccc tcaaatccgt 1500

gagttttgga agccccaggg cctctctccc ccgcccctcc tggatgaggc caccatcccc 1560

caaaccggct tgttttgcag tttccccagg atcctggaag ctcgctggcg ctcgagggtg 1620

gcggggacac gggggggtgg gtgaaggttc gttacctttt ctagtgcgtt ctatcatagt 1680

taacggttgc acactttttt aaaaaaagta aatggatttg ccacaattaa atgtcataac 1740

atttatgaca gaatataaaa tattaacata ttttaagcca agttttaggt gtattttttg 1800

aatcttggtt ataaacccaa ttttaaaggg cgatgtatcc agcgttgtga aggcaacaga 1860

gtgtacccat atttatattt ttataaaata cctataagac tgtgaatctc ttgtgctaat 1920

ggctgagtta attgaaggat cgttttgccc ctttttagcc tcccagagct tcgaggactc 1980

aattcgaacc cgaaatcctg ccgtggggga ggggttgcgt cgagacctgg gcccggggag 2040

gttctcctgc gtcactttct gtcctgaaag gcgcccttcc tggtttctgt ggctccaatt 2100

ttctatgcag ccccacaccc cttgttgttt tgatcctgag aaataaaagg gaggctgaat 2160

tattcaaatt taaatgaggt ttccccttca tggaagtgct gctgaccctt cgtgcagaaa 2220

tggggagcac ttgaggacac aggtgggtgg aggccctttg tgcgtggctg gtcgtattcg 2280

ggcagccctc cgtcgctttt tataaaactt tgtgtgagaa gaatatattg ataatgtcag 2340

```
tgaaacaagc agacattgaa atggaggcac agattactcc acaaggagtt cttctgtata     2400
tttttctag atgcaaatac ctttttaatt atgttaatta atgttaagac tttctaggct     2460
tatatcgaag ctgtgtgtgg gtcacggggt gatcactgct aactggataa agtttgtgca     2520
gcacattcct gagtgtacga tattgacctg tagcccagcg tgaaaaattt ataaataaat     2580
ttttcattga tctttttata ttaaaaaaaa gtttcttggt caaaaaaaaa aaaaaaaaa      2639
```

<210> 21
<211> 6918
<212> DNA
<213> Homo sapiens

<400> 21

```
aaagtttgca ttgcaatccc cctgccttcc tctcctttct cccgatcaat gcatatttgc      60
aaaaggatta agccacagat ttaagcgccg ggagcccatt tctgccttgc aaaggagacc     120
ggactgaaaa acctaaagcc agctctgatt tcttttcgcc aagtgggaag gtggtttatt     180
tttcttgctt tttggagtca acacccttcc ccaccagccc ttatccccac cctcaccccg     240
caaccccttc acgccccctc cccctccccc tcctcatcct cccaccatcc tctaaagagg     300
caaagggatt tttttttct tttggtcttc tttttcccc cttccctgtt tatcctgaaa       360
aggatttgaa gacaagcttg aaggataaaa agccttggtg cttcccagga gccgagccga     420
ggagcagaag aggaagagcc gggggctgcc gtagcctttg gagatggacg agcagcccag     480
gctgatgcat tcccatgctg ggtcgggat ggccggacac cccggcctgt cccagcactt      540
gcaggatggg gccggaggga ccgaggggga gggcgggagg aagcaggaca ttggagacat     600
tttacagcaa attatgacca tcacagacca gagtttggat gaggcgcagg ccagaaaaca     660
tgctttaaac tgccacagaa tgaagcctgc cttgtttaat gtgttgtgtg aaatcaaaga     720
aaaaacagtt ttgagtatcc gaggagccca ggaggaggaa cccacagacc cccagctgat     780
gcggctggac aacatgctgt tagcggaagg cgtggcgggg cctgagaagg gcggagggtc     840
ggcggcagcg gcggcagcgg cggcggcttc tggaggggca ggttcagaca actcagtgga     900
gcattcagat tacagagcca aactctcaca gatcagacaa atctaccata cggagctgga     960
gaaatacgag caggcctgca acgagttcac cacccacgtg atgaatctcc tgcgagagca    1020
aagccggacc aggcccatct ccccaaagga gattgagcgg atggtcagca tcatccaccg    1080
caagttcagc tccatccaga tgcagctcaa gcagagcacg tgcgaggcgg tgatgatcct    1140
gcgttcccga tttctggatg cgcggcggaa gagacggaat ttcaacaagc aagcgacaga    1200
aatcctgaat gaatatttct attcccatct cagcaaccct taccccagtg aggaagccaa    1260
agaggagtta gccaagaagt gtggcatcac agtctcccag gtatcaaact ggtttggaaa    1320
taagcgaatc cggtacaaga agaacatagg taaatttcaa gaggaagcca atatttatgc    1380
```

tgccaaaaca gctgtcactg ctaccaatgt gtcagcccat ggaagccaag ctaactcgcc 1440
ctcaactccc aactcggctg gttcttccag ttcttttaac atgtcaaact ctggagattt 1500
gttcatgagc gtgcagtcac tcaatgggga ttcttaccaa ggggcccagg ttggagccaa 1560
cgtgcaatca caggtggata cccttcgcca tgttatcagc cagacaggag gatacagtga 1620
tggactcgca gccagtcaga tgtacagtcc gcagggcatc agtgctaatg gaggttggca 1680
ggatgctact accccttcat cagtgacctc ccctacagaa ggccctggca gtgttcactc 1740
tgatacctcc aactgatctc ccagcaatcg catcccggct gaccctgtgc cccagttggg 1800
gcaggggcag gagggagggt ttctctccca acgctgaagc ggtcagactg gaggtcgaag 1860
caatcagcaa acacaataag agtctccttc tcttctcttc tttgggatgc tatttcagcc 1920
aatctggaca cttctttata ctctcttccc ttttttttct gggtagaagc caccccttccc 1980
tgcctccagc tgtcagcctg gttttcgtca tcttccctgc ccctgtgcct ctgtcctaga 2040
ctcccggggt ccccgccctc tctcatatca ctgaaggata ttttcaacaa ttagaggaat 2100
ttaaagagga aaaaaattac aaagaaaata ataaaagtgt ttgtacgttt tcatgctggt 2160
ggtttgagga gccaaattta cctcactcga atccctcact ccctatgtta acaggcaatc 2220
cttctctgtt tctcttatta ctctcactac ctcttagcag gaatactcca cattgcccta 2280
ttcattccag gcctccctgc ttcctcttgc tcttcctccc tggggacagt actgattgga 2340
acactttcct cctcttcctt cctagcccca gctattcact ggggactgtc atagctggga 2400
ttctaaaggt gccacatttt tcagtttcat ctccactagg ttggttcccg ggcaggaagt 2460
caggcagcag ggaaggacac gggaacagca ggtggagaat tcctacagtc tttcttaccc 2520
tgctagcaat agctctcagt ttcagaggca cagtctttgg agaccattca gcactgagaa 2580
agcaatattt agaacctatt gcaaaactgg gcctgagtta ggcatggtga tgaatgcatc 2640
agcaaggaat agaaagttct tatcgtgaaa cccttcaacc tcaactatgc cttcatagac 2700
acacacgttc atgcacatgt aggcacatgt accatctcac atcttcactt tcccgagatg 2760
ccatatacaa ttacctacat taataactgt agcactatgc cttttgagcc cgagagaggg 2820
aattagtgac tctaagtgaa ggtcactgac acagagaagc agtatgtgtc tggggcttcc 2880
aggacctgca ggcccactag cgtgcactta ccagaatggc atacacagga cctgatcatg 2940
aggaagacca ggtttccagt gtaaactact cttgttccca ccacctctgg agcactcagg 3000
gagccccata cagtacttac aatgtcttta atggacttga ttctgtttaa tttttttgttt 3060
tatattaggc acactgtatt aattttccaa aatgttatac cacactatgt tcttggtcct 3120
gacctattgc tctggaggaa agagttgtat aagaacgtgg ctcatgtgaa cttttgctag 3180
cttcatttga ggacctgaga atcatgggga aagggaaggt aatgttttca ttgaaatcat 3240

cacagtgatt tttattccct gggaacacag cgtgtactaa aaatacatga gaaaatagca 3300
tgtatatgaa agctattctc aaaagtcacc tgagctcacc atcttcatag ccaaccctac 3360
cagttataaa gatggcagct ctatcacttg attaagtggg aggtggtcaa atattttggt 3420
gcctcatttt cttcatctgt gagatgggaa ctgttatgcc tggcttacta agagtcttgt 3480
gagagactga gaagttgatt ttgttcatat ccaatctgta aatgcgaagt caggggaagt 3540
aatgtccctg aaataaacgg gttcatgcca tctagggaca ataaatggtt ttcttgttgt 3600
aacttctggt taatatcagt accttgatgt catcaccgtg atgacaaaga gaagagttat 3660
tgttgatctt cttggttttg gtctgtctct tttcttagga taaagaaaaa cttccaaact 3720
agaaaaacag gccctggttc ccttagtttg cacttgaacc caatatgttg ccttgtacat 3780
acttggtccc tgtcacattg actgcttggg aggcttccag ggagaagtat gagaccctga 3840
ggggtgagaa tgggcagcta gcaagaacat ggaaattctg cttggcacta cagtcataaa 3900
tagaaaacac tgtgtgtgct caggggagca ggggatgcca ctgaagaaac tcaagggaat 3960
gtgtatttga aggaaatgca aaaactaagt atttagcaaa atgaaattat gccttgatga 4020
ctaaaaggca ctagaaaggt tgtgtctact aacttcagcc ctaatcagaa cagatgccta 4080
gaaggagcat ttttgtgaca acttcatagt gattagaatc agtggagaac tccatcttag 4140
tggcaggaat ataatgaaac tacccacgca agaacatggt tgaatcacat ttgcttgact 4200
tagggcaaag tacgaaagag agacaaaagg gttctcttgg aaacaagaag agtgactcca 4260
gatgtggcct gaataattgc catgttaagt taatgcaaaa gatcagaaca gggctacatt 4320
tgcacaggca gtttctctcc gggccgtagt tttcactgat gatcaccttt cacagcattt 4380
tccccaacca gcatttcact tagtcttctc tatacccagc acctccccca gcacccccgg 4440
caagcccact atcacttccg acttccaacg tggcatccgt gagatctgtc cacattaggc 4500
gaagcaggag aacactgaga gcagcaggat gggtttggaa agagcatgcc tctggaaaca 4560
cagcttcctg ggaattcaca tgaggccagt cctacagaga gcaagatgca ccccaggatt 4620
tcttcatttt ctaatagatg tgggagtgct ccattttccc cgacagcgaa tttcccctga 4680
gaaacgatac tagaccctgg gtttgcccac cttgtaactc ttccttatct cctccttttc 4740
atccctaatc catcctccct ctggcatgga attgacgccc gtgcagtaca tttgccaagt 4800
ggcaccttct ttcaatttat gttttatttt gctatggtgg tgattcttta tttgctggtt 4860
gtcttttctc acacatcttt ctctctgtct ctctctttcc tgctctttgt ttttctgccc 4920
agaaaaacct gacttcgata ccaaaaaaga tgaaactaca gaaactcaaa tttaaaaaaa 4980
actttaaaag aaacaaaaaa atactcaacg attctttcag ctttattaac attttccatt 5040
gtttcttgcg acttgtgtct cgttctttgt agtattgatg atgaacattt gataatgaat 5100
gttcttgtat attcagataa agaaaaaaaa aaccaaaaaa gcggtctgaa tttaatagtg 5160

```
tttataataa aaattttaaa aatgaccctc atagcacgca aaacaggatg gggaatttcc     5220
cctcttcttt ctgtgacaat gcgcatcatt cctgcattag tttttaacac cagactacct     5280
acattcatca tttccctcat ttttctttta ttttcttgca tttgtgaatt agttcaagaa     5340
tgctagaaaa gtgtcgagtt gtgcacatcc atttcttgtt tcacaatgtt taaaagtgac     5400
agtaattcat tttgtaaact aaaaaaaaaa aaaaaaaggt tggaatagtg agcataatag     5460
gtacaaccta acacattatt atgtttatta actttgagac ccagaaataa attcttttct     5520
tttcttgatt cttgctctta aaaatacaaa aaaaaaaatg ttttgttttg tgttattttt     5580
ggtttgttta ttgggggggct tttttaatt gtcaggatta tgatcttgct gtttttcttc     5640
aatatgtata caaggtgatg tgaaaagatg acttgggcag aggagtaaga acaagtaggc     5700
ttgttcttct actttgcttc agaattcagt taatgccaaa agcgaagatc aagcccatgt     5760
tgatgtctcg ttgctcacct gcatttccag agagtgtgac actcatgcag tccctgagaa     5820
aaataaaatc agggacatac ttctcctttt agccttttaa aaattcaaaa acgtttagtc     5880
caagggaact ttttatgcta tcaggaaagg tttttgctgt ttttgattct gattatcaca     5940
gccaagtact ttgttttatt tctccctaat taataactac attccatgag gcctcttcca     6000
accaaagagg ccttttcttc caggagagtc ccgcaggaga tgctggtatg atgggcacca     6060
ttggttaagt aaactacatg caggaagaag tccttggggc cagtctgcca gctgagtcct     6120
ggttttggat gaagagttaa tgagatattg ggccaggctc aatgctgtag ttttaatgct     6180
aagaggttac gtttacttca cagagtacac ctcttagtaa cctctgactt aggcagctgc     6240
ttaaagcaaa ttgcaaaact ggcttgattt ggaatgtttt tattagagga aaaaagaaag     6300
ccatattatc tggaaaaaaa ttcattttaa ataccatcat tcaacaaatt atgttcagaa     6360
agtggtcaga acttaagcaa gaaaagtaaa gaaagaatgc agaattgtgg agcaatgctt     6420
taggaaatat ttctacctga acacttgtac tcttgaagtc acaacaaaat aatgatgagc     6480
ttttcacatc acctttatgg tttcaatccc tagctcaaag cttcctggaa tcttttattt     6540
tttgtaaact tttttttctt ttgttaaaat aaataaaaca ttcaatgttt ttctcctttt     6600
ctctcttatt acttctttcc tttggcattt tcaatttgaa atgctttcct ttggttgttg     6660
gttttattct ccccctaccc ctccccttttt cttattattc agaatataaa cctgcaaagc     6720
tctgctctgt tttggttttg aaagtttaag cttttctgct tctgtgagag cacaggcttc     6780
tgtccctttt gattccaact gaacttttgt gttctctaat gatactaaca cggtgtaggt     6840
tttacagtct cctaatttgt actggtaatg catattccaa ataaatagtt tcttttgttg     6900
caaaaaaaaa aaaaaaaa                                                   6918
```

<210> 22

<211> 1138
<212> DNA
<213> Homo sapiens

<400> 22

```
ggaccgttag ggagcccaat gggcgtcgcc gccaggcccc gttgcagagc gcgtctagcc      60
aataggcagc ggcggcgggc gggcgcgggc gacaggcggc gcagctgagg cggagcaggc     120
gctgcggcag gagggaagat ggcggacgag gagaagctgc cgcccggctg ggagaagcgc     180
atgagccgca gctcaggccg agtgtactac ttcaaccaca tcactaacgc cagccagtgg     240
gagcggccca gcggcaacag cagcagtggt ggcaaaaacg ggcagggggga gcctgccagg     300
gtccgctgct cgcacctgct ggtgaagcac agccagtcac ggcggccctc gtcctggcgg     360
caggagaaga tcacccggac caaggaggag gccctggagc tgatcaacgg ctacatccag     420
aagatcaagt cgggagagga ggactttgag tctctggcct cacagttcag cgactgcagc     480
tcagccaagg ccaggggaga cctgggtgcc ttcagcagag gtcagatgca gaagccattt     540
gaagacgcct cgtttgcgct gcggacgggg gagatgagcg ggcccgtgtt cacggattcc     600
ggcatccaca tcatcctccg cactgagtga gggtggggag cccaggcctg gcctcggggc     660
agggcagggc ggctaggccg gccagctccc ccttgcccgc cagccagtgg ccgaaccccc     720
cactccctgc caccgtcaca cagtatttat tgttcccaca atggctggga gggggccctt     780
ccagattggg ggccctgggg tccccactcc ctgtccatcc ccagttgggg ctgcgaccgc     840
cagattctcc cttaaggaat tgacttcagc aggggtggga ggctcccaga cccagggcag     900
tgtggtggga ggggtgttcc aaagagaagg cctggtcagc agagccgccc cgtgtccccc     960
caggtgctgg aggcagactc gagggccgaa ttgtttctag ttaggccacg ctcctctgtt    1020
cagtcgcaaa ggtgaacact catgcggccc agccatgggc cctctgagca actgtgcagc    1080
accctttcac ccccaattaa acccagaacc actgctctgc aaaaaaaaaa aaaaaaaa      1138
```

<210> 23
<211> 6977
<212> DNA
<213> Homo sapiens

<400> 23

cccgggcccg ccccccgcct cccgccgcct ccgggctccc ggctcccggc cgcgcctcgc 60
cccatgcact cgccgcgccg cgcagcccgc gcacgcccgg atggctcctc gcgccgcggg 120
cggcgcaccc cttagcgccc gggccgccgc cgccagcccc ccgccgttcc agacgccgcc 180
gcggtgcccg gtgccgctgc tgttgctgct gctcctgggg gcggcgcggg ccggcgccct 240
ggagatccag cgtcggttcc cctcgcccac gcccaccaac aacttcgccc tggacggcgc 300
ggcggggacc gtgtacctgg cggccgtcaa ccgcctctat cagctgtcgg gcgccaacct 360
gagcctggag gccgaggcgg ccgtgggccc ggtgcccgac agcccgctgt gtcacgctcc 420

gcagctgccg caggcctcgt gcgagcaccc gcggcgcctc acggacaact acaacaagat 480

cctgcagctg gacccccggcc agggcctggt agtcgtgtgc gggtccatct accagggctt 540

ctgccagctg cggcgccggg gcaacatctc ggccgtggcc gtgcgcttcc cgcccgccgc 600

gccgcccgcc gagcccgtca cggtgttccc cagcatgctg aacgtggcgg ccaaccaccc 660

gaacgcgtcc accgtggggc tagttctgcc tcccgccgcg ggcgcggggg gcagccgcct 720

gctcgtgggc gccacgtaca ccggttacgg cagctccttc ttcccgcgca accgcagcct 780

ggaggaccac cgcttcgaga acacgcccga gatcgccatc cgctccctgg acacgcgcgg 840

cgacctggcc aagctcttca ccttcgacct caacccctcc gacgacaaca tcctcaagat 900

caagcagggc gccaaggagc agcacaagct gggcttcgtg agcgccttcc tgcacccgtc 960

cgacccgccg ccgggtgcac agtcctacgc gtacctggcg ctcaacagcg aggcgcgcgc 1020

gggcgacaag gagagccagg cgcggagcct gctggcgcgc atctgcctgc cccacggcgc 1080

cggcggcgac gccaagaagc tcaccgagtc ctacatccag ttgggcttgc agtgcgcggg 1140

cggcgcgggc cgcggcgacc tctacagccg cctggtgtcg gtcttcccag cccgggagcg 1200

gctctttgct gtcttcgagc ggccccaggg gtcccccgcg gcccgcgctg ctccggccgc 1260

actctgcgcc ttccgcttcg ccgacgtgcg agccgccatc cgagctgcgc gcaccgcctg 1320

cttcgtggaa ccggcgcccg acgtggtggc ggtgctcgac agcgtggtgc agggcacggg 1380

accggcctgc gagcgcaagc tcaacatcca gctccagcca gagcagctgg actgtggagc 1440

tgctcacctg cagcacccgc tgtccatcct gcagcccctg aaggccacgc ccgtgttccg 1500

cgccccgggc ctcacctccg tggccgtggc cagcgtcaac aactacacag cggtcttcct 1560

gggcacggtc aacgggaggc ttctcaagat caacctgaac gagagcatgc aggtggtgag 1620

caggcgggtg gtgactgtgg cctatgggga gcccgtgcac catgtcatgc agtttgaccc 1680

agcagactcc ggttaccttt acctgatgac gtcccaccag atggccaggg tgaaggtcgc 1740

cgcctgcaac gtgcactcca cctgtgggga ctgcgtgggt gcggcggacg cctactgcgg 1800

ctggtgtgcc ctggagacgc ggtgcacctt gcagcaggac tgcaccaatt ccagccagca 1860

gcatttctgg accagtgcca gcgagggccc cagccgctgt cctgccatga ccgtcctgcc 1920

ttccgagatc gatgtgcgcc aggagtaccc aggcatgatc ctgcagatct cgggcagcct 1980

gcccagcctc agtggcatgg agatggcctg tgactatggg aacaacatcc gcactgtggc 2040

tcgggtccca ggccctgcct ttggtcacca gattgcctac tgcaacctcc tgccgaggga 2100

ccagtttccg cccttccccc ccaaccagga ccacgtgact gttgagatgt ctgtgagggt 2160

caatgggcgg aacatcgtca aggccaattt caccatctac gactgcagcc gcactgcaca 2220

agtgtacccc cacacagcct gtaccagctg cctgtcggca cagtggccct gtttctggtg 2280

cagccagcag cactcctgtg tttccaacca gtctcggtgc gaggcctcac caaaccccac 2340
gagccctcag gactgccccc ggaccctgct ctcacccctg gcacccgtgc ctacgggtgg 2400
ctcccagaac atcctggtgc ctctggccaa cactgccttt ttccagggtg cagccctgga 2460
gtgtagtttt gggctggagg agatcttcga ggctgtgtgg gtgaatgagt ctgttgtacg 2520
ctgtgaccag gtggtgctgc acacgacccg gaagagccag gtgttcccgc tcagcctcca 2580
actaaagggg cggccagccc gattcctgga cagccctgag cccatgacag tcatggtcta 2640
taactgtgcc atgggcagcc ccgactgttc ccagtgcctg ggccgcgaag acctgggtca 2700
cctgtgcatg tggagtgatg gctgccgcct gcgggggcct ctgcagccca tggctggcac 2760
ctgccccgcc cccgagatcc gcgcgattga gcccctgagt ggcccgttgg acggtgggac 2820
cctgctgacc atccgaggaa ggaacctggg ccggcggctc agtgacgtgg cccacggcgt 2880
gtggattggt ggtgtggcct gtgagccact gcctgacaga tacacggtgt cggaggagat 2940
cgtgtgtgtc acagggccag ccccaggacc actctcaggt gtggtgaccg tgaacgcctc 3000
taaggagggc aagtcccggg accgcttctc ctacgtgctg cccctggtcc actccctgga 3060
gcctaccatg ggccccaagg ccgggggcac caggatcacc atccatggga atgacctcca 3120
tgtaggctcc gagctccagg tcctggtgaa cgacacagac ccctgcacgg agctgatgcg 3180
cacagatacc agcatcgcct gcaccatgcc tgaggggccc ctgccggctc cggtgcctgt 3240
gtgtgtgcgc ttcgagcgtc ggggctgcgt gcacggcaac ctcaccttct ggtacatgca 3300
gaacccggtc atcacggcca tcagtccccg ccgcagccct gtcagtggcg gcaggaccat 3360
cacagtggct ggtgagcgtt tccacatggt gcagaatgtg tccatggccg tccaccacat 3420
tggccgggag cccacgctct gcaaggttct caactccacc ctcatcacct gcccgtcccc 3480
cggggccctg agcaacgcat cagcgccagt ggacttcttc atcaatgggc gggcctacgc 3540
agacgaggtg gctgtggctg aggagctact ggaccccgag gaggcacagc ggggcagcag 3600
gttccgcctg gactacctcc ccaacccaca gttctctacg gccaagaggg agaagtggat 3660
caagcaccac cccggggagc ctctcaccct cgttatccac aaggagcagg acagcctggg 3720
gctccagagt cacgagtacc gggtcaagat aggccaagta agctgcgaca tccagattgt 3780
ctctgacaga atcatccact gctcggtcaa cgagtccctg ggcgcggccg tggggcagct 3840
gcccatcaca atccaggtag ggaacttcaa ccagaccatc gccacactgc agctgggggg 3900
cagcgagacg gccatcatcg tgtccatcgt catctgcagc gtcctgctgc tgctctccgt 3960
ggtggccctg ttcgtcttct gtaccaagag ccgacgtgct gagcgttact ggcagaagac 4020
gctgctgcag atggaggaga tggaatctca gatccgagag gaaatccgca aaggcttcgc 4080
tgagctgcag acagacatga cagatctcac caaggagctg aaccgcagcc agggcatccc 4140
cttcctggag tataagcact tcgtgacccg caccttcttc cccaagtgtt cctcccttta 4200

tgaagagcgt tacgtgctgc cctcccagac cctcaactcc cagggcagct cccaggcaca 4260

ggaaacccac ccactgctgg gagagtggaa gattcctgag agctgccggc ccaacatgga 4320

agagggaatt agcttgttct cctcactact caacaacaag cacttcctca tcgtctttgt 4380

ccacgcgctg gagcagcaga aggactttgc ggtgcgcgac aggtgcagcc tggcctcgct 4440

gctgaccatc gcgctgcacg gcaagctgga gtactacacc agcatcatga aggagctgct 4500

ggtggacctc attgacgcct cggccgccaa gaaccccaag ctcatgctgc ggcgcacaga 4560

gtctgtggtg gagaagatgc tcaccaactg gatgtccatc tgcatgtaca gctgtctgcg 4620

ggagacggtg gggagccat tcttcctgct gctgtgtgcc atcaagcagc aaatcaacaa 4680

gggctccatc gacgccatca caggcaaggc ccgctacaca ctcaatgagg agtggctgct 4740

gcgggagaac atcgaggcca agccccggaa cctgaacgtg tccttccagg gctgtggcat 4800

ggactcgctg agcgtgcggg ccatggacac cgacacgctg acacaggtca aggagaagat 4860

cctggaggcc ttctgcaaga atgtgcccta ctcccagtgg ccgcgtgcag aggacgtcga 4920

ccttgagtgg ttcgcctcca gcacacagag ctacatcctt cgggacctgg acgacacctc 4980

agtggtggaa gacggccgca agaagcttaa cacgctggcc cattacaaga tccctgaagg 5040

tgcctccctg gccatgagtc tcatagacaa gaaggacaac acactgggcc gagtgaaaga 5100

cttggacaca gagaagtatt tccatttggt gctgcctacg gacgagctgg cggagcccaa 5160

gaagtctcac cggcagagcc atcgcaagaa ggtgctcccg gaaatctacc tgacccgcct 5220

gctctccacc aagggcacgt tgcagaagtt tctggatgac ctgttcaagg ccattctgag 5280

tatccgtgaa gacaagcccc cactggctgt caagtacttt ttcgacttcc tggaggagca 5340

ggctgagaag aggggaatct ccgaccccga caccctacac atctggaaga ccaacagcct 5400

tcctctccgg ttctgggtga acatcctgaa gaacccccag tttgtctttg acatcgacaa 5460

gacagaccac atcgacgcct gcctttcagt catcgcgcag gccttcatcg acgcctgctc 5520

catctctgac ctgcagctgg gcaaggattc gccaaccaac aagctcctct acgccaagga 5580

gattcctgag taccggaaga tcgtgcagcg ctactacaag cagatccagg acatgacgcc 5640

gctcagcgag caagagatga atgcccatct ggccgaggag tcgaggaaat accagaatga 5700

gttcaacacc aatgtggcca tggcagagat ttataagtac gccaagaggt atcggccgca 5760

gatcatggcc gcgctggagg ccaaccccac ggcccggagg acacaactgc agcacaagtt 5820

tgagcaggtg gtggctttga tggaggacaa catctacgag tgctacagtg aggcctgaga 5880

cacatggaga gttggtcagg ctgctgctgg gagaaatgga cgcccactgg gcctcaactt 5940

gatcttctac cccgtgcctg tgactcagac tgggaaatac tgagcagaga cggctggggc 6000

gggggcagga ggaggggctg ctctctgaga caggggcgcc cccgccttga cccctgggca 6060

cctccatccc ctcccacctg tccccagatc agtctctggg atggaggcca gagagctggt 6120

caggctcccc catctgccca gcacggcctg cactgtgccc acccacttgc tccacaacgt 6180

ccagttggtc ctgctgccaa gagccccgtg catccaggcg gccaagcaca aactggggga 6240

gaggaggccg ccagcccgga ggctgcagcc cagaaactct acctcatcca cactggtgca 6300

gggagccctc cttgaactga cctttgattg gtttctgctt caactaccaa aatgttatct 6360

ccacttcccc ctcacccgta gaggatcctg gccacagaca gtttcaagta gtgtcagatt 6420

tttgttgctt gggcggctgt tggtagagtg ggcagtgccc gcgccatggg gtgctctgtg 6480

ggcttctcca ggagcaggga gggtggaggg gagggatggg gggcacagga gctgggagcc 6540

ccgtctccag gaaaaggaga ggggttaaga tgcaccgagg ctgtagctgg gctacttgat 6600

cttgctgaaa gtgtttctaa agatagcacc actttttttt ttaaagcttt tatatattaa 6660

aaaacgtatc atgcaccaac tgtgaatagc tgccgcttgc gcagaggacc cggggagggg 6720

tcccgagagg ctccccatgc aacactggaa atgactgttc cagagagcgg gcagacctgg 6780

cagagcgccc ctggcgcctg agactaccac ccactccgtt cctgccagaa acgaccctct 6840

gtggccgatg ggccatgcgg gcccctcgca gccaactcag ccagtgttgg gactggctca 6900

gagcccatgg gggctggagg ggggcagctg ggactctgga atcttcttta taataaaagc 6960

cttacggaca aacctac 6977

<210> 24
<211> 1097
<212> DNA
<213> Homo sapiens

<400> 24

tagaaggcag tcttgtgggt gcctcctccc ccagccgcaa ctcaggtctg cagctgggtc 60
ctgcctcctt ccgagtgggc catggccggt acatggctgc tacttctcct ggcccttggg 120
tgtccagccc tacccacagg tgtgggcggc acaccctttc cttctctggc cccaccaatc 180
atgctgctgg tggatggaaa gcagcagatg gtggtggtct gcctggtcct tgatgttgca 240
ccccctggcc ttgacagccc catctggttc tcagccggca atggcagtgc actggatgcc 300
ttcacctatg gcccttcccc agcaacggat ggcacctgga ccaacttggc ccatctctcc 360
ctgccttctg aggagctggc atcctgggag cctttggtct gccacactgg gcctggggct 420
gagggtcaca gcaggagtac acagcccatg catctgtcag gagaggcttc tacagccagg 480
acctgccccc aggagcctct caggggggaca ccgggtgggg cgctgtggct gggggtcctg 540
cggctgctgc tcttcaagct gctgctgttt gacctgctcc tgacctgcag ctgcctgtgc 600
gaccccgcgg gcccgctgcc ttcccccgca accaccaccc gcctgcgagc cctcggctcc 660
catcgactgc acccggccac ggagactggg ggacgagagg ccaccagctc acccagaccc 720

cagcctcggg accgccgctg ggtgacaccc cctccgggtc ggaagcccgg gagcccagta 780
tggggggaag ggtcttacct cagcagttac cccacttgcc cagcacaggc ctggtgctca 840
agatctgccc tcagggctcc ttcctccagt cttggagcat tttttgcagg tgacctgcct 900
cctcctctgc aggctggagc tgcctgaggg cagggctcta cctcccctgc gtcacactgt 960
gtgaggctgt gtctctgcca tccaaaaggg ggccccttga gaatggtgat ccacccagtt 1020
acaggggcat ttagggagca gatgactgag aacattaaaa aagaacttaa atgacacagc 1080
aaaaaaaaaa aaaaaaa 1097

<210> 25
<211> 3963
<212> DNA
<213> Homo sapiens

<400> 25

```
ggagagccga aagcggagct cgaaactgac tggaaacttc agtggcgcgg agactcgcca      60
gtttcaaccc cggaaacttt tctttgcagg aggagaagag aaggggtgca agcgccccca     120
cttttgctct ttttcctccc ctcctcctcc tctccaattc gcctccccc acttggagcg      180
ggcagctgtg aactggccac cccgcgcctt cctaagtgct cgccgcggta gccggccgac     240
gcgccagctt ccccgggagc cgcttgctcc gcatccgggc agccgagggg agaggagccc     300
gcgcctcgag tccccgagcc gccgcggctt ctcgcctttc ccggccacca gccccctgcc     360
ccgggcccgc gtatgaatct cctggacccc ttcatgaaga tgaccgacga gcaggagaag     420
ggcctgtccg gcgcccccag ccccaccatg tccgaggact ccgcgggctc gccctgcccg     480
tcgggctccg gctcggacac cgagaacacg cggccccagg agaacacgtt ccccaagggc     540
gagcccgatc tgaagaagga gagcgaggag gacaagttcc ccgtgtgcat ccgcgaggcg     600
gtcagccagg tgctcaaagg ctacgactgg acgctggtgc ccatgccggt gcgcgtcaac     660
ggctccagca agaacaagcc gcacgtcaag cggcccatga acgccttcat ggtgtgggcg     720
caggcggcgc gcaggaagct cgcggaccag tacccgcact tgcacaacgc cgagctcagc     780
aagacgctgg gcaagctctg gagacttctg aacgagagcg agaagcggcc cttcgtggag     840
gaggcggagc ggctgcgcgt gcagcacaag aaggaccacc cggattacaa gtaccagccg     900
cggcggagga agtcggtgaa gaacgggcag gcggaggcag aggaggccac ggagcagacg     960
cacatctccc ccaacgccat cttcaaggcg ctgcaggccg actcgccaca ctcctcctcc    1020
ggcatgagcg aggtgcactc ccccggcgag cactcggggc aatcccaggg cccaccgacc    1080
ccacccacca cccccaaaac cgacgtgcag ccgggcaagg ctgacctgaa gcgagagggg    1140
cgccccttgc cagagggggg cagacagccc cctatcgact tccgcgacgt ggacatcggc    1200
gagctgagca gcgacgtcat ctccaacatc gagaccttcg atgtcaacga gtttgaccag    1260
```

tacctgccgc ccaacggcca cccgggggtg ccggccacgc acggccaggt cacctacacg 1320
ggcagctacg gcatcagcag caccgcggcc accccggcga gcgcgggcca cgtgtggatg 1380
tccaagcagc aggcgccgcc gccacccccg cagcagcccc cacaggcccc gccggccccg 1440
caggcgcccc cgcagccgca ggcggcgccc ccacagcagc cggcggcacc cccgcagcag 1500
ccacaggcgc acacgctgac cacgctgagc agcgagccgg gccagtccca gcgaacgcac 1560
atcaagacgg agcagctgag ccccagccac tacagcgagc agcagcagca ctcgccccaa 1620
cagatcgcct acagcccctt caacctccca cactacagcc cctcctaccc gcccatcacc 1680
cgctcacagt acgactacac cgaccaccag aactccagct cctactacag ccacgcggca 1740
ggccagggca ccggcctcta ctccaccttc acctacatga accccgctca gcgccccatg 1800
tacaccccca tcgccgacac ctctggggtc ccttccatcc cgcagaccca cagcccccag 1860
cactgggaac aacccgtcta cacacagctc actcgacctt gaggaggcct cccacgaagg 1920
gcgaagatgg ccgagatgat cctaaaaata accgaagaaa gagaggacca accagaattc 1980
cctttggaca tttgtgtttt tttgtttttt tattttgttt tgtttttttct tcttcttctt 2040
cttccttaaa gacatttaag ctaaaggcaa ctcgtaccca aatttccaag acacaaacat 2100
gacctatcca agcgcattac ccacttgtgg ccaatcagtg gccaggccaa ccttggctaa 2160
atggagcagc gaaatcaacg agaaactgga ctttttaaac cctcttcaga gcaagcgtgg 2220
aggatgatgg agaatcgtgt gatcagtgtg ctaaatctct ctgcctgttt ggactttgta 2280
attatttttt tagcagtaat taaagaaaaa agtcctctgt gaggaatatt ctctatttta 2340
aatattttta gtatgtactg tgtatgattc attaccattt tgaggggatt tatacatatt 2400
tttagataaa attaaatgct cttatttttc caacagctaa actactctta gttgaacagt 2460
gtgccctagc ttttcttgca accagagtat ttttgtacag atttgctttc tcttacaaaa 2520
agaaaaaaaa aatcctgttg tattaacatt taaaaacaga attgtgttat gtgatcagtt 2580
ttgggggtta actttgctta attcctcagg ctttgcgatt taaggaggag ctgccttaaa 2640
aaaaaataaa ggccttattt tgcaattatg ggagtaaaca atagtctaga gaagcatttg 2700
gtaagcttta tcatatatat attttttaaa gaagagaaaa acaccttgag ccttaaaacg 2760
gtgctgctgg gaaacatttg cactctttta gtgcatttcc tcctgccttt gcttgttcac 2820
tgcagtctta agaaagaggt aaaaggcaag caaaggagat gaaatctgtt ctgggaatgt 2880
ttcagcagcc aataagtgcc cgagcacact gcccccggtt gcctgcctgg gccccatgtg 2940
gaaggcagat gcctgctcgc tctgtcacct gtgcctctca gaacaccagc agttaacctt 3000
caagacattc cacttgctaa aattatttat tttgtaagga gaggttttaa ttaaaacaaa 3060
aaaaaattct tttttttttt tttttccaat tttaccttct ttaaaatagg ttgttggagc 3120
tttcctcaaa gggtatggtc atctgttgtt aaattatgtt cttaactgta accagttttt 3180

ttttatttat ctctttaatc tttttttatt attaaaagca agtttctttg tattcctcac 3240

cctagatttg tataaatgcc tttttgtcca tcccttttttt ctttgttgtt tttgttgaaa 3300

acaaactgga aacttgtttc tttttttgta taaatgagag attgcaaatg tagtgtatca 3360

ctgagtcatt tgcagtgttt tctgccacag acctttgggc tgccttatat tgtgtgtgtg 3420

tgtgggtgtg tgtgtgtttt gacacaaaaa caatgcaagc atgtgtcatc catatttctc 3480

tgcatcttct cttggagtga gggaggctac ctggagggga tcagcccact gacagacctt 3540

aatcttaatt actgctgtgg ctagagagtt tgaggattgc tttttaaaaa agacagcaaa 3600

cttttttttt tatttaaaaa aagatatatt aacagtttta gaagtcagta gaataaaatc 3660

ttaaagcact cataatatgg catccttcaa tttctgtata aaagcagatc tttttaaaaa 3720

gatacttctg taacttaaga aacctggcat ttaaatcata ttttgtcttt aggtaaaagc 3780

tttggtttgt gttcgtgttt tgtttgtttc acttgtttcc ctcccagccc caaacctttt 3840

gttctctccg tgaaacttac ctttcccttt ttctttctct tttttttttt tgtatattat 3900

tgtttacaat aaatatacat tgcattaaaa agaaaaaaaa aaaaaaaaaa aaaaaaaaaa 3960

aaa 3963

<210> 26
<211> 8605
<212> DNA
<213> Homo sapiens

<400> 26

```
aattcgccaa ctgaaaaagt gggaaaggat gtctggaggc gaggcgtccc attacagagg      60
aaggagctcg ctatataagc cagccaaagt tggctgcacc ggccacagcc tgcctactgt     120
cacccgcctc tcccgcgcgc agatacacgc ccccgcctcc gtgggcacaa aggcagcgct     180
gctggggaac tcgggggaac gcgcacgtgg gaaccgccgc agctccacac tccaggtact     240
tcttccaagg acctaggtct ctcgcccatc ggaaagaaaa taattctttc aagaagatca     300
gggacaactg atttgaagtc tactctgtgc ttctaaatcc ccaattctgc tgaaagtgag     360
ataccctaga gccctagagc cccagcagca cccagccaaa cccacctcca ccatgggggc     420
catgactcag ctgttggcag gtgtctttct tgctttcctt gccctcgcta ccgaaggtgg     480
ggtcctcaag aaagtcatcc ggcacaagcg acagagtggg gtgaacgcca ccctgccaga     540
agagaaccag ccagtggtgt ttaaccacgt ttacaacatc aagctgccag tgggatccca     600
gtgttcggtg gatctggagt cagccagtgg ggagaaagac ctggcaccgc cttcagagcc     660
cagcgaaagc tttcaggagc acacagtgga tggggaaaac cagattgtct tcacacatcg     720
catcaacatc ccccgccggg cctgtggctg tgccgcagcc cctgatgtta aggagctgct     780
gagcagactg gaggagctgg agaacctggt gtcttccctg agggagcaat gtactgcagg     840
```

agcaggctgc tgtctccagc ctgccacagg ccgcttggac accaggccct tctgtagcgg 900
tcggggcaac ttcagcactg aaggatgtgg ctgtgtctgc gaacctggct ggaaaggccc 960
caactgctct gagcccgaat gtccaggcaa ctgtcacctt cgaggccggt gcattgatgg 1020
gcagtgcatc tgtgacgacg gcttcacggg cgaggactgc agccagctgg cttgccccag 1080
cgactgcaat gaccagggca agtgcgtaaa tggagtctgc atctgtttcg aaggctacgc 1140
cggggctgac tgcagccgtg aaatctgccc agtgccctgc agtgaggagc acggcacatg 1200
tgtagatggc ttgtgtgtgt gccacgatgg ctttgcaggc gatgactgca acaagcctct 1260
gtgtctcaac aattgctaca accgtggacg atgcgtggag aatgagtgcg tgtgtgatga 1320
gggtttcacg ggcgaagact gcagtgagct catctgcccc aatgactgct tcgaccgggg 1380
ccgctgcatc aatggcacct gctactgcga agaaggcttc acaggtgaag actgcgggaa 1440
acccacctgc ccacatgcct gccacaccca gggccggtgt gaggaggggc agtgtgtatg 1500
tgatgagggc tttgccggtg tggactgcag cgagaagagg tgtcctgctg actgtcacaa 1560
tcgtggccgc tgtgtagacg ggcggtgtga gtgtgatgat ggtttcactg gagctgactg 1620
tggggagctc aagtgtccca atggctgcag tggccatggc cgctgtgtca atgggcagtg 1680
tgtgtgtgat gagggctata ctggggagga ctgcagccag ctacggtgcc ccaatgactg 1740
tcacagtcgg ggccgctgtg tcgagggcaa atgtgtatgt gagcaaggct tcaagggcta 1800
tgactgcagt gacatgagct gccctaatga ctgtcaccag cacggccgct gtgtgaatgg 1860
catgtgtgtt tgtgatgacg gctacacagg ggaagactgc cgggatcgcc aatgccccag 1920
ggactgcagc aacaggggcc tctgtgtgga cggacagtgc gtctgtgagg acggcttcac 1980
cggccctgac tgtgcagaac tctcctgtcc aaatgactgc catggccagg gtcgctgtgt 2040
gaatgggcag tgcgtgtgcc atgaaggatt tatgggcaaa gactgcaagg agcaaagatg 2100
tcccagtgac tgtcatggcc agggccgctg cgtggacggc cagtgcatct gccacgaggg 2160
cttcacaggc ctggactgtg gccagcactc ctgccccagt gactgcaaca acttaggaca 2220
atgcgtctcg ggccgctgca tctgcaacga gggctacagc ggagaagact gctcagaggt 2280
gtctcctccc aaagacctcg ttgtgacaga agtgacggaa gagacggtca acctggcctg 2340
ggacaatgag atgcgggtca cagagtacct tgtcgtgtac acgcccaccc acgagggtgg 2400
tctggaaatg cagttccgtg tgcctgggga ccagacgtcc accatcatcc aggagctgga 2460
gcctggtgtg gagtacttta tccgtgtatt tgccatcctg gagaacaaga agagcattcc 2520
tgtcagcgcc agggtggcca cgtacttacc tgcacctgaa ggcctgaaat tcaagtccat 2580
caaggagaca tctgtggaag tggagtggga tcctctagac attgcttttg aaacctggga 2640
gatcatcttc cggaatatga ataaagaaga tgagggagag atcaccaaaa gcctgaggag 2700

gccagagacc tcttaccggc aaactggtct agctcctggg caagagtatg agatatctct 2760
gcacatagtg aaaaacaata cccggggccc tggcctgaag agggtgacca ccacacgctt 2820
ggatgccccc agccagatcg aggtgaaaga tgtcacagac accactgcct tgatcacctg 2880
gttcaagccc ctggctgaga tcgatggcat tgagctgacc tacggcatca aagacgtgcc 2940
aggagaccgt accaccatcg atctcacaga ggacgagaac cagtactcca tcgggaacct 3000
gaagcctgac actgagtacg aggtgtccct catctcccgc agaggtgaca tgtcaagcaa 3060
cccagccaaa gagaccttca caacaggcct cgatgctccc aggaatcttc gacgtgtttc 3120
ccagacagat aacagcatca ccctggaatg gaggaatggc aaggcagcta ttgacagtta 3180
cagaattaag tatgccccca tctctggagg ggaccacgct gaggttgatg ttccaaagag 3240
ccaacaagcc acaaccaaaa ccacactcac aggtctgagg ccgggaactg aatatgggat 3300
tggagtttct gctgtgaagg aagacaagga gagcaatcca gcgaccatca acgcagccac 3360
agagttggac acgcccaagg accttcaggt ttctgaaact gcagagacca gcctgaccct 3420
gctctggaag acaccgttgg ccaaatttga ccgctaccgc ctcaattaca gtctccccac 3480
aggccagtgg gtgggagtgc agcttccaag aaacaccact tcctatgtcc tgagaggcct 3540
ggaaccagga caggagtaca atgtcctcct gacagccgag aaaggcagac acaagagcaa 3600
gcccgcacgt gtgaaggcat ccactgaaca agcccctgag ctggaaaacc tcaccgtgac 3660
tgaggttggc tgggatggcc tcagactcaa ctggaccgca gctgaccagg cctatgagca 3720
ctttatcatt caggtgcagg aggccaacaa ggtggaggca gctcggaacc tcaccgtgcc 3780
tggcagcctt cgggctgtgg acataccggg cctcaaggct gctacgcctt atacagtctc 3840
catctatggg gtgatccagg gctatagaac accagtgctc tctgctgagg cctccacagg 3900
ggaaactccc aatttgggag aggtcgtggt ggccgaggtg ggctgggatg ccctcaaact 3960
caactggact gctccagaag ggcctatga gtactttttc attcaggtgc aggaggctga 4020
cacagtagag gcagcccaga acctcaccgt cccaggagga ctgaggtcca cagacctgcc 4080
tgggctcaaa gcagccactc attataccat caccatccgc ggggtcactc aggacttcag 4140
cacaacccct ctctctgttg aagtcttgac agaggaggtt ccagatatgg gaaacctcac 4200
agtgaccgag gttagctggg atgctctcag actgaactgg accacgccag atggaaccta 4260
tgaccagttt actattcagg tccaggaggc tgaccaggtg gaagaggctc acaatctcac 4320
ggttcctggc agcctgcgtt ccatggaaat cccaggcctc agggctggca ctccttacac 4380
agtcaccctg cacggcgagg tcaggggcca cagcactcga ccccttgctg tagaggtcgt 4440
cacagaggat ctcccacagc tgggagattt agccgtgtct gaggttggct gggatggcct 4500
cagactcaac tggaccgcag ctgacaatgc ctatgagcac tttgtcattc aggtgcagga 4560
ggtcaacaaa gtggaggcag cccagaacct cacgttgcct ggcagcctca gggctgtgga 4620

catcccgggc ctcgaggctg ccacgcctta tagagtctcc atctatgggg tgatccgggg 4680

ctatagaaca ccagtactct ctgctgaggc ctccacagcc aaagaacctg aaattggaaa 4740

cttaaatgtt tctgacataa ctcccgagag cttcaatctc tcctggatgg ctaccgatgg 4800

gatcttcgag acctttacca ttgaaattat tgattccaat aggttgctgg agactgtgga 4860

atataatatc tctggtgctg aacgaactgc ccatatctca gggctacccc ctagtactga 4920

ttttattgtc tacctctctg gacttgctcc cagcatccgg accaaaacca tcagtgccac 4980

agccacgaca gaggccctgc cccttctgga aaacctaacc atttccgaca ttaatcccta 5040

cgggttcaca gtttcctgga tggcatcgga gaatgccttt gacagctttc tagtaacggt 5100

ggtggattct gggaagctgc tggaccccca ggaattcaca ctttcaggaa cccagaggaa 5160

gctggagctt agaggcctca taactggcat tggctatgag gttatggtct ctggcttcac 5220

ccaagggcat caaaccaagc ccttgagggc tgagattgtt acagaagccg aaccggaagt 5280

tgacaacctt ctggtttcag atgccacccc agacggtttc cgtctgtcct ggacagctga 5340

tgaaggggtc ttcgacaatt ttgttctcaa aatcagagat accaaaaagc agtctgagcc 5400

actggaaata accctacttg cccccgaacg taccagggac ataacaggtc tcagagaggc 5460

tactgaatac gaaattgaac tctatggaat aagcaaagga aggcgatccc agacagtcag 5520

tgctatagca acaacagcca tgggctcccc aaaggaagtc attttctcag acatcactga 5580

aaattcggct actgtcagct ggagggcacc cacagcccaa gtggagagct tccggattac 5640

ctatgtgccc attacaggag gtacaccctc catggtaact gtggacggaa ccaagactca 5700

gaccaggctg gtgaaactca tacctggcgt ggagtacctt gtcagcatca tcgccatgaa 5760

gggctttgag gaaagtgaac ctgtctcagg gtcattcacc acagctctgg atggcccatc 5820

tggcctggtg acagccaaca tcactgactc agaagccttg gccaggtggc agccagccat 5880

tgccactgtg gacagttatg tcatctccta cacaggcgag aaagtgccag aaattacacg 5940

cacggtgtcc gggaacacag tggagtatgc tctgaccgac ctcgagcctg ccacggaata 6000

cacactgaga atctttgcag agaaagggcc ccagaagagc tcaaccatca ctgccaagtt 6060

cacaacagac ctcgattctc caagagactt gactgctact gaggttcagt cggaaactgc 6120

cctccttacc tggcgacccc cccgggcatc agtcaccggt tacctgctgg tctatgaatc 6180

agtggatggc acagtcaagg aagtcattgt gggtccagat accacctcct acagcctggc 6240

agacctgagc ccatccaccc actacacagc caagatccag gcactcaatg ggcccctgag 6300

gagcaatatg atccagacca tcttcaccac aattggactc ctgtacccct tccccaagga 6360

ctgctcccaa gcaatgctga atggagacac gacctctggc ctctacacca tttatctgaa 6420

tggtgataag gctgaggcgc tggaagtctt ctgtgacatg acctctgatg gggtggatg 6480

gattgtgttc ctgagacgca aaaacggacg cgagaacttc taccaaaact ggaaggcata 6540
tgctgctgga tttggggacc gcagagaaga attctggctt gggctggaca acctgaacaa 6600
aatcacagcc caggggcagt acgagctccg ggtggacctg cgggaccatg gggagacagc 6660
ctttgctgtc tatgacaagt tcagcgtggg agatgccaag actcgctaca agctgaaggt 6720
ggaggggtac agtgggacag caggtgactc catggcctac cacaatggca gatccttctc 6780
cacctttgac aaggacacag attcagccat caccaactgt gctctgtcct acaaaggggc 6840
tttctggtac aggaactgtc accgtgtcaa cctgatgggg agatatgggg acaataacca 6900
cagtcagggc gttaactggt tccactggaa gggccacgaa cactcaatcc agtttgctga 6960
gatgaagctg agaccaagca acttcagaaa tcttgaaggc aggcgcaaac gggcataaat 7020
tccagggacc actgggtgag agaggaataa ggcccagagc gaggaaagga ttttaccaaa 7080
gcatcaatac aaccagccca accatcggtc cacacctggg catttggtga gagtcaaagc 7140
tgaccatgga tccctggggc caacggcaac agcatgggcc tcacctcctc tgtgatttct 7200
ttctttgcac caaagacatc agtctccaac atgtttctgt tttgttgttt gattcagcaa 7260
aaatctccca gtgacaacat cgcaatagtt ttttacttct cttaggtggc tctgggaatg 7320
ggagaggggt aggatgtaca ggggtagttt gttttagaac cagccgtatt ttacatgaag 7380
ctgtataatt aattgtcatt atttttgtta gcaaagatta aatgtgtcat tggaagccat 7440
ccctttttt acatttcata caacagaaac cagaaaagca atactgtttc cattttaagg 7500
atatgattaa tattattaat ataataatga tgatgatgat gatgaaaact aaggattttt 7560
caagagatct ttctttccaa aacatttctg gacagtacct gattgtattt ttttttaaa 7620
taaaagcaca agtacttttg agtttgttat tttgctttga attgttgagt ctgaatttca 7680
ccaaagccaa tcatttgaac aaagcgggga atgttgggat aggaaaggta agtagggata 7740
gtggtcaagt gggaggggtg gaaaggagac taaagactgg gagagaggga agcacttttt 7800
ttaaataaag ttgaacacac ttgggaaaag cttacaggcc aggcctgtaa tcccaacact 7860
ttgggaggcc aaggtgggag gatagcttaa ccccaggagt ttgagaccag cctgagcaac 7920
atagtgagaa cttgtctcta cagaaaaaaa aaaaaaaaaa aatttaatta ggcaagcgtg 7980
gtagtgcgca cctgtcgtcc cagctactca ggaggctgag gtaggaaaat cactggagcc 8040
caggagttag aggttacagt gagctatgat cacactactg cactccagcc tgggcaacag 8100
agggagaccc tgtctctaaa taaaaaaaga aaagaaaaaa aaagcttaca acttgagatt 8160
cagcatcttg ctcagtattt ccaagactaa tagattatgg tttaaaagat gcttttatac 8220
tcattttcta atgcaactcc tagaaactct atgatatagt tgaggtaagt attgttacca 8280
cacatgggct aagatcccca gaggcagact gcctgagttc aattcttggc tccaccattc 8340
ccaagttccc taacctctct atgcctcagt ttcctcttct gtaaagtagg gacactcata 8400

```
cttctcattt cagaacattt ttgtgaagaa taaattatgt tatccatttg aggcccttag     8460
aatggtaccc ggtgtatatt aagtgctagt acatgttagc tatcatcatt atcactttat     8520
atgagatgga ctggggttca tagaaaccca atgacttgat tgtggctact actcaataaa     8580
taatagaatt tggatttaaa aaaaa                                           8605
```

## Description

### FIELD OF THE INVENTION

The present invention generally relates to the field of bioinformatics, genomic processing, proteomic processing, and related arts. More particularly, the present invention relates to a computer-implemented method for inferring activity of a Notch cellular signaling pathway in a subject performed by a digital processing device, wherein the inferring is based on expression levels of five or more target genes of the Notch cellular signaling pathway measured in a sample of the subject. The present invention further relates to an apparatus for inferring activity of a Notch cellular signaling pathway in a subject comprising a digital processor configured to perform the method, to a non-transitory storage medium for inferring activity of a Notch cellular signaling pathway in a subject storing instructions that are executable by a digital processing device to perform the method, and to a computer program for inferring activity of a Notch cellular signaling pathway in a subject comprising program code means for causing a digital processing device to perform the method, when the computer program is run on the digital processing device. The present invention further relates to a kit for measuring expression levels of five or more target genes of the Notch cellular signaling pathway in a sample of a subject, to a kit for inferring activity of a Notch cellular signaling pathway in a subject, and to uses of the kits in performing the method.

The present invention is defined in the appended claims.

### BACKGROUND OF THE INVENTION

Genomic and proteomic analyses have substantial realized and potential promise for clinical application in medical fields such as oncology, where various cancers are known to be associated with specific combinations of genomic mutations/variations and/or high or low expression levels for specific genes, which play a role in growth and evolution of cancer, e.g., cell proliferation and metastasis.

Notch is an inducible transcription factor that regulates the expression of many genes involved in embryonic development, the immune response, and in cancer. Regarding pathological disorders, such as cancer (e.g., breast or ovarian cancer), abnormal Notch pathway activity plays an important role (see Aster J.C. et al., "The varied roles of Notch in cancer", Annual Review of Pathology, Vol. 12, No. 1, December 2016, pages 245 to 275). The Notch cellular signaling pathway consists of a protein receptor from the Notch family, and a family of (cell-bound) ligands (DSL family) which induce cleavage of the bound receptor, upon which the cleaved intracellular fragment moves to the nucleus, where it forms, together with other proteins, an active transcription factor complex which binds and transactivates a well-defined set of target genes (see also Fig. 1, which is based on Guruharsha K.G. et al., "The Notch signaling system: recent insights into the complexity of a conserved pathway", Nature Reviews Genetics, Vol. 13, September 2012, pages 654 to 666).

With respect to the Notch signaling in e.g. cancer, it is important to be able to detect abnormal Notch signaling activity in order to enable the right choice of targeted drug treatment. Currently anti-Notch therapies are being developed (see Espinoza I. and Miele L., "Notch inhibitors for cancer treatment", Pharmacology & Therapeutics, Vol. 139, No. 2, August 2013, pages 95 to 110). However, today there is no clinical assay available to assess the functional state resp. activity of the Notch cellular signaling pathway, which in its active state indicates that it is, for instance, more likely to be tumor-promoting compared to its passive state. It is therefore desirable to be able to improve the possibilities of characterizing patients that have a disease, such as a cancer, e.g., a breast, cervical, endometrial, ovarian, pancreatic or prostate cancer, or an immune disorder, which is at least partially driven by an abnormal activity of the Notch cellular signaling pathway, and that are therefore likely to respond to inhibitors of the Notch cellular signaling pathway.

Quantitative models for determining the pathway activity have been described for example in US 2016/117439 A1 (TGFbeta, PI3K, WNT, ER and HH), WO 2013/011479 (WNT, ER, AR, HH) and WO 2016/062891 (TGFbeta), wherein the models are based on specific target genes for the respective pathways.

S. S. RAO Et al., CANCER RESEARCH, vol. 69, no. 7, 24 March 2009 (2009-03-24), pages 3060-3068 relates to the role of Notch signaling in Acute lymphoblastic leukemia cells, and further discloses Notch target genes.

US 7 544 476 B1 discloses a method for identifying a cancer tissue sensitive to Notch inhibitors based on HeyL expression levels.

KR 2016 0086775 A relates to prevention or treatment of stroke using PIN1 inhibitors.

US 2016/266095 A1 relates to Notch inhibitors and potential uses thereof.

US 2016/125127 A1 discloses a model for identifying combinations of oncogenes in the Notch signaling pathway that may serve as potential drug target in glioblastoma.

WEIWEI LIU et al., THE SCIENTIFIC WORLD JOURNAL, vol. 2013, 1 January 2013 (2013-01-01), pages 1 -6, relates to the role of Notch signaling in the migration and proliferation of retinal pigment cells.

SUMMARY OF THE INVENTION

In accordance with a main aspect of the present invention, the above problem is solved by a computer-implemented method for inferring activity of a Notch cellular signaling pathway in a subject performed by a digital processing device, wherein the inferring comprises:

receiving expression levels of five or more, for example, six, seven, eight, nine, ten, eleven or more, target genes of the Notch cellular signaling pathway measured in a sample of the subject,

determining an activity level of a Notch transcription factor (TF) element in the sample of the subject, the Notch TF element controlling transcription of the five or more Notch target genes, the determining being based on evaluating a calibrated mathematical model pathway relating the expression levels of the five or more Notch target genes to the activity level of the Notch TF element, and

inferring the activity of the Notch cellular signaling pathway in the subject based on the determined activity level of the Notch TF element in the sample of the subject,

wherein the five or more Notch target genes are selected from the group consisting of: DTX1, EPHB3, HES1, HES4, HES5, HEY2, MYC, NFKB2, NRARP, PIN1, PLXND1, and SOX9, wherein two or more, for example, three, four, five, six or more, Notch target genes are selected from the group consisting of: DTX1, HES1, HES4, HES5, HEY2, MYC, and NRARP, and three or more, for example, four or more, Notch target genes are selected from the group consisting of: EPHB3, NFKB2, PIN1, PLXND1, and SOX9, wherein the method is used in at least one of the following activities: diagnosis based on the inferred activity of the Notch cellular signaling pathway in the subject; prognosis based on the inferred activity of the Notch cellular signaling pathway in the subject; and wherein the subject is suffering from, or suspected to be suffering from, a brain cancer.

Herein, the "activity level" of a TF element denotes the level of activity of the TF element regarding transcription of its target genes.

The present invention is based on the innovation of the inventors that a suitable way of identifying effects occurring in the Notch cellular signaling pathway can be based on a measurement of the signaling output of the Notch cellular signaling pathway, which is - amongst others - the transcription of the target genes, which is controlled by a Notch transcription factor (TF) element that is controlled by the Notch cellular signaling pathway. This innovation by the inventors assumes that the TF activity level is at a quasi-steady state in the sample, which can be detected by means of - amongst others - the expression values of the Notch target genes. The Notch cellular signaling pathway targeted herein is known to control many functions in many cell types in humans, such as proliferation, differentiation and wound healing. Regarding pathological disorders, such as cancer (e.g., breast, cervical, endometrial, ovarian, pancreatic or prostate cancer), the abnormal Notch cellular signaling activity plays an important role, which is detectable in the expression profiles of the target genes and thus exploited by means of a calibrated mathematical pathway model.

The present invention makes it possible to determine the activity of the Notch cellular signaling pathway in a subject by (i) determining an activity level of a Notch TF element in the sample of the subject, wherein the determining is based on evaluating a calibrated mathematical model relating the expression levels of the five or more target genes as defined above, of the Notch cellular signaling pathway, the transcription of which is controlled by the Notch TF element, to the activity level of the Notch TF element, and by (ii) inferring the activity of the Notch cellular signaling pathway in the subject based on the determined activity level of the Notch TF element in the sample of the subject. This preferably allows improving the possibilities of characterizing patients that have a disease, such as cancer, e.g., a breast, cervical, endometrial, ovarian, pancreatic or prostate cancer, which is at least partially driven by an abnormal activity of the Notch cellular signaling pathway, and that are therefore likely to respond to inhibitors of the Notch cellular signaling pathway. In particular embodiments, treatment determination can be based on a specific Notch cellular signaling pathway activity. In a particular embodiment, the Notch cellular signaling status can be set at a cutoff value of odds of the Notch cellular signaling pathway being active of, for example, 10:1, 5:1, 4:1, 2:1, 1:1, 1:2, 1:4, 1:5, or 1:10.

Herein, the term "Notch transcription factor element" or "Notch TF element" or "TF element" is defined to be a protein complex containing at least the intracellular domain of one of the Notch proteins (Notch1, Notch2, Notch3 and Notch4, with corresponding intracellular domains N1ICD, N2ICD, N3ICD and N4ICD), with a co-factor, such as the DNA-binding transcription factor CSL (CBF1/RBP-Jκ, Su(H) and LAG-1), which is capable of binding to specific DNA sequences, and preferably one co-activator protein from the mastermind-like (MAML) family (MAML1, MAML2 and MAML3), which is required to activate transcription, thereby controlling transcription of target genes. Preferably, the term refers to either a protein or protein complex transcriptional factor triggered by the cleavage of one of the Notch proteins (Notch1, Notch2, Notch3 and Notch4) resulting in a Notch intracellular domain (N1ICD, N2ICD, N3ICD and N4ICD). For example, it is known that DSL ligands (DLL1, DLL3, DLL4, Jagged1 and Jagged2) expressed on neighboring cells, bind to the extracellular domain of the Notch protein/receptor, initiating the intracellular Notch signaling pathway and that the Notch intracellular domain participates in the Notch signaling cascade which controls expression.

The calibrated mathematical pathway model may be a probabilistic model, preferably a Bayesian network model, based on conditional probabilities relating the activity level of the Notch TF element and the expression levels of the five or more Notch target genes, or the calibrated mathematical pathway model may be based on one or more linear combination(s) of the expression levels of the five or more Notch target genes. In particular, the inferring of the activity of the Notch cellular signaling pathway may be performed as disclosed in the published international patent application WO 2013/011479 A2 ("Assessment of cellular signaling pathway activity using probabilistic modeling of target gene expression") or as described in the published international patent application WO 2014/102668 A2 ("Assessment of cellular signaling pathway activity using linear combination(s) of target gene expressions"), the contents of which are herewith incorporated in their entirety. Further details regarding the inferring of cellular signaling pathway activity using mathematical modeling of target gene expression can be found in Verhaegh W. et al., "Selection of personalized patient therapy through the use of knowledge-based computational models that identify tumor-driving signal transduction pathways", Cancer Research, Vol. 74, No. 11, 2014, pages 2936 to 2945.

The term "subject", as used herein, refers to any living being. In some embodiments, the subject is an animal, preferably a mammal. In certain embodiments, the subject is a human being, preferably a medical subject. In still other embodiments, the subject is a cell line.

The term "target gene" as used herein, means a gene whose transcription is directly or indirectly controlled by a Notch transcription factor element. The "target gene" may be a "direct target gene" and/or an "indirect target gene" (as described herein). Moreover, the "target genes" may be "direct target genes" and/or "indirect target genes" (as described herein).

Particularly suitable Notch target genes which do not define the invention, are described in the following text passages as well as the examples below (see, e.g., Tables 1 to 3 below).

Thus, according to the present disclosure, which is not part of the invention, the Notch target genes are selected from the group consisting of the Notch target genes listed in Table 1, Table 2, or Table 3 below.

It has been found by the present inventors that the Notch target genes in the successively shorter lists become more and more probative for determining the activity of the Notch cellular signaling pathway.

Another aspect of the present invention relates to a method (as described herein), further comprising:
determining whether the Notch cellular signaling pathway is operating abnormally in the subject based on the inferred activity of the Notch cellular signaling pathway in the subject.

The present invention also relates to a method (as described herein), further comprising:

recommending prescribing a drug for the subject that corrects for the abnormal operation of the Notch cellular signaling pathway,
wherein the recommending is performed if the Notch cellular signaling pathway is determined to be operating abnormally in the subject based on the inferred activity of the Notch cellular signaling pathway.

The phrase "the cellular signaling pathway is operating abnormally" refers to the case where the "activity" of the pathway is not as expected, wherein the term "activity" may refer to the activity of the transcription factor complex in driving the target genes to expression, i.e., the speed by which the target genes are transcribed. "Normal" may be when it is inactive in tissue where it is expected to be inactive and active where it is expected to be active. Furthermore, there may be a certain level of activity that is considered "normal", and anything higher or lower maybe considered "abnormal".

The present invention also relates to a method (as described herein), wherein the abnormal operation of the Notch cellular signaling pathway is an operation in which the Notch cellular signaling pathway operates as a tumor promoter in the subject.

The sample(s) to be used in accordance with the present invention can be an extracted sample, that is, a sample that has been extracted from the subject. Examples of the sample include, but are not limited to, a tissue, cells, blood and/or a body fluid of a subject. If the subject is a medical subject that has or may have cancer, it can be, e.g., a sample obtained from a cancer lesion, or from a lesion suspected for cancer, or from a metastatic tumor, or from a body cavity in which fluid is present which is contaminated with cancer cells (e.g., pleural or abdominal cavity or bladder cavity), or from other body fluids containing cancer cells, and so forth, preferably via a biopsy procedure or other sample extraction procedure. The cells of which a sample is extracted may also be tumorous cells from hematologic malignancies (such as leukemia or lymphoma). In some cases, the cell sample may also be circulating tumor cells, that is, tumor cells that have entered the bloodstream and may be extracted using suitable isolation techniques, e.g., apheresis or conventional venous blood withdrawal. Aside from blood, a body fluid of which a sample is extracted may be urine, gastrointestinal contents, or an extravasate. The term "sample", as used herein, also encompasses the case where e.g. a tissue and/or cells and/or a body fluid of the subject have been taken from the subject and, e.g., have been put on a microscope slide, and where for performing the claimed method a portion of this sample is extracted, e.g., by means of Laser Capture Microdissection (LCM), or by scraping off the cells of interest from the slide, or by fluorescence-activated cell sorting techniques. In addition, the term "sample", as used herein, also encompasses the case where e.g. a tissue and/or cells and/or a body fluid of the subject have been taken from the subject and have been put on a microscope slide, and the claimed method is performed on the slide. In addition, the term "sample", as used herein, also encompasses the case where e.g. a cell line and/or cell culture has been generated based on the cells/tissue/body fluid that have been taken from the subject.

In accordance with another disclosed aspect, an apparatus for inferring activity of a Notch cellular signaling pathway in a subject comprises a digital processor configured to perform the method of the present invention as described herein.

In accordance with another disclosed aspect, a non-transitory storage medium for inferring activity of a Notch cellular signaling pathway in a subject stores instructions that are executable by a digital processing device to perform the method of the present invention as described herein. The non-transitory storage medium may be a computer-readable storage medium, such as a hard drive or other magnetic storage medium, an optical disk or other optical storage medium, a random access memory (RAM), read only memory (ROM), flash memory, or other electronic storage medium, a network server, or so forth. The digital processing device may be a handheld device (e.g., a personal data assistant or smartphone), a notebook computer, a desktop computer, a tablet computer or device, a remote network server, or so forth.

In accordance with another disclosed aspect, a computer program for inferring activity of a Notch cellular signaling pathway in a subject comprises program code means for causing a digital processing device to perform the method of the present invention as described herein, when the computer program is run on the digital processing device. The digital processing device may be a handheld device (e.g., a personal data assistant or smartphone), a notebook computer, a desktop computer, a tablet computer or device, a remote network server, or so forth.

In accordance with another disclosed aspect, but not part of the invention, a kit for measuring expression levels of five or more, for example, six, seven, eight, nine, ten, eleven or more, target genes of the Notch cellular signaling pathway in a sample of a subject comprises:

one or more components for determining the expression levels of the five or more Notch target genes in the sample of the subject,
wherein the five or more Notch target genes are selected from the group consisting of: DTX1, EPHB3, HES1, HES4, HES5, HEY2, MYC, NFKB2, NRARP, PIN1, PLXND1, and SOX9, wherein two or more, for example, three, four, five, six or more, Notch target genes are selected from the group consisting of: DTX1, HES1, HES4, HES5, HEY2, MYC, and NRARP, and three or more, for example, four or more, Notch target genes are selected from the group consisting of: EPHB3, NFKB2, PIN1, PLXND1, and SOX9.

The one or more components or means for measuring the expression levels of the five or more Notch target genes can be selected from the group consisting of: a DNA array chip, an oligonucleotide array chip, a protein array chip, an antibody, a plurality of probes, for example, labeled probes, a set of RNA reverse-transcriptase sequencing components, and/or RNA or DNA, including cDNA, amplification primers. In an embodiment, the kit includes a set of labeled probes directed to a portion of an mRNA or cDNA sequence of the five or more Notch target genes as described herein. In an embodiment, the kit includes a set of primers and probes directed to a portion of an mRNA or cDNA sequence of the five or more Notch target genes. In an embodiment, the labeled probes are contained in a standardized 96-well plate. In an embodiment, the kit further includes primers or probes directed to a set of reference genes. Such reference genes can be, for example, constitutively expressed genes useful in normalizing or standardizing expression levels of the target gene expression levels described herein.

Part of the invention is a kit for measuring the expression levels of five or more, for example, six, seven, eight, nine, ten, eleven or more, target genes of the Notch cellular signaling pathway in a sample of a subject comprises:

polymerase chain reaction primers directed to the five or more Notch target genes,
probes directed to the five or more Notch target genes,
wherein the five or more Notch target genes are selected from the group consisting of: DTX1, EPHB3, HES1, HES4, HES5, HEY2, MYC, NFKB2, NRARP, PIN1, PLXND1, and SOX9, wherein two or more, for example, three, four, five, six or more, Notch target genes are selected from the group consisting of: DTX1, HES1, HES4, HES5, HEY2, MYC, and NRARP, and three or more, for example, four or more, Notch target genes are selected from the group consisting of: EPHB3, NFKB2, PIN1, PLXND1, and SOX9.

In accordance with another disclosed aspect, but not part of the invention, a kit for inferring activity of a Notch cellular signaling pathway in a subject comprises:

the kit as described herein, and
the apparatus of the present invention as described herein, the non-transitory storage medium of the present invention as described herein, or the computer program of the present invention as described herein.

In accordance with another disclosed aspect, but not part of the invention, the kits as described herein are used in performing the method of the present invention as described herein.

The present disclosure, but not part of the invention, as described herein can, e.g., also advantageously be used in at least one of the following activities:

diagnosis based on the inferred activity of the Notch cellular signaling pathway in the subject;
prognosis based on the inferred activity of the Notch cellular signaling pathway in the subject;
drug prescription based on the inferred activity of the Notch cellular signaling pathway in the subject;
prediction of drug efficacy based on the inferred activity of the Notch cellular signaling pathway in the subject;
prediction of adverse effects based on the inferred activity of the Notch cellular signaling pathway in the subject;
monitoring of drug efficacy;
drug development;
assay development;
pathway research;
cancer staging;
enrollment of the subject in a clinical trial based on the inferred activity of the Notch cellular signaling pathway in the subject;
selection of subsequent test to be performed; and
selection of companion diagnostics tests.

Further advantages will be apparent to those of ordinary skill in the art upon reading and understanding the attached figures, the following description and, in particular, upon reading the detailed examples provided herein below.

It shall be understood that the method of claim 1, the apparatus of claim 6, the non-transitory storage medium of claim 7, the computer program of claim 8, the kits of claims 9-10, and the use of the kits of claim 11, have similar and/or identical preferred embodiments, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

Fig. 1 shows schematically and exemplarily the Notch cellular signaling pathway. The pathway is activated when the Notch extracellular domain binds to a DSL-ligand. After cleavage of the receptor the Notch intracellular domain moves to the nucleus and forms, together with other proteins, an active transcription factor complex (see Guruharsha K.G. et al., "The Notch signaling system: recent insights into the complexity of a conserved pathway" Nature Reviews Genetics, Vol. 13, September 2012, pages 654 to 666; "TS" = transcriptional switch; "TG" = target genes).

Fig. 2 shows schematically and exemplarily a mathematical model, herein, a Bayesian network model, used to model the transcriptional program of the Notch cellular signaling pathway.

Fig. 3 shows a flow chart exemplarily illustrating a process for inferring activity of the Notch cellular signaling pathway in a subject based on expression levels of target genes of the Notch cellular signaling pathway measured in a sample of a subject.

Fig. 4 shows a flow chart exemplarily illustrating a process for obtaining a calibrated mathematical pathway model as described herein.

Fig. 5 shows a flow chart exemplarily illustrating a process for determining an activity level of a Notch transcription factor (TF) element in a sample of a subject as described herein.

Fig. 6 shows a flow chart exemplarily illustrating a process for inferring activity of a Notch cellular signaling pathway in a subject using discretized observables.

Fig. 7 shows a flow chart exemplarily illustrating a process for inferring activity of a Notch cellular signaling pathway in a subject using continuous observables.

Fig. 8 shows a flow chart exemplarily illustrating a process for determining Cq values from RT-qPCR analysis of the target genes of the Notch cellular signaling pathway.

Fig. 9 shows calibration results of the Bayesian network model based on the 18 target genes shortlist from Table 2 and the methods as described herein using publically available expression data sets of 11 normal ovary (group 1) and 20 high grade papillary serous ovarian carcinoma (group 2) samples (subset of samples taken from data sets GSE2109, GSE9891, GSE7307, GSE18520, GSE29450, GSE36668).

Fig. 10 shows calibration results of the Bayesian network model based on the evidence curated list of target genes (26 target genes list) from Table 1 and the methods as described herein using publically available expression data sets of 11 normal ovary (group 1) and 20 high grade papillary serous ovarian carcinoma (group 2) samples (subset of samples taken from data sets GSE2109, GSE9891, GSE7307, GSE18520, GSE29450, GSE36668).

Fig. 11 shows Notch cellular signaling pathway activity predictions of the trained exemplary Bayesian network model using the 18 target genes shortlist from Table 2 on three independent cultures of the MOLT4 cell line from data set GSE6495.

Fig. 12 shows Notch cellular signaling pathway activity predictions of the trained exemplary Bayesian network model using the evidence curated list of target genes (26 target genes list) from Table 2 on three independent cultures of the MOLT4 cell line from data set GSE6495.

Fig. 13 shows Notch cellular signaling pathway activity predictions of the trained exemplary Bayesian network model using the 18 target genes shortlist from Table 2 on IMR32 cells that were transfected with an inducible Notch3-intracellular construct.

Fig. 14 shows Notch cellular signaling pathway activity predictions of the trained exemplary Bayesian network model using the 18 target genes shortlist from Table 2 on CD34+CD45RA-Lin-HPCs that were cultured for 72 hrs with graded doses of plastic-immobilized Notch ligand Delta1ext-IgG (data set GSE29524).

Fig. 15 shows Notch cellular signaling pathway activity predictions of the trained exemplary Bayesian network model using the 18 target genes shortlist from Table 2 on CUTLL1 cells, which are known to have high Notch activity.

Fig. 16 shows Notch cellular signaling pathway activity predictions of the trained exemplary Bayesian network model using the evidence curated list of target genes (26 target gene list) from Table 1 on CUTLL1 cells, which are known to have high Notch activity.

Fig. 17 shows Notch cellular signaling pathway activity predictions of the trained exemplary Bayesian network model using the 18 target genes shortlist from Table 2 on HUVEC cells that were transfected with COUP-TFII siRNA (data set GSE33301).

Fig. 18 shows Notch cellular signaling pathway activity predictions of the trained exemplary Bayesian network model using the 18 target genes shortlist from Table 2 on breast cancer subgroups in samples from GSE6532, GSE9195, GSE12276, GSE20685, GSE21653 and EMTAB365.

Fig. 19 shows Notch cellular signaling pathway activity predictions of the trained exemplary Bayesian network model using the 12 target genes shortlist from Table 3 on CD34+CD45RA-Lin-HPCs that were cultured for 72 hrs with graded doses of plastic-immobilized Notch ligand Deltalext-IgG (data set GSE29524).

Fig. 20 shows Notch cellular signaling pathway activity predictions of the trained exemplary Bayesian network model using the 12 target genes shortlist from Table 3 on CUTLL1 cells, which are known to have high Notch activity.

Fig. 21 shows the correlation between the trained exemplary Bayesian network mode using the evidence curated list of target genes (26 target genes list) from Table 1 and the 12 target genes shortlist from Table 3, respectively.

Fig. 22 shows a comparison of the Notch cellular signaling pathway activity predictions using the list of 7 Notch target genes vs. the list of 10 Notch target genes.

Fig. 23 shows a comparison of the Notch cellular signaling pathway activity predictions using the list of 8 Notch target genes vs. the list of 12 Notch target genes.

Fig. 24 shows calibration results of the Bayesian model based on the 10 target genes mouse list from Table 6 and the methods as described herein using publically available expression dataset GSE15268 containing 2 control Embryonic Stem Cells (ESc), 2 control Mesodermal Progenitor Cells (MPc), 2 ESc samples containing a tamoxifen inducible NERT construct (NotchIC), not OHT treated, 2 ESc samples containing a tamoxifen inducible NERT construct (NotchIC), OHT treated, 4 MPc samples containing a tamoxifen inducible NERT construct (NotchIC), not OHT treated and 4 MPc samples containing a tamoxifen inducible NERT construct (NotchIC), OHT treated.

Fig. 25 shows Notch cellular signaling pathway activity predictions of the trained exemplary Bayesian network model using the 10 target genes mouse list from Table 6 on mouse mammary glands with an inducible constitutively active Notch1 intracellular domain (NICD1) (data set GSE51628).

Fig. 26 shows Notch cellular signaling pathway activity predictions of the trained exemplary Bayesian network model using the 10 target genes mouse list from Table 6 on mouse yolk sac tissue with an conditional transgenic system to activate Notch1 and mouse yolk sac tissue from transgenic mouse with RBPJ (part of the Notch transcription factor complex) loss-of-function (data set GSE22418).

Fig. 27 shows Notch cellular signaling pathway activity predictions of the trained exemplary Bayesian network model using the 10 target genes mouse list from Table 6 on mouse bone marrow cells (adult myeloerythroid progenitors) with a conditional gain of function allele of Notch2 receptor (data set GSE46724).

DETAILED DESCRIPTION OF EMBODIMENTS

The following examples merely illustrate particularly preferred methods and selected aspects in connection therewith. The teaching provided therein may be used for constructing several tests and/or kits, e.g., to detect, predict and/or diagnose the abnormal activity of the Notch cellular signaling pathway. Furthermore, upon using methods as described herein drug prescription can advantageously be guided, drug response prediction and monitoring of drug efficacy (and/or adverse effects) can be made, drug resistance can be predicted and monitored, e.g., to select subsequent test(s) to be performed (like a companion diagnostic test). The following examples are not to be construed as limiting the scope of the present invention.

**Example 1: Mathematical model construction**

As described in detail in the published international patent application WO 2013/011479 A2 ("Assessment of cellular signaling pathway activity using probabilistic modeling of target gene expression"), by constructing a probabilistic model, e.g., a Bayesian network model, and incorporating conditional probabilistic relationships between the expression levels of five or more target genes of a cellular signaling pathway, herein, the Notch cellular signaling pathway, and the activity level of a transcription factor (TF) element, herein, the Notch TF element, the TF element controlling transcription of the five or more target genes of the cellular signaling pathway, such a model may be used to determine the activity of the cellular signaling pathway with a high degree of accuracy. Moreover, the probabilistic model can be readily updated to incorporate additional knowledge obtained by later clinical studies, by adjusting the conditional probabilities and/or adding new nodes to the model to represent additional information sources. In this way, the probabilistic model can be updated as appropriate to embody the most recent medical knowledge.

In another easy to comprehend and interpret approach described in detail in the published international patent application WO 2014/102668 A2 ("Assessment of cellular signaling pathway activity using linear combination(s) of target gene expressions"), the activity of a cellular signaling pathway, herein, the Notch cellular signaling pathway, may be determined by constructing and evaluating a linear or (pseudo-)linear model incorporating relationships between expression levels of five or more target genes of the cellular signaling pathway and the level of a transcription factor (TF) element, herein, the Notch TF element, the TF element controlling transcription of the five or more target genes of the cellular signaling pathway, the model being based on one or more linear combination(s) of expression levels of the five or more target genes.

In both approaches, the expression levels of the five or more target genes may preferably be measurements of the level of mRNA, which can be the result of, e.g., (RT)-PCR and microarray techniques using probes associated with the target genes mRNA sequences, and of RNA-sequencing. In another embodiment, the expression levels of the five or more target genes can be measured by protein levels, e.g., the concentrations and/or activity of the protein(s) encoded by the target genes.

The aforementioned expression levels may optionally be converted in many ways that might or might not suit the application better. For example, four different transformations of the expression levels, e.g., microarray-based mRNA levels, may be:

- "continuous data", i.e., expression levels as obtained after preprocessing of microarrays using well known algorithms such as MAS5.0 and fRMA,
- "z-score", i.e., continuous expression levels scaled such that the average across all samples is 0 and the standard deviation is 1,
- "discrete", i.e., every expression above a certain threshold is set to 1 and below it to 0 (e.g., the threshold for a probeset may be chosen as the (weighted) median of its value in a set of a number of positive and the same number of negative clinical samples),
- "fuzzy", i.e., the continuous expression levels are converted to values between 0 and 1 using a sigmoid function of the following format: 1 / (1 + *exp*((*thr* - *expr*) / *se*)), with *expr* being the continuous expression levels, *thr* being the threshold as mentioned before and *se* being a softening parameter influencing the difference between 0 and 1.

One of the simplest linear models that can be constructed is a model having a node representing the transcription factor (TF) element, herein, the Notch TF element, in a first layer and weighted nodes representing direct measurements of the target genes expression levels, e.g., by one probeset that is particularly highly correlated with the particular target gene, e.g., in microarray or (q)PCR experiments, in a second layer. The weights can be based either on calculations from a training data set or based on expert knowledge. This approach of using, in the case where possibly multiple expression levels are measured per target gene (e.g., in the case of microarray experiments, where one target gene can be measured with multiple probesets), only one expression level per target gene is particularly simple. A specific way of selecting the one expression level that is used for a particular target gene is to use the expression level from the probeset that is able to separate active and passive samples of a training data set the best. One method to determine this probeset is to perform a statistical test, e.g., the t-test, and select the probeset with the lowest p-value. The training data set's expression levels of the probeset with the lowest p-value is by definition the probeset with the least likely probability that the expression levels of the (known) active and passive samples overlap. Another selection method is based on odds-ratios. In such a model, one or more expression level(s) are provided for each of the five or more target genes and the one or more linear combination(s) comprise a linear combination including for each of the five or more target genes a weighted term, each weighted term being based on only one expression level of the one or more expression level(s) provided for the respective target gene. If only one expression level is chosen per target gene as described above, the model may be called a "most discriminant probesets" model.

In an alternative to the "most discriminant probesets" model, it is possible, in the case where possibly multiple expression levels are measured per target gene, to make use of all the expression levels that are provided per target gene. In such a model, one or more expression level(s) are provided for each of the five or more target genes and the one or more linear combination(s) comprise a linear combination of all expression levels of the one or more expression level(s) provided for the five or more target genes. In other words, for each of the five or more target genes, each of the one or more expression level(s) provided for the respective target gene may be weighted in the linear combination by its own (individual) weight. This variant may be called an "all probesets" model. It has an advantage of being relatively simple while making use of all the provided expression levels.

Both models as described above have in common that they are what may be regarded as "single-layer" models, in which the activity level of the TF element is calculated based on a linear combination of expression levels of the one or more probeset of the five or more target genes.

After the activity level of the TF element, herein, the Notch TF element, has been determined by evaluating the respective model, the determined TF element activity level can be thresholded in order to infer the activity of the cellular signaling pathway, herein, the Notch cellular signaling pathway. A preferred method to calculate such an appropriate threshold is by comparing the determined TF element activity levels *wlc* (weighted linear combination) of training samples known to have a passive cellular signaling pathway and training samples with an active cellular signaling pathway. A method that does so and also takes into account the variance in these groups is given by using a threshold

$$thr = \frac{\sigma_{wlc_{\text{pas}}}\,\mu_{wlc_{\text{act}}} + \sigma_{wlc_{\text{act}}}\mu_{wlc_{\text{pas}}}}{\sigma_{wlc_{\text{pas}}} + \sigma_{wlc_{\text{act}}}} \quad (1)$$

where σ and μ are the standard deviation and the mean of the determined TF element activity levels *wlc* for the training samples. In case only a small number of samples are available in the active and/or passive training samples, a pseudo count may be added to the calculated variances based on the average of the variances of the two groups:

$$\begin{aligned} \tilde{v} &= \frac{v_{wlc_{act}} + v_{wlc_{pas}}}{2} \\ \tilde{v}_{wlc_{act}} &= \frac{x\,\tilde{v} + (n_{act} - 1)v_{wlc_{act}}}{x + n_{act} - 1} \\ \tilde{v}_{wlc_{pas}} &= \frac{x\,\tilde{v} + (n_{pas} - 1)v_{wlc_{pas}}}{x + n_{pas} - 1} \end{aligned} \quad (2)$$

where *v* is the variance of the determined TF element activity levels *wlc* of the groups, *x* is a positive pseudo count, e.g., 1 or 10, and $n_{act}$ and $n_{pas}$ are the number of active and passive samples, respectively. The standard deviation σ can next be obtained by taking the square root of the variance *v*.

The threshold can be subtracted from the determined TF element activity levels *wlc* for ease of interpretation, resulting in a cellular signaling pathway's activity score in which negative values correspond to a passive cellular signaling pathway and positive values correspond to an active cellular signaling pathway.

As an alternative to the above-described "single-layer" models, a "two-layer" may also be used in an example. In such a model, a summary value is calculated for every target gene using a linear combination based on the measured intensities of its associated probesets ("first (bottom) layer"). The calculated summary value is subsequently combined with the summary values of the other target genes of the cellular signaling pathway using a further linear combination ("second (upper) layer"). Again, the weights can be either learned from a training data set or based on expert knowledge or a combination thereof. Phrased differently, in the "two-layer" model, one or more expression level(s) are provided for each of the five or more target genes and the one or more linear combination(s) comprise for each of the five or more target genes a first linear combination of all expression levels of the one or more expression level(s) provided for the respective target gene ("first (bottom) layer"). The model is further based on a further linear combination including for each of the five or more target genes a weighted term, each weighted term being based on the first linear combination for the respective target gene ("second (upper) layer").

The calculation of the summary values can, in a preferred version of the "two-layer" model, include defining a threshold for each target gene using the training data and subtracting the threshold from the calculated linear combination, yielding the target gene summary. Here the threshold may be chosen such that a negative target gene summary value corresponds to a down-regulated target gene and that a positive target gene summary value corresponds to an up-regulated target gene. Also, it is possible that the target gene summary values are transformed using, e.g., one of the above-described transformations (fuzzy, discrete, etc.), before they are combined in the "second (upper) layer".

After the activity level of the TF element has been determined by evaluating the "two-layer" model, the determined TF element activity level can be thresholded in order to infer the activity of the cellular signaling pathway, as described above.

In the following, the models described above are collectively denoted as "(pseudo-)linear" models. A more detailed description of the training and use of probabilistic models, e.g., a Bayesian network model, is provided in Example 3 below.

**Example 2: Selection of target genes**

A transcription factor (TF) is a protein complex (i.e., a combination of proteins bound together in a specific structure) or a protein that is able to regulate transcription from target genes by binding to specific DNA sequences, thereby controlling the transcription of genetic information from DNA to mRNA. The mRNA directly produced due to this action of the TF complex is herein referred to as a "direct target gene" (of the transcription factor). Cellular signaling pathway activation may also result in more secondary gene transcription, referred to as "indirect target genes". In the following, (pseudo-)linear models or Bayesian network models (as exemplary mathematical models) comprising or consisting of direct target genes as direct links between cellular signaling pathway activity and mRNA level, are preferred, however the distinction between direct and indirect target genes is not always evident. Herein, a method to select direct target genes using a scoring function based on available scientific literature data is presented. Nonetheless, an accidental selection of indirect target genes cannot be ruled out due to limited information as well as biological variations and uncertainties. In order to select the target genes, the MEDLINE database of the National Institute of Health accessible at "www.ncbi.nlm.nih.gov/pubmed" and herein further referred to as "Pubmed" was employed to generate a list of target genes. Furthermore, two additional lists of target genes were selected based on the probative nature of their expression.

Publications containing putative Notch target genes were searched for by using queries such as ("Notch" AND "target gene") in the period of the fourth quarter of 2016 and the first quarter of 2017. The Notch pathway is an embryonic pathway that activates different (but overlapping) target gene profiles depending on the embryonic lineage (see Meier-Stiegen F. et al., "Activated Notch1 target genes during embryonic cell differentiation depend on the cellular context and include lineage determinants and inhibitors", PLoS One, Vol. 5, No. 7, July 2010). The search was focused on sets of target genes that are differentially expressed between cell type / tissue / organ derivatives from the three different embryonic lineages (ectoderm, endoderm, mesoderm), with a specific emphasis on target genes that are expressed in ectodermal and endodermal derived organs/tissues/cells. The resulting publications were further analyzed manually following the methodology described in more detail below.

Specific cellular signaling pathway mRNA target genes were selected from the scientific literature, by using a ranking system in which scientific evidence for a specific target gene was given a rating, depending on the type of scientific experiments in which the evidence was accumulated. While some experimental evidence is merely suggestive of a gene being a direct target gene, like for example an mRNA increasing as detected by means of an increasing intensity of a probeset on a microarray of a cell line in which it is known that the Notch cellular signaling pathway is active, other evidence can be very strong, like the combination of an identified Notch cellular signaling pathway TF binding site and retrieval of this site in a chromatin immunoprecipitation (ChIP) assay after stimulation of the specific cellular signaling pathway in the cell and increase in mRNA after specific stimulation of the cellular signaling pathway in a cell line.

Several types of experiments to find specific cellular signaling pathway target genes can be identified in the scientific literature:

1. ChIP experiments in which direct binding of a TF of the cellular signaling pathway of interest to its binding site on the genome is shown. Example: By using chromatin immunoprecipitation (ChIP) technology putative functional Notch TF binding sites in the DNA of cell lines with and without active induction of the Notch cellular signaling pathway, e.g., by stimulation with a Notch ligand or transfection with NICD, were identified, as a subset of the binding sites recognized purely based on nucleotide sequence. Putative functionality was identified as ChIP-derived evidence that the TF was found to bind to the DNA binding site.

2. Electrophoretic Mobility Shift (EMSA) assays which show in vitro binding of a TF to a fragment of DNA containing the binding sequence. Compared to ChIP-based evidence EMSA-based evidence is less strong, since it cannot be translated to the in vivo situation.

3. Stimulation of the cellular signaling pathway and measuring mRNA expression using a microarray, RNA sequencing, quantitative PCR or other techniques, using Notch cellular signaling pathway-inducible cell lines and measuring mRNA profiles measured at least one, but preferably several time points after induction - in the presence of cycloheximide, which inhibits translation to protein, thus the induced mRNAs are assumed to be direct target genes.

4. Similar to 3, but alternatively measure the mRNAs expression further downstream with protein abundance measurements, such as western blot.

5. Inhibition of the cellular signaling pathway using a Notch inhibitor, e.g., a Gamma-Secretase Inhibitor (GSI) and measuring mRNA expression using a microarray, RNA sequencing, quantitative PCR or other techniques, using Notch cellular signaling pathway-active cell lines and measuring mRNA profiles measured at least one, but preferably several time points after inhibition.

6. Similar to 5, but alternatively measure the mRNAs expression further downstream with protein abundance measurements, such as western blot.

7. Identification of TF binding sites in the genome using a bioinformatics approach. Example for the Notch TF element: Using the CSL/RBP-J binding motif 5'-CGTGGGAA-3', a software program was run on the human genome sequence, and potential binding sites were identified, both in gene promoter regions and in other genomic regions.

8. Similar as 3, only in the absence of cycloheximide.

9. Similar to 4, only in the absence of cycloheximide.

In the simplest form one can give every potential gene 1 point for each of these experimental approaches in which the gene was identified as being a target gene of the Notch family of transcription factors. Using this relative ranking strategy, one can make a list of most reliable target genes.

Alternatively, ranking in another way can be used to identify the target genes that are most likely to be direct target genes, by giving a higher number of points to the technology that provides most evidence for an in vivo direct target gene. In the list above, this would mean 9 points for experimental approach 1), 8 for 2), and going down to 1 point for experimental approach 9). Such a list may be called a "general list of target genes".

Despite the biological variations and uncertainties, the inventors assumed that the direct target genes are the most likely to be induced in a tissue-independent manner. A list of these target genes may be called an "evidence curated list of target genes". Such an evidence curated list of target genes has been used to construct computational models of the Notch cellular signaling pathway that can be applied to samples coming from different tissue sources.

The following will illustrate exemplary how the selection of an evidence curated target gene list specifically was constructed for the Notch cellular signaling pathway.

A scoring function was introduced that gave a point for each type of experimental evidence, such as ChIP, EMSA, differential expression, knock down/out, luciferase gene reporter assay, sequence analysis, that was reported in a publication. Further analysis was performed to allow only for genes that had diverse types of experimental evidence and not only one or two types of experimental evidence, e.g., differential expression. Those genes that had more than two types of experimental evidence available were selected (as shown in Table 1).

A further selection of the evidence curated list of target genes (listed in Table 2, "18 target genes shortlist") was made by the inventors. This selection was made by removing target genes of the evidence curated list that had relatively little evidence, e.g. evidence was found in only one manuscript, and/or were highly specific, e.g. for blood or brain tissue. The target genes of the "18 target genes shortlist" that were proven to be more probative in determining the activity of the Notch signaling pathway from the training samples were selected for the "12 target genes shortlist" (listed in Table 3, "12 target genes shortlist"). Herein, the 12 target genes that had the highest odds ratio (see below) between patient samples from respectively a set of high grade papillary serous ovarian cancer patients (Notch active, subset taken from GSE2109 and GSE9891, from the gene expression omnibus (GEO, www.ncbi.nlm.nih.gov/ geo/, last accessed December 3rd, 2016, and a corresponding set of normal ovarian tissue samples (Notch inactive, subset taken from GSE7307, GSE18520, GSE29450 and GSE36668), and/or scored very high on the evidence ranking, were selected.

Table 1: "Evidence curated list of target genes" (26 target genes list) of the Notch cellular signaling pathway used in the Notch cellular signaling pathway models and associated probesets used to measure the mRNA expression level of the target genes.

| Target gene | Probeset | Target gene | Probeset |
|---|---|---|---|
| CD28 | 206545_at | HEY2 | 219743_at |
| | 211856_x_at | | 222921_s_at |
| | 211861_x_at | HEYL | 220662_s_at |
| CD44 | 1557905_s_at | | 226828_s_at |
| | 204489_s_at | KLF5 | 209211_at |
| | 204490_s_at | | 209212_s_at |
| | 209835_x_at | MYC | 202431_s_at |
| | 210916_s_at | NFKB2 | 207535_s_at |
| | 212014_x_at | | 209636_at |
| | 212063_at | | 211524_at |
| DLGAP5 | 203764_at | NOX1 | 206418_at |
| DTX1 | 227336_at | | 207217_s_at |
| EPHB3 | 1438_at | | 207380_x_at |
| | 204600_at | | 210808_s_at |
| FABP7 | 205029_s_at | NRARP | 226499_at |
| | 205030_at | PBX1 | 205253_at |
| | 216192_at | PIN1 | 202927_at |
| GFAP | 203540_at | PLXND1 | 1563657_at |
| | 229259_at | | 212235_at |
| GIMAP5 | 218805_at | | 38671_at |
| | 64064_at | PTCRA | 211252_x_at |
| HES1 | 203393_at | | 211837_s_at |
| | 203394_s_at | | 215492_x_at |
| | 203395_s_at | SOX9 | 202935_s_at |
| HES4 | 227347_x_at | | 202936_s_at |
| HES5 | 239230_at | TNC | 201645_at |
| HES7 | 224548_at | | 237169_at |
| HEY1 | 218839_at | | |
| | 44783_s_at | | |

Table 2: "18 target genes shortlist" of Notch target genes based on the evidence curated list of Notch target genes. (The associated probesets are the same as in Table 1.)

| Target gene |
|---|
| CD44 |
| DTX1 |
| EPHB3 |
| HES1 |

(continued)

| Target gene |
|---|
| HES4 |
| HES5 |
| HES7 |
| HEY1 |
| HEY2 |
| HEYL |
| MYC |
| NFKB2 |
| NOX1 |
| NRARP |
| PBX1 |
| PIN1 |
| PLXND1 |
| SOX9 |

Table 3: "12 target genes shortlist" of Notch target genes based on the evidence curated list of Notch target genes. (The associated probesets are the same as in Table 1.)

| Target gene |
|---|
| DTX1 |
| EPHB3 |
| HES1 |
| HES4 |
| HES5 |
| HEY2 |
| MYC |
| NFKB2 |
| NRARP |
| PIN1 |
| PLXND1 |
| SOX9 |

**Example 3: Training and using the mathematical model**

Before the mathematical model can be used to infer the activity of the cellular signaling pathway, herein, the Notch cellular signaling pathway, in a subject, the model must be appropriately trained.

If the mathematical pathway model is a probabilistic model, e.g., a Bayesian network model, based on conditional probabilities relating the activity level of the Notch TF element and expression levels of five or more target genes of the Notch cellular signaling pathway measured in the sample of the subject, the training may preferably be performed as described in detail in the published international patent application WO 2013/011479 A2 ("Assessment of cellular signaling pathway activity using probabilistic modeling of target gene expression").

If the mathematical pathway model is based on one or more linear combination(s) of expression levels of five or more target genes of the Notch cellular signaling pathway measured in the sample of the subject, the training may preferably be performed as described in detail in the published international patent application WO 2014/102668 A2 ("Assessment of cellular signaling pathway activity using linear combination(s) of target gene expressions").

Herein, an exemplary Bayesian network model as shown in Fig. 2 was used to model the transcriptional program of the Notch cellular signaling pathway in a simple manner. The model consists of three types of nodes: (a) a transcription factor (TF) element (with states "absent" and "present") in a first layer 1; (b) target genes $TG_1$, $TG_2$, $TG_n$ (with states "down" and "up") in a second layer 2, and; (c) measurement nodes linked to the expression levels of the target genes in a third layer 3. These can be microarray probesets $PS_{1,1}$, $PS_{1,2}$, $PS_{1,3}$, $PS_{2,1}$, $PS_{n,1}$, $PS_{n,m}$ (with states "low" and "high"), as preferably used herein, but could also be other gene expression measurements such as RNAseq or RT-qPCR.

A suitable implementation of the mathematical model, herein, the exemplary Bayesian network model, is based on microarray data. The model describes (i) how the expression levels of the target genes depend on the activation of the TF element, and (ii) how probeset intensities, in turn, depend on the expression levels of the respective target genes. For the latter, probeset intensities may be taken from fRMA pre-processed Affymetrix HG-U133Plus2.0 microarrays, which are widely available from the Gene Expression Omnibus (GEO, www.ncbi.nlm.nih.gov/geo) and ArrayExpress (www.ebi.ac.uk/arrayexpress).

As the exemplary Bayesian network model is a simplification of the biology of a cellular signaling pathway, herein, the Notch cellular signaling pathway, and as biological measurements are typically noisy, a probabilistic approach was opted for, i.e., the relationships between (i) the TF element and the target genes, and (ii) the target genes and their respective probesets, are described in probabilistic terms. Furthermore, it was assumed that the activity of the oncogenic cellular signaling pathway which drives tumor growth is not transiently and dynamically altered, but long term or even irreversibly altered. Therefore the exemplary Bayesian network model was developed for interpretation of a static cellular condition. For this reason complex dynamic cellular signaling pathway features were not incorporated into the model.

Once the exemplary Bayesian network model is built and calibrated (see below), the model can be used on microarray data of a new sample by entering the probeset measurements as observations in the third layer 3, and inferring backwards in the model what the probability must have been for the TF element to be "present". Here, "present" is considered to be the phenomenon that the TF element is bound to the DNA and is controlling transcription of the cellular signaling pathway's target genes, and "absent" the case that the TF element is not controlling transcription. This probability is hence the primary read-out that may be used to indicate activity of the cellular signaling pathway, herein, the Notch cellular signaling pathway, which can next be translated into the odds of the cellular signaling pathway being active by taking the ratio of the probability of it being active vs. it being passive (i.e., the odds are given by p/(1-p), where p is the predicted probability of the cellular signaling pathway being active).

In the exemplary Bayesian network model, the probabilistic relations have been made quantitative to allow for a quantitative probabilistic reasoning. In order to improve the generalization behavior across tissue types, the parameters describing the probabilistic relationships between (i) the TF element and the target genes have been carefully hand-picked. If the TF element is "absent", it is most likely that the target gene is "down", hence a probability of 0.95 is chosen for this, and a probability of 0.05 is chosen for the target gene being "up". The latter (non-zero) probability is to account for the (rare) possibility that the target gene is regulated by other factors or that it is accidentally observed as being "up" (e.g. because of measurement noise). If the TF element is "present", then with a probability of 0.70 the target gene is considered "up", and with a probability of 0.30 the target gene is considered "down". The latter values are chosen this way, because there can be several causes why a target gene is not highly expressed even though the TF element is present, e.g., because the gene's promoter region is methylated. In the case that a target gene is not up-regulated by the TF element, but down-regulated, the probabilities are chosen in a similar way, but reflecting the down-regulation upon presence of the TF element. The parameters describing the relationships between (ii) the target genes and their respective probesets have been calibrated on experimental data. For the latter, in this example, microarray data was used from patients samples which are known to have an active Notch cellular signaling pathway whereas normal, healthy samples from a different data set were used as passive Notch cellular signaling pathway samples, but this could also be performed using cell line experiments or other patient samples with known cellular signaling pathway activity status. The resulting conditional probability tables are given by:

*A: for upregulated target genes*

| | $PS_{i,j}$ = low | $PS_{i,j}$ = high |
|---|---|---|
| $TG_i$ = down | $\frac{AL_{i,j}+1}{AL_{i,j}+AH_{i,j}+2}$ | $\frac{AH_{i,j}+1}{AL_{i,j}+AH_{i,j}+2}$ |

(continued)

| | $PS_{i,j}$ = low | $PS_{i,j}$ = high |
|---|---|---|
| $TG_i$ = up | $\frac{PL_{i,j}+1}{PL_{i,j}+PH_{i,j}+2}$ | $\frac{PH_{i,j}+1}{PL_{i,j}+PH_{i,j}+2}$ |

*B: for downregulated target genes*

| | $PS_{i,j}$ = low | $PS_{i,j}$ = high |
|---|---|---|
| $TG_i$ = down | $\frac{PL_{i,j}+1}{PL_{i,j}+PH_{i,j}+2}$ | $\frac{PH_{i,j}+1}{PL_{i,j}+PH_{i,j}+2}$ |
| $TG_i$ = up | $\frac{AL_{i,j}+1}{AL_{i,j}+AH_{i,j}+2}$ | $\frac{AH_{i,j}+1}{AL_{i,j}+AH_{i,j}+2}$ |

In these tables, the variables $AL_{i,j}$, $AH_{i,j}$, $PL_{i,j}$, and $PH_{i,j}$ indicate the number of calibration samples with an "absent" (A) or "present" (P) transcription complex that have a "low" (L) or "high" (H) probeset intensity, respectively. Dummy counts have been added to avoid extreme probabilities of 0 and 1.

To discretize the observed probeset intensities, for each probeset $PS_{i,j}$ a threshold $t_{i,j}$ was used, below which the observation is called "low", and above which it is called "high". This threshold has been chosen to be the (weighted) median intensity of the probeset in the used calibration data set. Due to the noisiness of microarray data, a fuzzy method was used when comparing an observed probeset intensity to its threshold, by assuming a normal distribution with a standard deviation of 0.25 (on a log2 scale) around the reported intensity, and determining the probability mass below and above the threshold.

If instead of the exemplary Bayesian network described above, a (pseudo-)linear model as described in Example 1 above was employed, the weights indicating the sign and magnitude of the correlation between the nodes and a threshold to call whether a node is either "absent" or "present" would need to be determined before the model could be used to infer cellular signaling pathway activity in a test sample. One could use expert knowledge to fill in the weights and the threshold a priori, but typically the model would be trained using a representative set of training samples, of which preferably the ground truth is known, e.g., expression data of probesets in samples with a known "present" transcription factor complex (= active cellular signaling pathway) or "absent" transcription factor complex (= passive cellular signaling pathway).

Known in the field are a multitude of training algorithms (e.g., regression) that take into account the model topology and changes the model parameters, here, the weights and the threshold, such that the model output, here, a weighted linear score, is optimized. Alternatively, it is also possible to calculate the weights directly from the observed expression levels without the need of an optimization algorithm.

A first method, named "black and white"-method herein, boils down to a ternary system, in which each weight is an element of the set {-1, 0, 1}. If this is put in a biological context, the -1 and 1 correspond to target genes or probesets that are down- and up-regulated in case of cellular signaling pathway activity, respectively. In case a probeset or target gene cannot be statistically proven to be either up- or down-regulated, it receives a weight of 0. In one example, a left-sided and right-sided, two sample t-test of the expression levels of the active cellular signaling pathway samples versus the expression levels of the samples with a passive cellular signaling pathway can be used to determine whether a probe or gene is up- or down-regulated given the used training data. In cases where the average of the active samples is statistically larger than the passive samples, i.e., the p-value is below a certain threshold, e.g., 0.3, the target gene or probeset is determined to be up-regulated. Conversely, in cases where the average of the active samples is statistically lower than the passive samples, the target gene or probeset is determined to be down-regulated upon activation of the cellular signaling pathway. In case the lowest p-value (left- or right-sided) exceeds the aforementioned threshold, the weight of the target gene or probeset can be defined to be 0.

A second method, named "log odds"-weights herein, is based on the logarithm (e.g., base e) of the odds ratio. The odds ratio for each target gene or probeset is calculated based on the number of positive and negative training samples for which the probeset/target gene level is above and below a corresponding threshold, e.g., the (weighted) median of all training samples. A pseudo-count can be added to circumvent divisions by zero. A further refinement is to count the samples above/below the threshold in a somewhat more probabilistic manner, by assuming that the probeset/target gene levels are e.g. normally distributed around its observed value with a certain specified standard deviation (e.g., 0.25 on a 2-log scale), and counting the probability mass above and below the threshold. Herein, an odds ratio calculated in combination with a pseudo-count and using probability masses instead of deterministic measurement values is called a "soft" odds ratio.

Further details regarding the inferring of cellular signaling pathway activity using mathematical modeling of target gene expression can be found in Verhaegh W. et al., "Selection of personalized patient therapy through the use of knowledge-based computational models that identify tumor-driving signal transduction pathways", Cancer Research, Vol. 74, No. 11, 2014, pages 2936 to 2945.

Herein, we have used publically available data on the expression of patient samples from respectively a set of high grade papillary serous ovarian cancer patients (data sets GSE2109 and GSE9891, from the gene expression omnibus (GEO, www.ncbi.nlm. nih.gov/geo/, last accessed December 3rd, 2016) and a corresponding set of normal ovarian tissue samples (data sets GSE7307, GSE18520, GSE29450 and GSE36668). High grade serous ovarian cancer is known to have an active Notch cellular signaling pathway in the majority of cases while normal ovarian tissue samples have a passive Notch cellular signaling pathway. Before selecting calibration samples, a quality control was performed on the data sets to ensure that samples were reliable. For calibration purposes, the most active Notch ovarian cancer samples were chosen from the available sets, as determined by adding Affymetrix mRNA expression values for all target genes, for each individual sample and subsequently ranking the samples according to total value. The 20 highest ranking samples were assumed to be Notch active. From the 12 normal ovary samples that passed the quality control, 11 samples were chosen as Notch passive calibration samples (1 normal ovary sample was found to be Notch active), sample numbers: GSM176237, GSM729048, GSM462651, GSM729050, GSM729051, GSM175789, GSM462652, GSM176131, GSM176318, GSM898306, GSM898307. (Samples from data set GSE42259 were also considered as Notch passive calibration samples, but after a quality control none of these samples remained.) These were used to calibrate the model for Notch activity and passivity respectively. The calibrated model was evaluated on a number of public data sets from the GEO database, which contained a ground truth with respect to Notch activity, that is, cell lines in which Notch activity was either induced or inhibited (e.g. treated with a Notch inhibitor like gamma-secretase, or having the possibility to induce Notch3-intracellular). As an application example, the model was run on a data set of breast cancer samples for which survival data is known.

Fig. 9 shows calibration results of the Bayesian network model based on the 18 target genes shortlist from Table 2 and the methods as described herein using publically available expression data sets of 11 normal ovary (group 1) and 20 high grade papillary serous ovarian carcinoma (group 2) samples (subset of samples taken from data sets GSE2109, GSE9891, GSE7307, GSE18520, GSE29450, GSE36668). In the diagram, the vertical axis indicates the odds that the TF element is "present" resp. "absent", which corresponds to the Notch cellular signaling pathway being active resp. passive, wherein values above the horizontal axis correspond to the TF element being more likely "present"/active and values below the horizontal axis indicate that the odds that the TF element is "absent"/passive are larger than the odds that it is "present"/active. The model was able to separate clearly the inactive from the active calibration samples.

Fig. 10 shows calibration results of the Bayesian network model based on the evidence curated list of target genes (26 target genes list) from Table 1 and the methods as described herein using publically available expression data sets of 11 normal ovary (group 1) and 20 high grade papillary serous ovarian carcinoma (group 2) samples (subset of samples taken from data sets GSE2109, GSE9891, GSE7307, GSE18520, GSE29450, GSE36668). In the diagram, the vertical axis indicates the odds that the TF element is "present" resp. "absent", which corresponds to the Notch cellular signaling pathway being active resp. passive, wherein values above the horizontal axis correspond to the TF element being more likely "present"/active and values below the horizontal axis indicate that the odds that the TF element is "absent"/passive are larger than the odds that it is "present"/active. Again, the model was able to separate clearly the inactive from the active calibration samples.

In the following, validation results of the trained exemplary Bayesian network models using the evidence curated list of target genes (26 target genes list) and the 18 target genes shortlist, respectively, are shown in Figs. 11 to 18.

Fig. 11 shows Notch cellular signaling pathway activity predictions of the trained exemplary Bayesian network model using the 18 target genes shortlist from Table 2 on three independent cultures of the MOLT4 cell line from data set GSE6495. In the diagram, the vertical axis indicates the odds that the TF element is "present" resp. "absent", which corresponds to the Notch cellular signaling pathway being active resp. passive, wherein values above the horizontal axis correspond to the TF element being more likely "present"/active and values below the horizontal axis indicate that the odds that the TF element is "absent"/passive are larger than the odds that it is "present"/active. The MOLT4 cell line is known to have high Notch signaling, which the model correctly predicted (group 1). Cells were treated for 48 hours with 5 $\mu$M DAPT, a gamma-secretase inhibitor (GSI) (group 2). GSIs are known to inhibit Notch signaling and the model correctly detected a decrease in Notch activity in this group (see Dohda T. et al., "Notch signaling induces SKP2 expression and promotes reduction of p27Kip1 in T-cell acute lymphoblastic leukemia cell", Experimental Cell Research, Vol. 313, No. 14, August 2007, pages 3141 to 3152).

Fig. 12 shows Notch cellular signaling pathway activity predictions of the trained exemplary Bayesian network model using the evidence curated list of target genes (26 target genes list) from Table 1 on three independent cultures of the MOLT4 cell line from data set GSE6495. In the diagram, the vertical axis indicates the odds that the TF element is "present" resp. "absent", which corresponds to the Notch cellular signaling pathway being active resp. passive, wherein values above the horizontal axis correspond to the TF element being more likely "present"/active and values below the horizontal axis indicate that the odds that the TF element is "absent"/passive are larger than the odds that it is "present"/active. The MOLT4 cell line is known to have high Notch signaling, which the model correctly predicted (group 1). Cells were treated for 48 hours with 5 μM DAPT, a gamma-secretase inhibitor (GSI) (group 2). GSIs are known to inhibit Notch signaling and the model correctly detected a decrease in Notch activity in this group (see Dohda T. et al., "Notch signaling induces SKP2 expression and promotes reduction of p27Kip1 in T-cell acute lymphoblastic leukemia cell", Experimental Cell Research, Vol. 313, No. 14, August 2007, pages 3141 to 3152).

Fig. 13 shows Notch cellular signaling pathway activity predictions of the trained exemplary Bayesian network model using the 18 target genes shortlist from Table 2 on IMR32 cells that were transfected with an inducible Notch3-intracellular construct. In the diagram, the vertical axis indicates the odds that the TF element is "present" resp. "absent", which corresponds to the Notch cellular signaling pathway being active resp. passive, wherein values above the horizontal axis correspond to the TF element being more likely "present"/active and values below the horizontal axis indicate that the odds that the TF element is "absent"/passive are larger than the odds that it is "present"/active. Two independent single-cell derived clones (c6, c8) are shown which drive Notch3-intracellular expression in the presence of 50 ng/mL doxycycline. At t=0 hr, for both clones the trained exemplary Bayesian network model using the 18 target genes shortlist from Table 2 detects low Notch activity. After induction of Notch3-intracellular, we correctly observe that Notch activity goes up in both clones and stabilizes at t = 24 hrs (data set GSE16477, van Nes J. et al., "A NOTCH3 Transcriptional Module Induces Cell Motility in Neuroblastoma", Clinical Cancer Research, Vol. 19, No. 13, July 2013, pages 3485 to 3494).

Fig. 14 shows Notch cellular signaling pathway activity predictions of the trained exemplary Bayesian network model using the 18 target genes shortlist from Table 2 on CD34+CD45RA-Lin-HPCs that were cultured for 72 hrs with graded doses of plastic-immobilized Notch ligand Delta1ext-IgG (data set GSE29524). In the diagram, the vertical axis indicates the odds that the TF element is "present" resp. "absent", which corresponds to the Notch cellular signaling pathway being active resp. passive, wherein values above the horizontal axis correspond to the TF element being more likely "present"/active and values below the horizontal axis indicate that the odds that the TF element is "absent"/passive are larger than the odds that it is "present"/active. The trained exemplary Bayesian network model using the 18 target genes shortlist from Table 2 correctly predicts higher Notch activity in the cells cultured on Delta1ext-IgG (group 2) compared to the control (group 1).

Fig. 15 shows Notch cellular signaling pathway activity predictions of the trained exemplary Bayesian network model using the 18 target genes shortlist from Table 2 on CUTLL1 cells, which are known to have high Notch activity. In the diagram, the vertical axis indicates the odds that the TF element is "present" resp. "absent", which corresponds to the Notch cellular signaling pathway being active resp. passive, wherein values above the horizontal axis correspond to the TF element being more likely "present"/active and values below the horizontal axis indicate that the odds that the TF element is "absent"/passive are larger than the odds that it is "present"/active. Treatment with a gamma-secretase inhibitor (GSI) inhibits Notch signaling. In data set GSE29544, it was observed that Notch activity is high 2 hours after a GSI washout. In this figure data from untreated CUTLL1 cells and CUTLL1 cells after GSI washout are pooled, since in both cases Notch activity is expected to be high. Six groups can be distinguished: 1) Untreated CUTLL1 cells and CUTLL1 cells after GSI washout. Here, the trained exemplary Bayesian network model using the 18 target genes shortlist correctly predicts high Notch activity in this group. 2) GSI treated CUTLL1 cells for which the model correctly predicts low Notch activity. 3+4) CUTLL1 cells treated with an empty MigRI retrovirus, which is not expected to affect Notch signaling. Here, the trained exemplary Bayesian network model using the 18 target genes shortlist from Table 2 correctly predicts high Notch activity for cells after GSI washout (group 3) and GSI treated cells (group 4). 5+6) CUTLL cells transduced with MigRI-dominant negative MAML1 virus. DNMAML1 is a Notch antagonist and Notch signaling is expected to be low in these cells. The model correctly predicts low Notch activity for both the cells after GSI washout (group 5) as for GSI treated cells (group 6) (see Wang H. et al., "Genome-wide analysis reveals conserved and divergent features of Notch1/RBPJ binding in human and murine T-lymphoblastic leukemia cells", Proceedings of the National Academy of Sciences of the USA, Vol. 108, No. 36, 2011, pages 14908 to 14913).

Fig. 16 shows Notch cellular signaling pathway activity predictions of the trained exemplary Bayesian network model using the evidence curated list of target genes (26 target gene list) from Table 1 on CUTLL1 cells, which are known to have high Notch activity. In the diagram, the vertical axis indicates the odds that the TF element is "present" resp. "absent", which corresponds to the Notch cellular signaling pathway being active resp. passive, wherein values above the horizontal axis correspond to the TF element being more likely "present"/active and values below the horizontal axis indicate that the odds that the TF element is "absent"/passive are larger than the odds that it is "present"/active.

Treatment with a gamma-secretase inhibitor (GSI) inhibits Notch signaling. In data set GSE29544, it was observed that Notch activity is high 2 hours after a GSI washout. In this figure data from untreated CUTLL1 cells and CUTLL1 cells after GSI washout are pooled, since in both cases Notch activity is expected to be high. Six groups can be distinguished: 1) Untreated CUTLL1 cells and CUTLL1 cells after GSI washout. Here, the trained exemplary Bayesian network model using the 18 target genes shortlist correctly predicts high Notch activity in this group. 2) GSI treated CUTLL1 cells for which the model correctly predicts low Notch activity. 3+4) CUTLL1 cells treated with an empty MigRI retrovirus, which is not expected to affect Notch signaling. Here, the trained exemplary Bayesian network model using the evidence curated list of target genes (26 target gene list) from Table 1 correctly predicts high Notch activity for cells after GSI washout (group 3) and GSI treated cells (group 4). 5+6) CUTLL cells transduced with MigRI-dominant negative MAML1 virus. DNMAML1 is a Notch antagonist and Notch signaling is expected to be low in these cells. The model correctly predicts low Notch activity for both the cells after GSI washout (group 5) as for GSI treated cells (group 6) (see Wang H. et al., "Genome-wide analysis reveals conserved and divergent features of Notch1/RBPJ binding in human and murine T-lymphoblastic leukemia cells", Proceedings of the National Academy of Sciences of the USA, Vol. 108, No. 36, 2011, pages 14908 to 14913).

Fig. 17 shows Notch cellular signaling pathway activity predictions of the trained exemplary Bayesian network model using the 18 target genes shortlist from Table 2 on HUVEC cells that were transfected with COUP-TFII siRNA (data set GSE33301). In the diagram, the vertical axis indicates the odds that the TF element is "present" resp. "absent", which corresponds to the Notch cellular signaling pathway being active resp. passive, wherein values above the horizontal axis correspond to the TF element being more likely "present"/active and values below the horizontal axis indicate that the odds that the TF element is "absent"/passive are larger than the odds that it is "present"/active. COUP-TFII is known to repress Notch signaling (see You L.R. et al., "Suppression of Notch signaling by the COUP-TFII transcription factor regulates vein identity", Vol. 435, No. 7038, May 2005, pages 98 to 104). The trained exemplary Bayesian network model using the 18 target genes shortlist from Table 2 correctly detects higher Notch activity in COUP-TFII siRNA transfected cells (group 2) compared to control cells (group 1) (see Chen X. et al., "COUP-TFII is a major regulator of cell cycle and Notch signaling pathways", Molecular Endocrinology, Vol. 26, No. 8, August 2012, pages 1268 to 1277).

Fig. 18 shows Notch cellular signaling pathway activity predictions of the trained exemplary Bayesian network model using the 18 target genes shortlist on breast cancer subgroups in samples from GSE6532, GSE9195, GSE12276, GSE20685, GSE21653 and EMTAB365. In the diagram, the vertical axis indicates the odds that the TF element is "present" resp. "absent", which corresponds to the Notch cellular signaling pathway being active resp. passive. It is observed that Notch activity is high in all breast cancer samples in those data sets. Results of doing a one-way ANOVA followed by a Games-Howell post-hoc test show that almost all groups have significant differences except for NormL vs. Basal and LumA vs. HER2, see Table 4. (subgroups: Basal, HER2, LumA = Luminal A, LumB = Luminal B, NormL = Normal-like)

Table 4: Results of Games-Howell post-hoc test comparing different subgroups of breast cancer samples as shown in Fig. 18. p-values $< 0.05$ are considered to be significant.

| Comparison | p adj |
|---|---|
| HER2-Basal | 2.2e-04 |
| LumA-Basal | 7.0e-08 |
| LumB-Basal | 9.2e-10 |
| NormL-Basal | 1 |
| LumA-HER2 | 1 |
| LumB-HER2 | 1.5e-03 |
| NormL-HER2 | 5.6e-03 |
| LumB-LumA | 1.5e-03 |
| NormL-LumA | 2.6e-04 |
| NormL-LumB | 3.2e-09 |

Table 5 shows results of Cox regression on Notch activity for the trained exemplary Bayesian network model using the 18 target genes shortlist on data sets as used in Fig. 18. For all samples together and more specifically for Luminal A end Luminal B there is a significantly worse prognosis with increasing Notch activity predicted by our model. This is supported by a recent publication in which it was found that patients testing positive for Notch1 had shorter disease-free survival (see Zhong Y. et al., "NOTCH1 is a Poor Prognostic Factor for Breast Cancer and Is Associated With Breast Cancer Stem Cells", Oncotargets and Therapy, Vol. 9, November 2016, pages 6865 to 6871).

Table 5: Results of Cox regression on Notch activity for the trained exemplary Bayesian network model using the 18 target genes shortlist from Table 2 on data sets as used in Fig. 18.

| | Cox's coef | HR | se(Cox's coef) | z | p |
|---|---|---|---|---|---|
| **All** | 0.0593 | 1.061093 | 0.015547 | 3.814204 | 0.000137 |
| **Basal** | -0.00439 | 0.995624 | 0.036854 | -0.11899 | 0.905283 |
| **HER2** | 0.085358 | 1.089107 | 0.04685 | 1.821967 | 0.06846 |
| **LumA** | 0.075129 | 1.078023 | 0.036091 | 2.081647 | 0.037375 |
| **LumB** | 0.076441 | 1.079439 | 0.024199 | 3.158812 | 0.001584 |
| **NormL** | 0.080338 | 1.083653 | 0.054621 | 1.470822 | 0.141339 |

Fig. 19 shows Notch cellular signaling pathway activity predictions of the trained exemplary Bayesian network model using the 12 target genes shortlist from Table 3 on CD34+CD45RA-Lin-HPCs that were cultured for 72 hrs with graded doses of plastic-immobilized Notch ligand Delta1ext-IgG (data set GSE29524). In the diagram, the vertical axis indicates the odds that the TF element is "present" resp. "absent", which corresponds to the Notch cellular signaling pathway being active resp. passive, wherein values above the horizontal axis correspond to the TF element being more likely "present"/active and values below the horizontal axis indicate that the odds that the TF element is "absent"/passive are larger than the odds that it is "present"/active. The trained exemplary Bayesian network model using the 12 target genes shortlist from Table 3 correctly predicts higher Notch activity in the cells cultured on Delta1ext-IgG (group 2) compared to the control (group 1).

Fig. 20 shows Notch cellular signaling pathway activity predictions of the trained exemplary Bayesian network model using the 12 target genes shortlist from Table 3 on CUTLL1 cells, which are known to have high Notch activity. In the diagram, the vertical axis indicates the odds that the TF element is "present" resp. "absent", which corresponds to the Notch cellular signaling pathway being active resp. passive, wherein values above the horizontal axis correspond to the TF element being more likely "present"/active and values below the horizontal axis indicate that the odds that the TF element is "absent"/passive are larger than the odds that it is "present"/active. Treatment with a gamma-secretase inhibitor (GSI) inhibits Notch signaling. In data set GSE29544, it was observed that Notch activity is high 2 hours after a GSI washout. In this figure data from untreated CUTLL1 cells and CUTLL1 cells after GSI washout are pooled, since in both cases Notch activity is expected to be high. Six groups can be distinguished: 1) Untreated CUTLL1 cells and CUTLL1 cells after GSI washout. Here, the trained exemplary Bayesian network model using the 18 target genes shortlist correctly predicts high Notch activity in this group. 2) GSI treated CUTLL1 cells for which the model correctly predicts low Notch activity. 3+4) CUTLL1 cells treated with an empty MigRI retrovirus, which is not expected to affect Notch signaling. Here, the trained exemplary Bayesian network model using the 12 target genes shortlist from Table 3 correctly predicts high Notch activity for cells after GSI washout (group 3) and GSI treated cells (group 4). 5+6) CUTLL cells transduced with MigRI-dominant negative MAML1 virus. DNMAML1 is a Notch antagonist and Notch signaling is expected to be low in these cells. The model correctly predicts low Notch activity for both the cells after GSI washout (group 5) as for GSI treated cells (group 6) (see Wang H. et al., "Genome-wide analysis reveals conserved and divergent features of Notch1/RBPJ binding in human and murine T-lymphoblastic leukemia cells", Proceedings of the National Academy of Sciences of the USA, Vol. 108, No. 36, 2011, pages 14908 to 14913).

Fig. 21 shows the correlation between the trained exemplary Bayesian network mode using the evidence curated list of target genes (26 target genes list) from Table 1 and the 12 target genes shortlist from Table 3, respectively. In the diagram, the horizontal axis indicates the odds (on a log2 scale) that the TF element is "present" resp. "absent", which corresponds to the Notch cellular signaling pathway being active resp. passive, as predicted by the trained exemplary Bayesian network model using the evidence curated list of target genes (26 target genes list) from Table 1. The vertical axis indicates the same information, as predicted by the trained exemplary Bayesian network model using the 12 target gene shortlist from Table 3 (data sets GSE5682, GSE5716, GSE6495, GSE9339, GSE14995, GSE15947, GSE16477, GSE16906, GSE18198, GSE20011, GSE20285, GSE20667, GSE24199, GSE27424, GSE29524, GSE29544, GSE29850, GSE29959, GSE32375, GSE33301, GSE33562, GSE34602, GSE35340, GSE36176, GSE37645, GSE39223, GSE42259, GSE46909, GSE49673, GSE53537, GSE54378, GSE57022, GSE61827, GSE74996, GSE81156, GSE82298). The two models are significantly correlated with a p-value of 2.2e-16 and a correlation coefficient of 0.929.

Figs. 22 and 23 show additional comparisons of Notch cellular signaling pathway activity predictions from a trained exemplary Bayesian network mode using (i) a list of 7 Notch target genes (DTX1, HES1, HES4, HES5, HEY2, MYC, and NRARP) and a list of 10 Notch target genes (the 7 Notch target genes plus EPHB3, SOX9, and NFKB2), and (ii) a list of 8 Notch target genes (DTX1, HES1, HES4, HES5, HEY2, MYC, NRARP, and PTCRA) and a list of 12 Notch target genes (the 8 Notch target genes plus HEYL, HEY1, PLXND1, and GFAP). The 7 Notch target genes are included in each of the lists of target genes from Tables 1 to 3 and the 8 Notch target genes include an additional target gene (PTCRA) that is only included in the evidence curated list of target genes (26 target genes list) from Table 1. The 3 additional target genes of the list of 10 Notch target genes were taken from the 12 target genes shortlist from Table 3 and the 4 additional target genes of the list of 12 Notch target genes, which differ from the 3 additional target genes, were taken from the evidence curated list of target genes (26 target genes list) from Table 1. The comparisons exemplarily show that the Notch cellular signaling pathway activity predictions from the trained exemplary Bayesian network mode using a list of 7 Notch target genes which is a subset of each of the lists of target genes from Tables 1 to 3, and a list of 8 Notch target genes, which is a subset of the evidence curated list of target genes (26 target genes list) from Table 1, can be further improved by adding additional target genes from the respective lists.

In detail:

Fig. 22 shows a comparison of the Notch cellular signaling pathway activity predictions using the list of 7 Notch target genes vs. the list of 10 Notch target genes. The models were run on samples from IMR32 cells that were transfected with an inducible Notch3-intracellular construct. In the diagram, the horizontal axis indicates time in hours and the vertical axis indicates the relative Notch cellular signaling pathway activity (on a log2odds scale). Both models correctly show the expected increase in Notch activity after induction of the Notch3-intracellular construct. The 10-target gene model (stippled line), however, shows a bigger increase in activity compared to the 7-target gene model (solid line). The Notch activity has been set to 0 at t=0 hours, to make comparison easier (data set GSE16477, see also van Nes J. et al., "A NOTCH3 Transcriptional Module Induces Cell Motility in Neuroblastoma", Clinical Cancer Research, Vol. 19, No. 13, July 2013, pages 3485 to 3494).

Fig. 23 shows a comparison of the Notch cellular signaling pathway activity predictions using the list of 8 Notch target genes vs. the list of 12 Notch target genes. The models were run on samples from endometrial stromal cells that were infected by a Jag1 retrovirus (data set GSE16906). Jag1 is a Notch ligand which induces cleavage of the Notch receptor upon binding, thereby ultimately inducing Notch target gene transcription. The 12-target gene model (right side of the graph) shows a better separation of the Notch activity (given on the vertical axis as log2odds) between control ("C" in the figure) and Jag1 infected cells ("Jag1 INF" in the figure) compared to the 8-target gene model (left side of the graph) (see also Mikhailik A. et al. "Notch ligand-dependent gene expression in human endometrial stromal cells", Biochemical and Biophysical Research Communications, Vol. 388, No. 3, October 2009, pages 479 to 482).

Instead of applying the calibrated mathematical model, e.g., the exemplary Bayesian network model, on mRNA input data coming from microarrays or RNA sequencing, it may be beneficial in clinical applications to develop dedicated assays to perform the sample measurements, for instance on an integrated platform using qPCR to determine mRNA levels of target genes. The RNA/DNA sequences of the disclosed target genes can then be used to determine which primers and probes to select on such a platform.

Validation of such a dedicated assay can be done by using the microarray-based mathematical model as a reference model, and verifying whether the developed assay gives similar results on a set of validation samples. Next to a dedicated assay, this can also be done to build and calibrate similar mathematical models using RNA sequencing data as input measurements.

The set of target genes which are found to best indicate specific cellular signaling pathway activity, e.g., Tables 1 to 3, based on microarray/RNA sequencing based investigation using the calibrated mathematical model, e.g., the exemplary Bayesian network model, can be translated into a multiplex quantitative PCR assay to be performed on a sample of the subject and/or a computer to interpret the expression measurements and/or to infer the activity of the Notch cellular signaling pathway. To develop such a test (e.g., FDA-approved or a CLIA waived test in a central service lab or a laboratory developed test for research use only) for cellular signaling pathway activity, development of a standardized test kit is required, which needs to be clinically validated in clinical trials to obtain regulatory approval.

The present invention relates to a computer-implemented method for inferring activity of a Notch cellular signaling pathway in a subject performed by a digital processing device, wherein the inferring is based on expression levels of five or more target genes of the Notch cellular signaling pathway measured in a sample of the subject. The present invention further relates to an apparatus for inferring activity of a Notch cellular signaling pathway in a subject comprising a digital processor configured to perform the method, to a non-transitory storage medium for inferring activity of a Notch cellular signaling pathway in a subject storing instructions that are executable by a digital processing device to perform the method, and to a computer program for inferring activity of a Notch cellular signaling pathway in a subject comprising program code means for causing a digital processing device to perform the method, when the computer program is run on the digital processing device.

The method may be used, for instance, in diagnosing an (abnormal) activity of the Notch cellular signaling pathway, in prognosis based on the inferred activity of the Notch cellular signaling pathway, in the enrollment of a subject in a clinical trial based on the inferred activity of the Notch cellular signaling pathway, in the selection of subsequent test(s) to be performed, in the selection of companion diagnostics tests, in clinical decision support systems, or the like. In this regard, reference is made to the published international patent application WO 2013/011479 A2 ("Assessment of cellular signaling pathway activity using probabilistic modeling of target gene expression"), to the published international patent application WO 2014/102668 A2 ("Assessment of cellular signaling pathway activity using linear combination(s) of target gene expressions"), and to Verhaegh W. et al., "Selection of personalized patient therapy through the use of knowledge-based computational models that identify tumor-driving signal transduction pathways", Cancer Research, Vol. 74, No. 11, 2014, pages 2936 to 2945, which describe these applications in more detail.

**Example 4: Additional results for mouse tissue**

Signal transduction pathways are often conserved across different species, having a similar function and similar direct target genes. The direct target genes are, however, not exactly the same, and the DNA/mRNA sequence of the gene is in general different between different species. Gene sequence similarity (homology) between species depends on the evolutionary distance between those species, e.g. the difference between mouse and human is smaller than the difference between human and lizard.

Because of these similarities between species, animal models are often used to study biological processes, like (organ/tissue) development, cell division and diseases. Mouse is a popular model organism because of its genetic proximity to humans. An example is the use of mouse models to study neurological disorders, like epilepsy and Alzheimer's. For such disorders it is invasive to obtain human tissue (contrary to cancer where often a biopsy of the tumour is taken anyway) and mouse models have been developed that mimic the disorder.

To be able to assess signal transduction pathway activity in mouse models is very useful, since it tells us something about the functional state of cells in the extracted tissue. In the case of a disease mouse model signal transduction pathway activity can give information on the human version of the disease, since these mouse models are usually generated to reflect the human disease in the best way possible.

The Notch cellular signaling pathway model was originally developed for human tissue, i.e. the selected target genes in Tables 1 to 3 are direct target genes in human, the input for the model is expression levels of human mRNA (e.g. from microarrays, qPCR, or RNAseq experiments), and calibration is done on expression data from human samples.

Herein, we also show a Notch cellular signaling pathway model for use in mouse. By selecting direct target genes of the Notch cellular signaling pathway in mouse and by using appropriate calibration samples (Affymetrix microarray data from a public database), a model was created which uses mouse mRNA expression levels as input and infers activity of the Notch cellular signaling pathway activity from this input. We then validated it using independent samples (Affymetrix microarray data from a public database) to show that it correctly measures the activity of the Notch cellular signalling pathway in mouse.

The selection of direct target genes for the mouse Notch cellular signaling pathway model was done in a similar manner as described before. The 26 gene list as used for the human Notch model was used as a starting point. This list was ranked on evidence score (which is calculated as described before) and a literature search was performed for the top ranking gene, using search keywords such as ("mouse" AND "direct target gene") and references from previously found literature for human direct target genes.

First it was confirmed that the gene actually exists in mouse and then it was confirmed that the gene was also a direct Notch target gene in mouse. This was done using similar evidence as used for the human target genes (i.e. the presence of transcription factor complex binding site, experimental evidence, like ChIP, luciferase assay, differential expression, GSI treatment, etc.). If multiple sources of evidence was found the gene was accepted as being a direct target gene for mouse Notch. In this manner a selection of 10 direct target genes was made for the Notch mouse model, as shown in Table 6.

Table 6: "10 target genes mouse list" of Notch target genes based on the evidence curated list of Notch target genes (from Affymetrix Mouse Genome 430 2.0 array).

| **Target gene** | **Probeset** |
|---|---|
| *Dtxl* | 1425822_a_at |
| | 1458643_at |
| *Hes1* | 1418102_at |

(continued)

| Target gene | Probeset |
|---|---|
| *Hes5* | 1456010_x_at |
| | 1423146_at |
| *Hes7* | 1422950_at |
| *Hey1* | 1415999_at |
| *Hey2* | 1418106_at |
| *Heym* | 1419302_at |
| | 1419303_at |
| | 1438886_at |
| *Myc* | 1424942_a_at |
| *Nrarp* | 1417985_at |
| | 1417986_at |
| *Sox9* | 1424950_at |
| | 1451538_at |

The Notch mouse model was calibrated on samples from dataset GSE15268, a publicly available dataset from the GEO (Gene Expression Omnibus) Database. This dataset contains Affymetrix microarray data from mouse embryonic stem cells with a NotchIC (Notch Intracellular Domain) inducible construct (induced by addition of hydrotamoxifen (OHT)). From this dataset 4 samples, where NotchIC was not induced, were used as Notch inactive samples (GSM381312, GSM381313, GSM381317, GSM381316) and 4 samples, where Notch was induced by adding OHT, were used as Notch active samples (GSM381324, GSM381325, GSM381320, GSM381321).

The calibrated Notch mouse model was then run on several datasets: the calibration set and several independent validation sets, to show that the model can successfully distinguish Notch active from Notch inactive samples. These results are shown in Figs. 24 to 27.

Fig. 24 shows calibration results of the Bayesian model based on the 10 target genes mouse list from Table 6 and the methods as described herein using publically available expression dataset GSE15268 containing 2 control Embryonic Stem Cells ("C ESc" in the figure), 2 control Mesodermal Progenitor Cells ("C MPc" in the figure), 2 ESc samples containing a tamoxifen inducible NERT construct (NotchIC), not OHT treated ("NERT ESc, no OHT" in the figure), 2 ESc samples containing a tamoxifen inducible NERT construct (NotchIC), OHT treated ("NERT ESc, OHT" in the figure), 4 MPc samples containing a tamoxifen inducible NERT construct (NotchIC), not OHT treated ("NERT MPc, no OHT" in the figure) and 4 MPc samples containing a tamoxifen inducible NERT construct (NotchIC), OHT treated ("NERT MPc, OHT" in the figure). The model was able to separate clearly the inactive (Control ESc and Control MPc) from the active (NERT MPc, OHT) calibration samples. The other samples in the data set were also correctly separated (see also Meier-Stiegen F. et al. "Activated Notch1 Target Genes during Embryonic Cell Differentiation Depend on the Cellular Context and Include Lineage Determinants and Inhibitors", PLoS One, Vol. 5, No. 7, July 2010).

Fig. 25 shows Notch cellular signaling pathway activity predictions of the trained exemplary Bayesian network model using the 10 target genes mouse list from Table 6 on mouse mammary glands with an inducible constitutively active Notch1 intracellular domain (NICD1) (data set GSE51628). For mammary gland samples where NICD1 is not induced ("M g" in the figure), the Notch mouse model (10 target genes) detects low Notch activity. As expected, mammary gland samples where NICD1 is induced using doxycycline correctly ("M g, NICD1 a" in the figure) show significantly higher Notch activity. Time points 48h and 96h have been combined in this figure (see also Abravanel D.L. et al. "Notch promotes recurrence of dormant tumor cells following HER2/neu-targeted therapy", Journal of Clinical Investigation, Vol. 125, No. 6, June 2015, pages 2484 to 2496).

Fig. 26 shows Notch cellular signaling pathway activity predictions of the trained exemplary Bayesian network model using the 10 target genes mouse list from Table 6 on mouse yolk sac tissue with an conditional transgenic system to activate Notch1 and mouse yolk sac tissue from transgenic mouse with RBPJ (part of the Notch transcription factor complex) loss-of-function (data set GSE22418). Both wild type samples ("W t" in the figure) and the RBPJ loss-of-function samples ("RBPJ 1-o-f" in the figure) show low Notch activity, and samples from yolk sac tissue where Notch1 is activated ("Notch1 a" in the figure) show elevated Notch activity, as expected (see also Copeland J.N. et al. "Notch signaling regulates remodeling and vessel diameter in the extraembryonic yolk sac", BMC Developmental Biology, February 2011).

Fig. 27 shows Notch cellular signaling pathway activity predictions of the trained exemplary Bayesian network model using the 10 target genes mouse list from Table 6 on mouse bone marrow cells (adult myeloerythroid progenitors) with a conditional gain of function allele of Notch2 receptor (data set GSE46724). The mouse Notch model (10 target genes) correctly calculates higher Notch activity for the ICN2 positive (IntraCellular Notch2) samples ("ICN2 p" in the figure), compared to the ICN2 negative samples ("ICN2 p" in the figure) (see also Oh P. et al. "In vivo mapping of notch pathway activity in normal and stress hematopoiesis", Cell Stem Cell, Vol. 13, No. 1, August 2013, pages 190 to 204).

**Example 5: Further information for illustrating the present invention**

(1) Measuring Levels of gene expression

Data derived from the unique set of target genes described herein is further utilized to infer an activity of the Notch cellular signaling pathway using the methods described herein.

Methods for analyzing gene expression levels in extracted samples are generally known. For example, methods such as Northern blotting, the use of PCR, nested PCR, quantitative real-time PCR (qPCR), RNA-seq, or microarrays can all be used to derive gene expression level data. All methods known in the art for analyzing gene expression of the target genes are contemplated herein.

Methods of determining the expression product of a gene using PCR based methods may be of particular use. In order to quantify the level of gene expression using PCR, the amount of each PCR product of interest is typically estimated using conventional quantitative real-time PCR (qPCR) to measure the accumulation of PCR products in real time after each cycle of amplification. This typically utilizes a detectible reporter such as an intercalating dye, minor groove binding dye, or fluorogenic probe whereby the application of light excites the reporter to fluoresce and the resulting fluorescence is typically detected using a CCD camera or photomultiplier detection system, such as that disclosed in U.S. Pat. No. 6,713,297 which is hereby incorporated by reference.

In some embodiments, the probes used in the detection of PCR products in the quantitative real-time PCR (qPCR) assay can include a fluorescent marker. Numerous fluorescent markers are commercially available. For example, Molecular Probes, Inc. (Eugene, Oreg.) sells a wide variety of fluorescent dyes. Non-limiting examples include Cy5, Cy3, TAMRA, R6G, R110, ROX, JOE, FAM, Texas Red™, and Oregon Green™. Additional fluorescent markers can include IDT ZEN Double-Quenched Probes with traditional 5' hydrolysis probes in qPCR assays. These probes can contain, for example, a 5' FAM dye with either a 3' TAMRA Quencher, a 3' Black Hole Quencher (BHQ, Biosearch Technologies), or an internal ZEN Quencher and 3' Iowa Black Fluorescent Quencher (IBFQ).

Fluorescent dyes useful according to the invention can be attached to oligonucleotide primers using methods well known in the art. For example, one common way to add a fluorescent label to an oligonucleotide is to react an N-Hydroxysuccinimide (NHS) ester of the dye with a reactive amino group on the target. Nucleotides can be modified to carry a reactive amino group by, for example, inclusion of an allyl amine group on the nucleobase. Labeling via allyl amine is described, for example, in U.S. Pat. Nos. 5,476,928 and 5,958,691, which are incorporated herein by reference. Other means of fluorescently labeling nucleotides, oligonucleotides and polynucleotides are well known to those of skill in the art.

Other fluorogenic approaches include the use of generic detection systems such as SYBR-green dye, which fluoresces when intercalated with the amplified DNA from any gene expression product as disclosed in U.S. Pat. Nos. 5,436,134 and 5,658,751.

Another useful method for determining target gene expression levels includes RNA-seq, a powerful analytical tool used for transcriptome analyses, including gene expression level difference between different physiological conditions, or changes that occur during development or over the course of disease progression.

Another approach to determine gene expression levels includes the use of microarrays for example RNA and DNA microarray, which are well known in the art. Microarrays can be used to quantify the expression of a large number of genes simultaneously.

(2) Generalized workflow for determining the activity of Notch cellular signaling

A flowchart exemplarily illustrating a process for inferring the activity of Notch cellular signaling from a sample isolated from a subject is shown in Fig. 3. First, the mRNA from a sample is isolated (11). Second, the mRNA expression levels of a unique set of at least five or more Notch target genes, as described herein, are measured (12) using methods for measuring gene expression that are known in the art. Next, an activity level of a Notch transcription factor (TF) element (13) is determined using a calibrated mathematical pathway model (14) relating the expression levels of the five or more Notch target genes to the activity level of the Notch TF element. Finally, the activity of the Notch cellular signaling pathway in the subject is inferred (15) based on the determined activity level of the Notch TF element in the sample of the subject. For example, the Notch cellular signaling pathway is determined to be active if the activity is above a certain threshold, and can be categorized as passive if the activity falls below a certain threshold.

(3) Calibrated mathematical pathway model

As contemplated herein, the expression levels of the unique set of five or more Notch target genes described herein are used to determine an activity level of a Notch TF element using a calibrated mathematical pathway model as further described herein. The calibrated mathematical pathway model relates the expression levels of the five or more Notch target genes to the activity level of the Notch TF element.

As contemplated herein, the calibrated mathematical pathway model is based on the application of a mathematical pathway model. For example, the calibrated mathematical pathway model can be based on a probabilistic model, for example, a Bayesian network model, or a linear or pseudo-linear model.

In an embodiment, the calibrated mathematical pathway model is a probabilistic model incorporating conditional probabilistic relationships relating the Notch TF element and the expression levels of the five or more Notch target genes. In an embodiment, the probabilistic model is a Bayesian network model.

In an alternative embodiment, the calibrated pathway mathematical model can be a linear or pseudo-linear model. In an embodiment, the linear or pseudo-linear model is a linear or pseudo-linear combination model as further described herein.

A flowchart exemplarily illustrating a process for generating a calibrated mathematical pathway model is shown in Fig. 4. As an initial step, the training data for the mRNA expression levels is collected and normalized. The data can be collected using, for example, microarray probeset intensities (101), real-time PCR Cq values (102), raw RNAseq reads (103), or alternative measurement modalities (104) known in the art. The raw expression level data can then be normalized for each method, respectively, by normalization using a normalization algorithm, for example, frozen robust multiarray analysis (fRMA) or MAS5.0 (111), normalization to average Cq of reference genes (112), normalization of reads into reads/fragments per kilobase of transcript per million mapped reads (RPKM/FPKM) (113), or normalization w.r.t. reference genes/proteins (114). This normalization procedure leads to a normalized probeset intensity (121), normalized Cq values (122), normalized RPKM/FPKM (123), or normalized measurement (124) for each method, respectively, which indicate target gene expression levels within the training samples.

Once the training data has been normalized, a training sample ID or IDs (131) is obtained and the training data of these specific samples is obtained from one of the methods for determining gene expression (132). The final gene expression results from the training sample are output as training data (133). All of the data from various training samples are incorporated to calibrate the model (including for example, thresholds, CPTs, for example in the case of the probabilistic or Bayesian network, weights, for example, in the case of the linear or pseudo-linear model, etc) (144). In addition, the pathway's target genes and measurement nodes (141) are used to generate the model structure for example, as described in Fig. 2 (142). The resulting model structure (143) of the pathway is then incorporated with the training data (133) to calibrate the model (144), wherein the gene expression levels of the target genes is indicative of the transcription factor element activity. As a result of the TF element determination in the training samples, a calibrated pathway model (145) is generated, which assigns the Notch cellular signaling pathway activity for a subsequently examined sample of interest, for example from a subject with a cancer, based on the target gene expression levels in the training samples.

(4) TF element determination

A flowchart exemplarily illustrating a process for determining an activity level of a TF element is shown in Fig. 5. The expression level data (test data) (163) from a sample extracted from a subject is input into the calibrated mathematical pathway model (145). The mathematical pathway model may be a probabilistic model, for example, a Bayesian network model, a linear model, or a pseudo-linear model.

The mathematical pathway model may be a probabilistic model, for example, a Bayesian network model, based on conditional probabilities relating the Notch TF element and expression levels of the five or more target genes of the Notch cellular signaling pathway measured in the sample of the subject, or the mathematical model may be based on one or more linear combination(s) of expression levels of the five or more target genes of the Notch cellular signaling pathway measured in the sample of the subject. In particular, the determining of the activity of the Notch cellular signaling pathway may be performed as disclosed in the published international patent application WO 2013/011479 A2 ("Assessment of cellular signaling pathway activity using probabilistic modeling of target gene expression"), the contents of which are herewith incorporated in their entirety. Briefly, the data is entered into a Bayesian network (BN) inference engine call (for example, a BNT toolbox) (154). This leads to a set of values for the calculated marginal BN probabilities of all the nodes in the BN (155). From these probabilities, the transcription factor (TF) node's probability (156) is determined and establishes the TF element's activity level (157).

Alternatively, the mathematical model may be a linear model. For example, a linear model can be used as described in the published international patent application WO 2014/102668 A2 ("Assessment of cellular signaling pathway activity using linear combination(s) of target gene expressions"), the contents of which are herewith incorporated in their entirety. Further details regarding the calculating/determining of cellular signaling pathway activity using mathematical modeling of target gene expression can also be found in Verhaegh W. et al., "Selection of personalized patient therapy through the use of knowledge-based computational models that identify tumor-driving signal transduction pathways", Cancer Research, Vol. 74, No. 11, 2014, pages 2936 to 2945. Briefly, the data is entered into a calculated weighted linear combination score (w/c) (151). This leads to a set of values for the calculated weighted linear combination score (152). From these weighted linear combination scores, the transcription factor (TF) node's weighted linear combination score (153) is determined and establishes the TF's element activity level (157).

(5) Procedure for discretized observables

A flowchart exemplarily illustrating a process for inferring activity of a Notch cellular signaling pathway in a subject as a discretized observable is shown in Fig. 6. First, the test sample is extracted and given a test sample ID (161). Next, the test data for the mRNA expression levels is collected and normalized (162). The test data can be collected using the same methods as discussed for the training samples in Fig. 5, using microarray probeset intensities (101), real-time PCR Cq values (102), raw RNAseq reads (103), or an alternative measurement modalities (104). The raw expression level data can then be normalized for each method, respectively, by normalization using an algorithm, for example fRMA or MAS5.0 (111), normalization to average Cq of reference genes (112), normalization of reads into RPKM/FPKM (113), and normalization w.r.t. reference genes/proteins (114). This normalization procedure leads to a normalized probeset intensity (121), normalized Cq values (122), normalized RPKM/FPKM (123), or normalized measurement (124) for each method, respectively.

Once the test data has been normalized, the resulting test data (163) is analyzed in a thresholding step (164) based on the calibrated mathematical pathway model (145), resulting in the thresholded test data (165). In using discrete observables, in one non-limiting example, every expression above a certain threshold is, for example, given a value of 1 and values below the threshold are given a value of 0, or in an alternative embodiment, the probability mass above the threshold as described herein is used as a thresholded value. Based on the calibrated mathematical pathway model, this value represents the TF element's activity level (157), which is then used to calculate the cellular signaling pathway's activity (171). The final output gives the cellular signaling pathway's activity (172) in the subject.

(6) Procedure for continuous observables

A flowchart exemplarily illustrating a process for inferring activity of a Notch cellular signaling pathway in a subject as a continuous observable is shown in Fig. 7. First, the test sample is extracted and given a test sample ID (161). Next, the test data for the mRNA expression levels is collected and normalized (162). The test data can be collected using the same methods as discussed for the training samples in Figure 5, using microarray probeset intensities (101), real-time PCR Cq values (102), raw RNAseq reads (103), or an alternative measurement modalities (104). The raw expression level data can then be normalized for each method, respectively, by normalization using an algorithm, for example fRMA (111), normalization to average Cq of reference genes (112), normalization of reads into RPKM/FPKM (113), and normalization w.r.t. reference genes/proteins (114). This normalization procedure leads to a normalized probeset intensity (121), normalized Cq values (122), normalized RPKM/FPKM (123), or normalized measurement (124) for each method, respectively.

Once the test data has been normalized, the resulting test data (163) is analyzed in the calibrated mathematical pathway model (145). In using continuous observables, as one non-limiting example, the expression levels are converted to values between 0 and 1 using a sigmoid function as described in further detail herein. The TF element determination as described herein is used to interpret the test data in combination with the calibrated mathematical pathway model, the resulting value represents the TF element's activity level (157), which is then used to calculate the cellular signaling pathway's activity (171). The final output gives the cellular signaling pathway's activity (172) in the subj ect.

(7) Target gene expression level determination procedure

A flowchart exemplary illustrating a process for deriving target gene expression levels from a sample extracted from a subject is shown in Fig. 8. In an exemplary embodiment, samples are received and registered in a laboratory. Samples can include, for example, Formalin-Fixed, Paraffin-Embedded (FFPE) samples (181) or fresh frozen (FF) samples (180). FF samples can be directly lysed (183). For FFPE samples, the paraffin can be removed with a heated incubation step upon addition of Proteinase K (182). Cells are then lysed (183), which destroys the cell and nuclear membranes which makes the nucleic acid (NA) available for further processing. The nucleic acid is bound to a solid phase (184) which could for example, be beads or a filter. The nucleic acid is then washed with washing buffers to remove all the cell debris which is present after lysis (185). The clean nucleic acid is then detached from the solid phase with an elution buffer (186). The DNA is removed by DNAse treatment to ensure that only RNA is present in the sample (187). The nucleic acid sample can then be directly used in the RT-qPCR sample mix (188). The RT-qPCR sample mixes contains the RNA sample, the RT enzyme to prepare cDNA from the RNA sample and a PCR enzyme to amplify the cDNA, a buffer solution to ensure functioning of the enzymes and can potentially contain molecular grade water to set a fixed volume of concentration. The sample mix can then be added to a multiwell plate (i.e., 96 well or 384 well plate) which contains dried RT-qPCR assays (189). The RT-qPCR can then be run in a PCR machine according to a specified protocol (190). An example PCR protocol includes i) 30 minutes at 50°C; ii) 5 minutes at 95°C; iii) 15 seconds at 95°C; iv) 45 seconds at 60°C; v) 50 cycles repeating steps iii and iv. The Cq values are then determined with the raw data by using the second derivative method (191). The Cq values are exported for analysis (192).

(8) Notch Mediated diseases and disorders and methods of treatment

As contemplated herein, the methods and apparatuses of the present invention can be utilized to assess Notch cellular signaling pathway activity in a subject, for example, a subject suspected of having, or having, a disease or disorder wherein the status of the Notch signaling pathway is probative, either wholly or partially, of disease presence or progression. In an embodiment, provided herein is a method of treating a subject comprising receiving information regarding the activity status of a Notch cellular signaling pathway derived from a sample extracted from the subject using the methods described herein and administering to the subject a Notch inhibitor if the information regarding the activity of the Notch cellular signaling pathway is indicative of an active Notch signaling pathway. In a particular embodiment, the Notch cellular signaling pathway activity indication is set at a cutoff value of odds of the Notch cellular signaling pathway being active of 10:1, 5:1, 4:1, 2:1, 1:1, 1:2, 1:4, 1:5, 1:10.

Notch inhibitors that may be used in the present invention are well known. Examples of Notch inhibitors include, but are not limited to, DAPT, PF-03084014, MK-0752, RO-4929097, LY450139, BMS-708163, LY3039478, IMR-1, Dibenzazepine, LY411575, FLI-06.

The Notch pathway plays a role in a large number of diseases, and notably in different types of neoplasms, e.g., carcinomas, sarcomas and hematological malignancies, immune-mediated diseases, degenerative diseases, inflammatory diseases, infectious diseases. These can be categorized according to the embryonic lineage-derived organ or tissue in which they mainly occur, for example, brain, breast, skin, esophagus, gastrointestinal tract, blood (hematological), ovarian, etc.

In a particular embodiment, the subject is suffering, or suspected to be suffering from, a breast cancer, lung cancer, a colon cancer, pancreatic cancer, brain cancer, hematological cancer, ovarian cancer. In a particular embodiment, the subject is suffering from, or suspected to be suffering from, a breast cancer.

In another particular embodiment, the subject is suffering from, or suspected to be suffering from, a brain cancer or more preferred a neuroblastoma cancer. In another particular embodiment, the subject is suffering form, or suspected to be suffering from, a hematological cancer or more preferred a T-cell lymphoblastic leukemia.

This application describes several preferred embodiments. Modifications and alterations may occur to others upon reading and understanding the preceding detailed description.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Calculations like the determination of the risk score performed by one or several units or devices can be performed by any other number of units or devices.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

**Example 5: Sequence Listings Used in Application**

SEQUENCE LISTING:

| Seq. No. | Gene: |
|---|---|
| Seq. 1 | CD28 |
| Seq. 2 | CD44 |
| Seq. 3 | DLGAP5 |

| | |
|---|---|
| Seq. 4 | DTX1 |
| Seq. 5 | EPHB3 |
| Seq. 6 | FABP7 |
| Seq. 7 | GFAP |
| Seq. 8 | GIMAP5 |
| Seq. 9 | HES1 |
| Seq. 10 | HES4 |
| Seq. 11 | HES5 |
| Seq. 12 | HES7 |
| Seq. 13 | HEY1 |
| Seq. 14 | HEY2 |
| Seq. 15 | HEYL |
| Seq. 16 | KLF5 |
| Seq. 17 | MYC |
| Seq. 18 | NFKB2 |
| Seq. 19 | NOX1 |
| Seq. 20 | NRARP |
| Seq. 21 | PBX1 |
| Seq. 22 | PIN1 |
| Seq. 23 | PLXND1 |
| Seq. 24 | PTCRA |
| Seq. 25 | SOX9 |
| Seq. 26 | TNC |

## Claims

1. A computer-implemented method for inferring activity of a Notch cellular signaling pathway in a subject performed by a digital processing device, wherein the inferring comprises:

    receiving expression levels of five or more target genes of the Notch cellular signaling pathway measured in a sample of the subject,
    determining an activity level of a Notch transcription factor (TF) element in the sample of the subject, the Notch TF element controlling transcription of the five or more Notch target genes, the determining being based on evaluating a calibrated mathematical pathway model relating the expression levels of the five or more Notch target genes to the activity level of the Notch TF element, and
    inferring the activity of the Notch cellular signaling pathway in the subject based on the determined activity level of the Notch TF element in the sample of the subject,
    wherein the five or more Notch target genes are selected from the group consisting of: DTX1, EPHB3, HES1, HES4, HES5, HEY2, MYC, NFKB2, NRARP, PIN1, PLXND1, and SOX9, wherein two or more Notch target genes are selected from the group consisting of: DTX1, HES1, HES4, HES5, HEY2, MYC, and NRARP, and three or more Notch target genes are selected from the group consisting of: EPHB3, NFKB2, PIN1, PLXND1, and SOX9,
    wherein the method is used in at least one of the following activities:

        diagnosis based on the inferred activity of the Notch cellular signaling pathway in the subject;
        prognosis based on the inferred activity of the Notch cellular signaling pathway in the subject; and
        wherein the subject is suffering from, or suspected to be suffering from, a brain cancer.

2. The method of claim 1, further comprising:
determining whether the Notch cellular signaling pathway is operating abnormally in the subject based on the inferred activity of the Notch cellular signaling pathway in the subject.

3. The method of claim 2, further comprising:

    recommending prescribing a drug for the subject that corrects for abnormal operation of the Notch cellular signaling pathway,
    wherein the recommending is performed if the Notch cellular signaling pathway is determined to be operating abnormally in the subject based on the inferred activity of the Notch cellular signaling pathway.

4. The method of claim 2 or 3, wherein the abnormal operation of the Notch cellular signaling pathway is an operation in which the Notch cellular signaling pathway operates as a tumor promoter in the subject.

5. The method of any of claims 1 to 4, wherein the calibrated mathematical pathway model is a probabilistic model, preferably a Bayesian network model, based on conditional probabilities relating the activity level of the Notch TF element and the expression levels of the five or more Notch target genes, or wherein the mathematical pathway model is based on one or more linear combination(s) of the expression levels of the three or more Notch target genes.

6. An apparatus for inferring activity of a Notch cellular signaling pathway in a subject comprising a digital processor configured to perform the method of any of claims 1 to 6.

7. A non-transitory storage medium for inferring activity of a Notch cellular signaling pathway in a subject storing instructions that are executable by a digital processing device to perform the method of any of claims 1 to 6.

8. A computer program for inferring activity of a Notch cellular signaling pathway in a subject comprising program code means for causing a digital processing device to perform the method of any of claims 1 to 6, when the computer program is run on the digital processing device.

9. A kit for measuring expression levels of five or more target genes of the Notch cellular signaling pathway in a sample of a subject, comprising:

polymerase chain reaction primers directed to the five or more Notch target genes,
probes directed to the five or more Notch target genes,
wherein the five or more Notch target genes are selected from the group consisting of: DTX1, EPHB3, HES1, HES4, HES5, HEY2, MYC, NFKB2, NRARP, PIN1, PLXND1, and SOX9, wherein two or more Notch target genes are selected from the group consisting of: DTX1, HES1, HES4, HES5, HEY2, MYC, and NRARP, and three or more Notch target genes are selected from the group consisting of: EPHB3, NFKB2, PIN1, PLXND1, and SOX9.

10. A kit for inferring activity of a Notch cellular signaling pathway in a subject, comprising:

one or more components for determining expression levels of five or more target genes of the Notch cellular signaling pathway in a sample of the subject, and
the apparatus of claim 6, the non-transitory storage medium of claim 7 or the computer program of claim 8,
wherein the one or more components are preferably selected from the group consisting of: a DNA array chip, an oligonucleotide array chip, a protein array chip, an antibody, a plurality of probes, for example, labeled probes, a set of RNA reverse-transcriptase sequencing components, and/or RNA or DNA, including cDNA, amplification primers,
wherein the five or more Notch target genes are selected from the group consisting of: DTX1, EPHB3, HES1, HES4, HES5, HEY2, MYC, NFKB2, NRARP, PIN1, PLXND1, and SOX9, wherein two or more Notch target genes are selected from the group consisting of: DTX1, HES1, HES4, HES5, HEY2, MYC, and NRARP, and three or more Notch target genes are selected from the group consisting of: EPHB3, NFKB2, PIN1, PLXND1, and SOX9.

11. Use of the kit of claim 9 or 10 in performing the method of any of claims 1 to 5.

**Patentansprüche**

1. Eine auf einem Computer umzusetzende Methode zum Ableiten der Aktivität des zellulären Notch-Signalwegs eines Patienten, die mit einem digitalen Verarbeitungsgerät durchgeführt wird, wobei das Ableiten folgende Schritte umfasst:

Abrufen der Expressionsniveaus von mindestens fünf in einer Patientenprobe gemessenen Zielgenen des zellulären Notch-Signalwegs,
Ermitteln des Aktivitätsniveaus eines Notch-Transkriptionsfaktor-(TF)-Elements in der Patientenprobe, wobei das Notch-TF-Element die Transkription der mindestens fünf Notch-Zielgene steuert, wobei das Ermitteln auf dem Auswerten eines kalibrierten mathematischen Pfadmodells beruht, das die Expressionsniveaus der mindestens fünf Notch-Zielgene mit dem Aktivitätsniveau des Notch-TF-Elements in Beziehung setzt, und
Ableiten der Aktivität des zellulären Notch-Signalwegs des Patienten anhand des ermittelten Aktivitätsniveaus des Notch-TF-Elements der Patientenprobe,
wobei die mindestens fünf Notch-Zielgene aus der folgenden Gruppe ausgewählt werden: DTX1, EPHB3, HES1, HES4, HES5, HEY2, MYC, NFKB2, NRARP, PIN1, PLXND1 und SOX9, wobei die mindestens zwei Notch-Zielgene aus der folgenden Gruppe ausgewählt werden: DTX1, HES1, HES4, HES5, HEY2, MYC und NRARP, und wobei die mindestens drei Notch-Zielgene aus der folgenden Gruppe ausgewählt werden: EPHB3, NFKB2, PIN1, PLXND1 und SOX9,
wobei die Methode für mindestens eine der folgenden Aktivitäten eingesetzt wird: Diagnose anhand der abgeleiteten Aktivität des zellulären Notch-Signalwegs des Patienten;
Prognose anhand der abgeleiteten Aktivität des zellulären Notch-Signalwegs des Patienten; und
wobei der Patient an einem Hirntumor leidet oder der Verdacht besteht, dass dieser an einem solchen leidet.

2. Die Methode gemäß Anspruch 1, die zudem folgenden Schritt umfasst:

anhand der abgeleiteten Aktivität des zellulären Notch-Signalwegs des Patienten Ermitteln, ob der zelluläre Notch-Signalweg des Patienten ungewöhnliche Aktivitäten aufweist.

**3.** Die Methode gemäß Anspruch 2, die zudem folgenden Schritt umfasst:

Empfehlen der Verschreibung eines Medikaments für den Patienten, das die ungewöhnliche Aktivität des zellulären Notch-Signalwegs korrigiert,
wobei die Empfehlung nur dann erfolgt, wenn anhand der abgeleiteten Aktivität des zellulären Notch-Signalwegs ermittelt wurde, dass der zelluläre Notch-Signalweg des Patienten ungewöhnliche Aktivitäten aufweist.

**4.** Die Methode gemäß Anspruch 2 oder 3, wobei sich die ungewöhnlichen Aktivitäten des zellulären Notch-Signalwegs dadurch auszeichnen, das es sich beim zellulären Notch-Signalweg des Patienten um einen Tumorpromotor handelt.

**5.** Die Methode gemäß einem der Ansprüche 1 bis 4, wobei es sich beim kalibrierten mathematischen Pfadmodell um ein probabilistisches Modell und vorzugsweise um ein Bayes-Netzwerkmodell handelt, das auf bedingten Wahrscheinlichkeiten beruht, die das Aktivitätsniveau des Notch-TF-Elements und die Expressionsniveaus der mindestens fünf Notch-Zielgene in Beziehung setzt, oder wobei das mathematische Pfadmodell auf mindestens einer linearen Kombination der Expressionsniveaus der mindestens drei Notch-Zielgene beruht.

**6.** Ein Gerät zum Ableiten der Aktivität eines zellulären Notch-Signalwegs eines Patienten, das einen digitalen Prozessor umfasst, der die Methode gemäß einem der Ansprüche 1 bis 6 durchführt.

**7.** Ein nicht flüchtiges Speichermedium zum Ableiten der Aktivität eines zellulären Notch-Signalwegs eines Patienten, auf dem Anweisungen gespeichert sind, die von einem digitalen Verarbeitungsgerät ausgeführt werden, um die Methode gemäß einem der Ansprüche 1 bis 6 durchzuführen.

**8.** Ein Computerprogramm zum Ableiten der Aktivität eines zellulären Notch-Signalwegs eines Patienten, das Programmcode umfasst, der ein digitales Verarbeitungsgerät veranlasst, die Methode gemäß einem der Ansprüche 1 bis 6 durchzuführen, wenn das Computerprogramm auf dem digitalen Verarbeitungsgerät ausgeführt wird.

**9.** Ein Kit zum Messen des Expressionsniveaus der mindestens fünf Zielgene des zellulären Notch-Signalwegs einer Patientenprobe, das Folgendes umfasst:

Polymerase-Kettenreaktionsprimere, die auf die mindestens fünf Notch-Zielgene ausgerichtet sind, Sonden, die auf die mindestens fünf Notch-Zielgene ausgerichtet sind,
wobei die mindestens fünf Notch-Zielgene aus der folgenden Gruppe ausgewählt werden: DTX1, EPHB3, HES1, HES4, HES5, HEY2, MYC, NFKB2, NRARP, PIN1, PLXND1 und SOX9, wobei die mindestens zwei Notch-Zielgene aus der folgenden Gruppe ausgewählt werden: DTX1, HES1, HES4, HES5, HEY2, MYC und NRARP, und wobei die mindestens drei Notch-Zielgene aus der folgenden Gruppe ausgewählt werden: EPHB3, NFKB2, PIN1, PLXND1 und SOX9.

**10.** Ein Kit zum Ableiten der Aktivität des zellulären Notch-Signalwegs eines Patienten, das Folgendes umfasst:

mindestens eine Komponente zum Ermitteln des Expressionsniveaus der mindestens fünf Zielgene des zellulären Notch-Signalwegs einer Patientenprobe, und
das Gerät gemäß Anspruch 6, das nicht flüchtige Speichermedium gemäß Anspruch 7 oder das Computerprogramm gemäß Anspruch 8,
wobei die mindestens eine Komponente bevorzugt aus der folgenden Gruppe ausgewählt wird: DNA-Array-Chip, Oligonukleotid-Array-Chip, Protein-Array-Chip, Antikörper, mehrere Sonden, beispielsweise markierten Sonden, Satz von Sequenzierungskomponenten für die reverse RNA-Transkriptase und/oder RNA- oder DNAeinschließlich cDNA-Amplifikationsprimere,
wobei die mindestens fünf Notch-Zielgene aus der folgenden Gruppe ausgewählt werden: DTX1, EPHB3, HES1, HES4, HES5, HEY2, MYC, NFKB2, NRARP, PIN1, PLXND1 und SOX9, wobei die mindestens zwei Notch-Zielgene aus der folgenden Gruppe ausgewählt werden: DTX1, HES1, HES4, HES5, HEY2, MYC und NRARP, und wobei die mindestens drei Notch-Zielgene aus der folgenden Gruppe ausgewählt werden: EPHB3, NFKB2, PIN1, PLXND1 und SOX9.

**11.** Verwenden des Kits gemäß Anspruch 9 oder 10 zum Durchführen der Methode gemäß einem der A

## Revendications

**1.** Procédé mis en œuvre par ordinateur permettant de déduire l'activité d'une voie de signalisation cellulaire Notch chez un sujet réalisé par un dispositif de traitement numérique, dans lequel la déduction comprend :

la réception des niveaux d'expression d'au moins cinq gènes cibles de la voie de signalisation cellulaire Notch mesurés dans un échantillon du sujet,
la détermination d'un niveau d'activité d'un élément de facteur de transcription Notch (TF) dans l'échantillon du sujet, l'élément Notch TF contrôlant la transcription d'au moins cinq gènes cibles Notch,
la détermination étant basée sur l'évaluation d'un modèle de voie mathématique calibré relatif aux niveaux d'expression d'au moins cinq gènes cibles Notch par rapport au niveau d'activité de l'élément Notch (TF), et
la déduction de l'activité de la voie de signalisation cellulaire Notch chez le sujet sur la base du niveau d'activité déterminé de l'élément Notch TF dans l'échantillon du sujet,
dans lequel au moins cinq gènes cibles Notch sont choisis parmi le groupe composé de : DTX1, EPHB3, HES1, HES4, HES5, HEY2, MYC, NFKB2, NRARP, PIN1,
PLXND1 et SOX9, dans lequel au moins deux gènes cibles Notch sont choisis parmi le groupe composé de : DTX1, HES1, HES4, HES5, HEY2, MYC et NRARP, et au moins trois gènes cibles Notch sont choisis parmi le groupe composé de : EPHB3, NFKB2, PIN1, PLXND1 et SOX9,
dans lequel le procédé est utilisé dans au moins une des activités suivantes : diagnostic basé sur l'activité déduite de la voie de signalisation cellulaire Notch chez le sujet :
pronostic basé sur l'activité déduite de la voie de signalisation cellulaire Notch chez le sujet ; et
dans lequel le sujet souffre, ou est suspecté de souffrir, d'un cancer du sein.

**2.** Procédé selon la revendication 1, consistant en outre à :
déterminer si la voie de signalisation cellulaire Notch fonctionne anormalement chez le sujet sur la base de l'activité déduite de la voie de signalisation cellulaire Notch chez le sujet.

**3.** Procédé selon la revendication 2, comprenant en outre :

la recommandation de prescription d'un médicament pour le sujet qui corrige le fonctionnement anormal de la voie de signalisation cellulaire Notch,
dans lequel la recommandation est réalisée si la voie de signalisation cellulaire Notch est déterminée comme fonctionnant anormalement chez le sujet sur la base de l'activité déduite de la voie de signalisation cellulaire Notch.

**4.** Procédé selon la revendication 2 ou 3, dans lequel le fonctionnement anormal de la voie de signalisation cellulaire Notch est un fonctionnement dans lequel la voie de signalisation cellulaire Notch fonctionne comme un promoteur de tumeur chez le sujet.

**5.** Procédé selon l'une quelconque des revendications 1 à 4, dans lequel le modèle de voie mathématique étalonné est un modèle probabiliste, de préférence un modèle de réseau Bayésien, basé sur les probabilités conditionnelles relatives au niveau d'activité de l'élément Notch TF et les niveaux d'expression d'au moins cinq gènes cibles Notch, ou dans lequel le modèle de voie mathématique est basé sur au moins une combinaison linéaire des niveaux d'expression d'au moins trois gènes cibles Notch.

**6.** Appareil pour déduire l'activité d'une voie de signalisation cellulaire Notch chez un sujet comprenant un processeur numérique configuré pour réaliser les procédés selon l'une quelconque des revendications 1 à 6.

**7.** Support de stockage non transitoire pour déduire l'activité d'une voie de signalisation cellulaire Notch chez un sujet stockant des instructions qui sont exécutables par un dispositif de traitement numérique pour réaliser le procédé selon l'une quelconque des revendications 1 à 6.

**8.** Programme informatique pour déduire l'activité d'une voie de signalisation cellulaire Notch chez un sujet comprenant des moyens de code de programmation pour amener un dispositif de traitement numérique à réaliser le procédé selon l'une quelconque des revendications 1 à 6, lorsque le programme informatique tourne sur le dispositif de traitement numérique.

**9.** Kit permettant de mesurer les niveaux d'expression d'au moins cinq gènes cibles de la voie de signalisation cellulaire Notch dans un échantillon d'un sujet, comprenant :

des amorces de réaction en chaîne de polymérase dirigées vers au moins cinq gènes cibles Notch,
des sondes dirigées vers au moins cinq gènes cibles Notch,
dans lequel au moins cinq gènes cibles Notch sont choisis parmi le groupe composé de : DTX1, EPHB3, HES1, HES4, HES5, HEY2, MYC, NFKB2, NRARP, PIN1, PLXND1 et SOX9, dans lequel au moins deux gènes cibles Notch sont choisis parmi le groupe composé de : DTX1, HES1, HES4, HES5, HEY2, MYC, et NRARP, et au moins trois gènes cibles Notch sont choisis parmi le groupe composé de : EPHB3, NFKB2, PIN1, PLXND1 et SOX9.

**10.** Kit permettant de déduire l'activité d'une voie de signalisation cellulaire Notch chez un sujet comprenant :

un ou plusieurs éléments permettant de déterminer les niveaux d'expression d'au moins cinq gènes cibles de la voie de signalisation cellulaire Notch dans un échantillon du sujet
et l'appareil de la revendication 6, le support de stockage non transitoire de la revendication 7 ou le programme informatique de la revendication 8,
dans lequel un ou plusieurs éléments sont de préférence choisis parmi le groupe composé : d'une puce de réseau ADN, d'une puce de réseau d'oligonucléotides, d'une puce de réseau de protéines, d'un anticorps, d'une pluralité de sondes, par exemple, les sondes marquées, un ensemble d'éléments de séquençage de transcriptase inverse d'ARN, et/ou d'ARN ou d'ADN, y compris d'ADNc, les amorces d'amplification,
dans lequel au moins cinq gènes cibles Notch sont choisis parmi le groupe composé de : DTX1, EPHB3, HES1, HES4, HES5, HEY2, MYC, NFKB2, NRARP, PIN1, PLXND1 et SOX9, dans lequel au moins deux gènes cibles Notch sont choisis parmi le groupe composé de : DTX1, HES1, HES4, HES5, HEY2, MYC, et NRARP et au moins trois gènes cibles Notch sont choisis parmi le groupe composé de : EPHB3, NFKB2, PIN1, PLXND1 et SOX9.

**11.** Utilisation du kit de la revendication 9 ou 10 dans l'exécution du procédé de l'une quelconque des revendications 1 à 5.

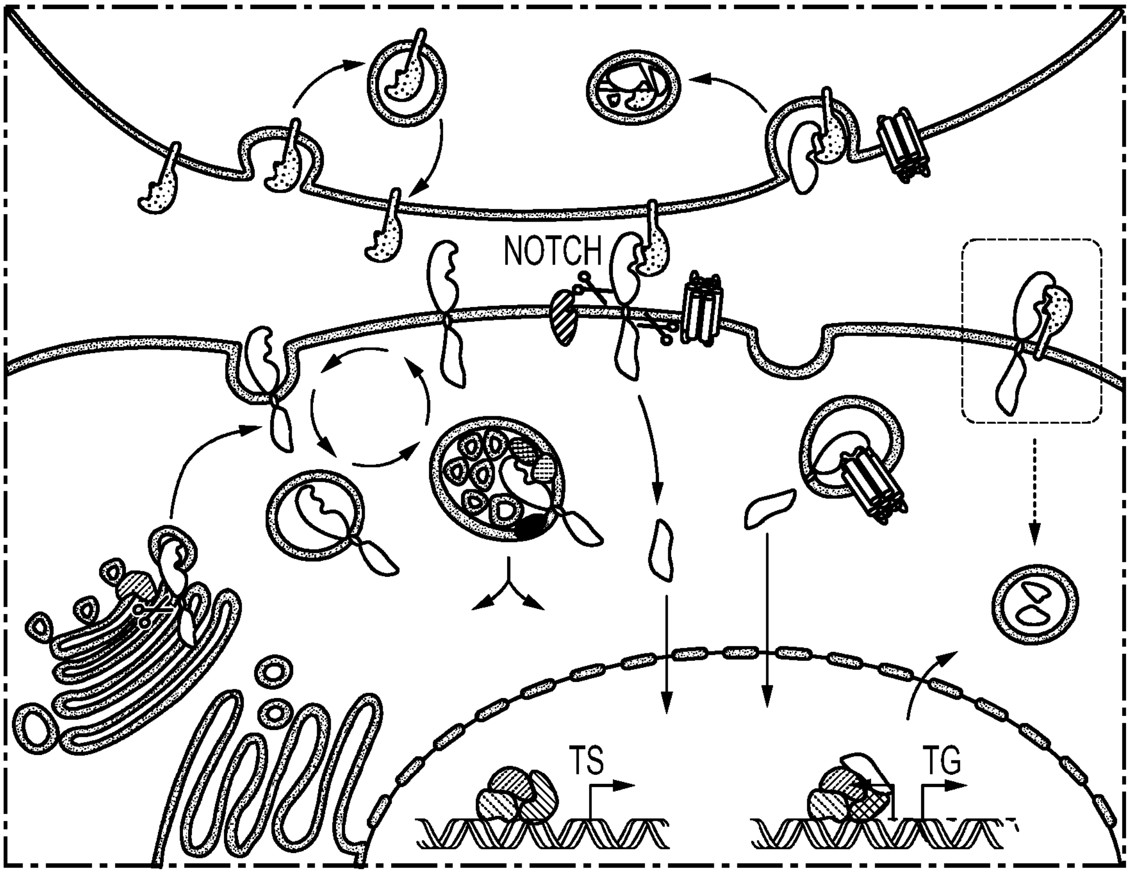


FIG. 1

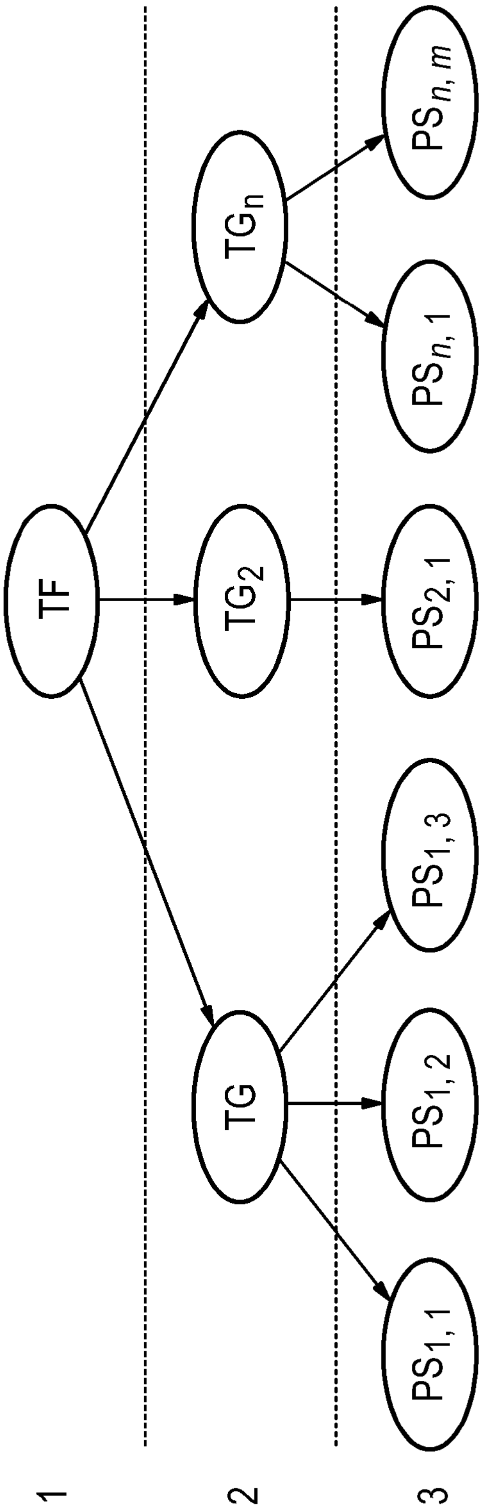


FIG. 2

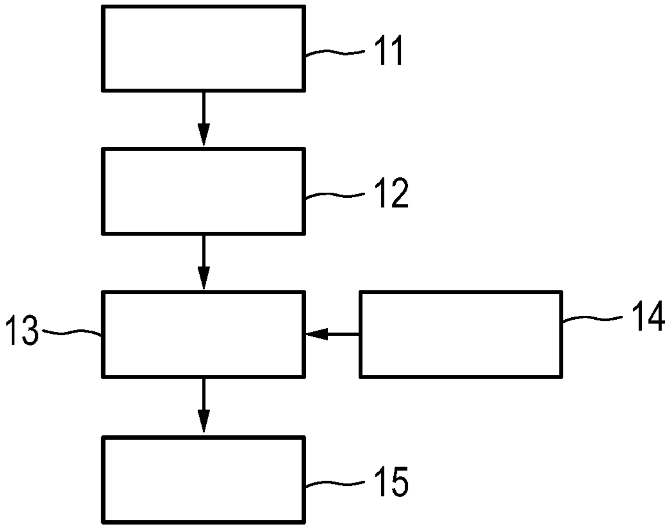


FIG. 3

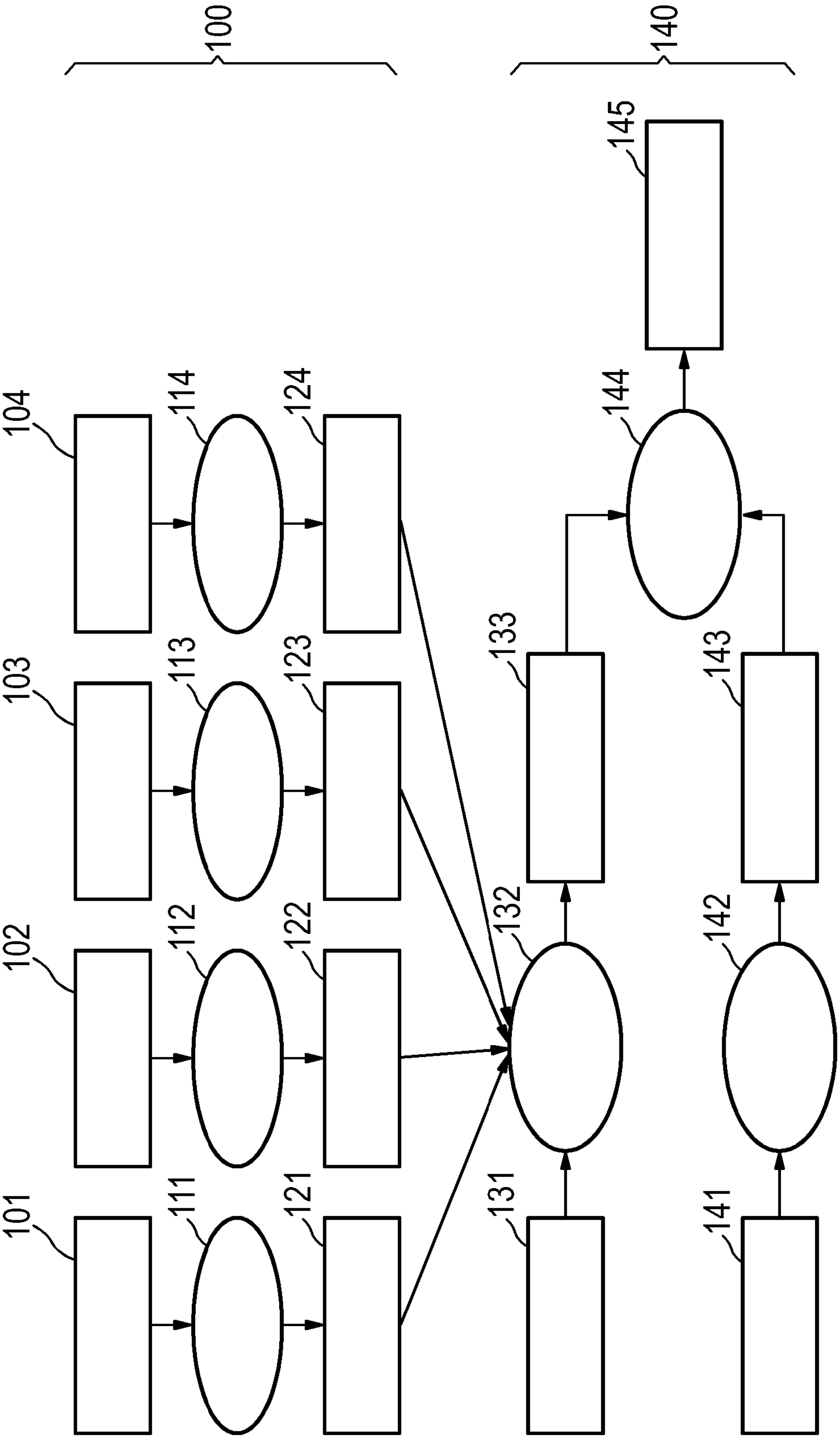
100
140
101
102
103
104
111
112
113
114
121
122
123
124
131
132
133
141
142
143
144
145

FIG. 4

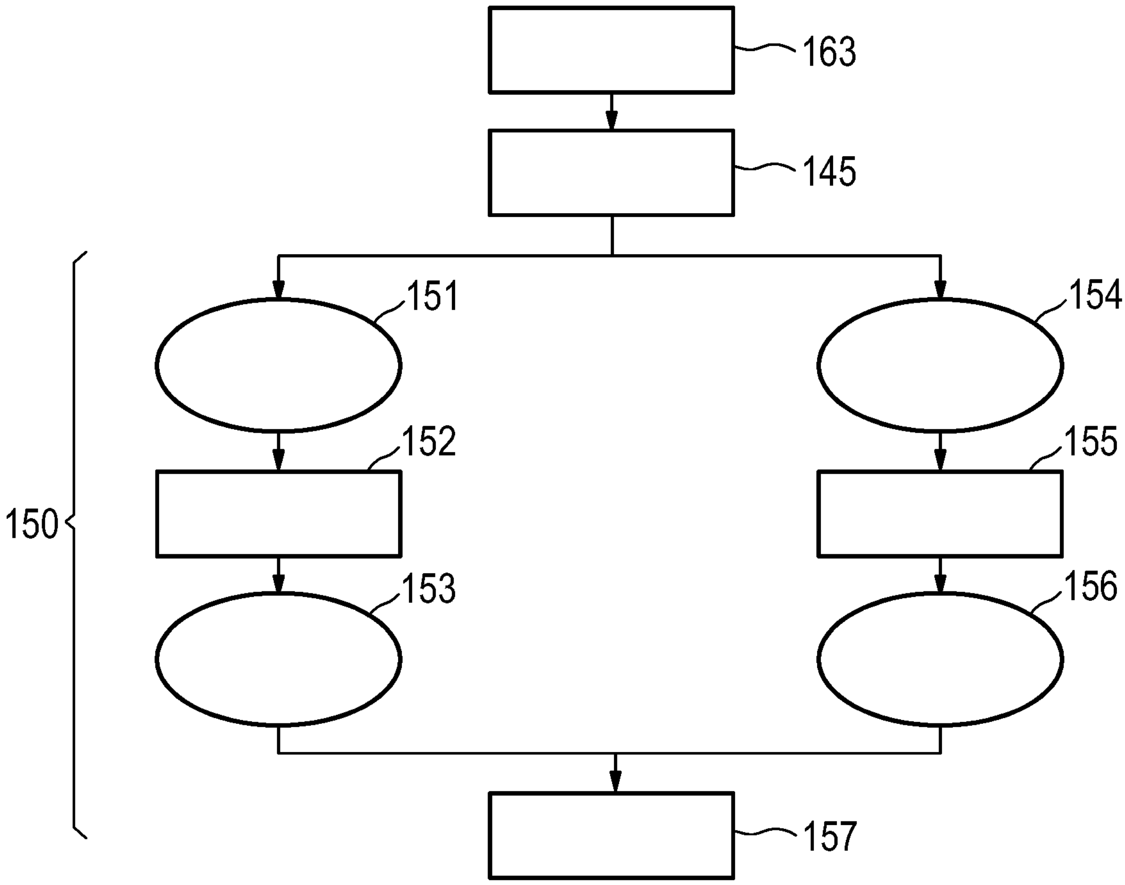


FIG. 5

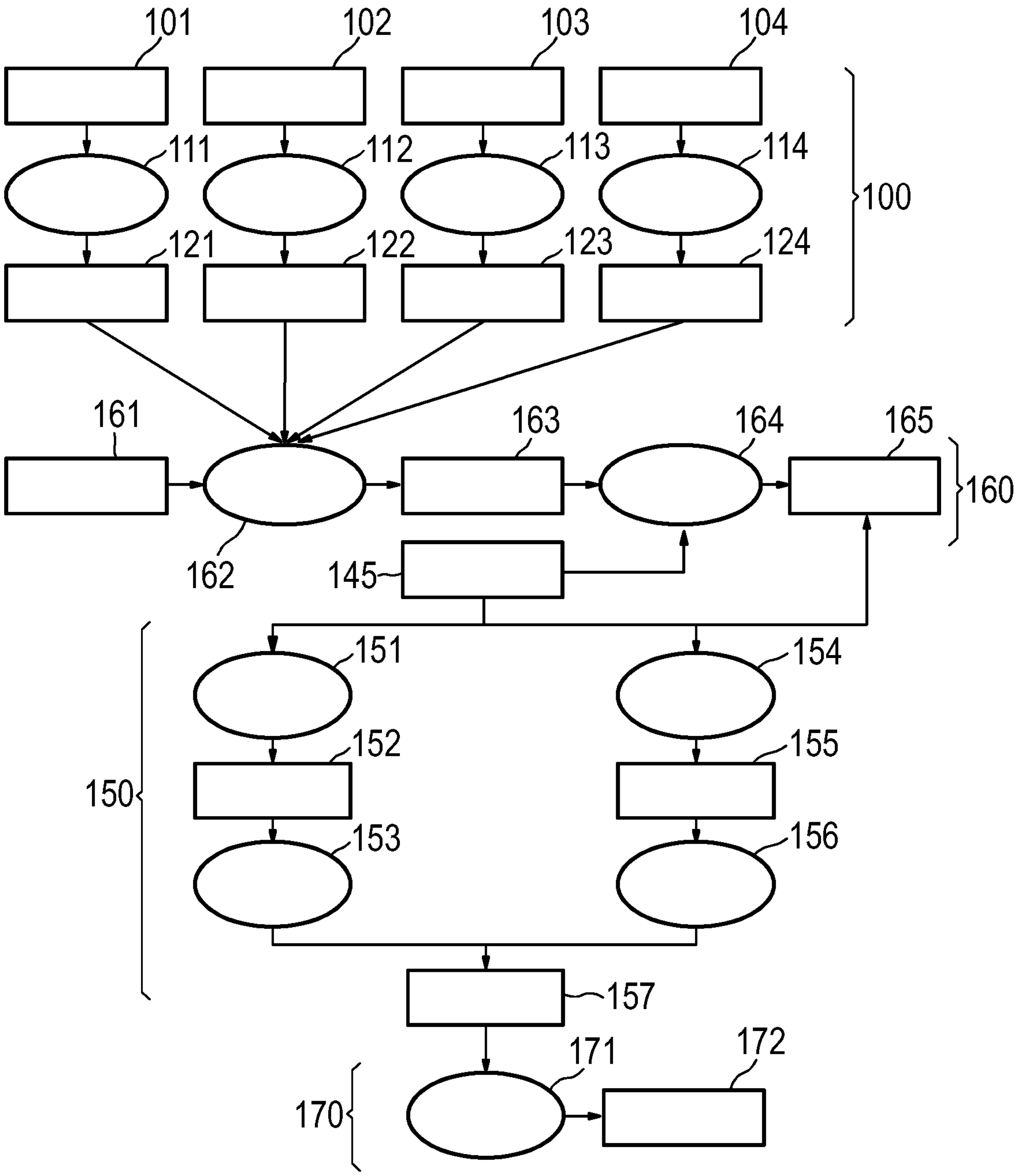
101
102
103
104
111
112
113
114
100
121
122
123
124
161
163
164
165
160
162
145
151
154
152
155
150
153
156
157
171
172
170

FIG. 6

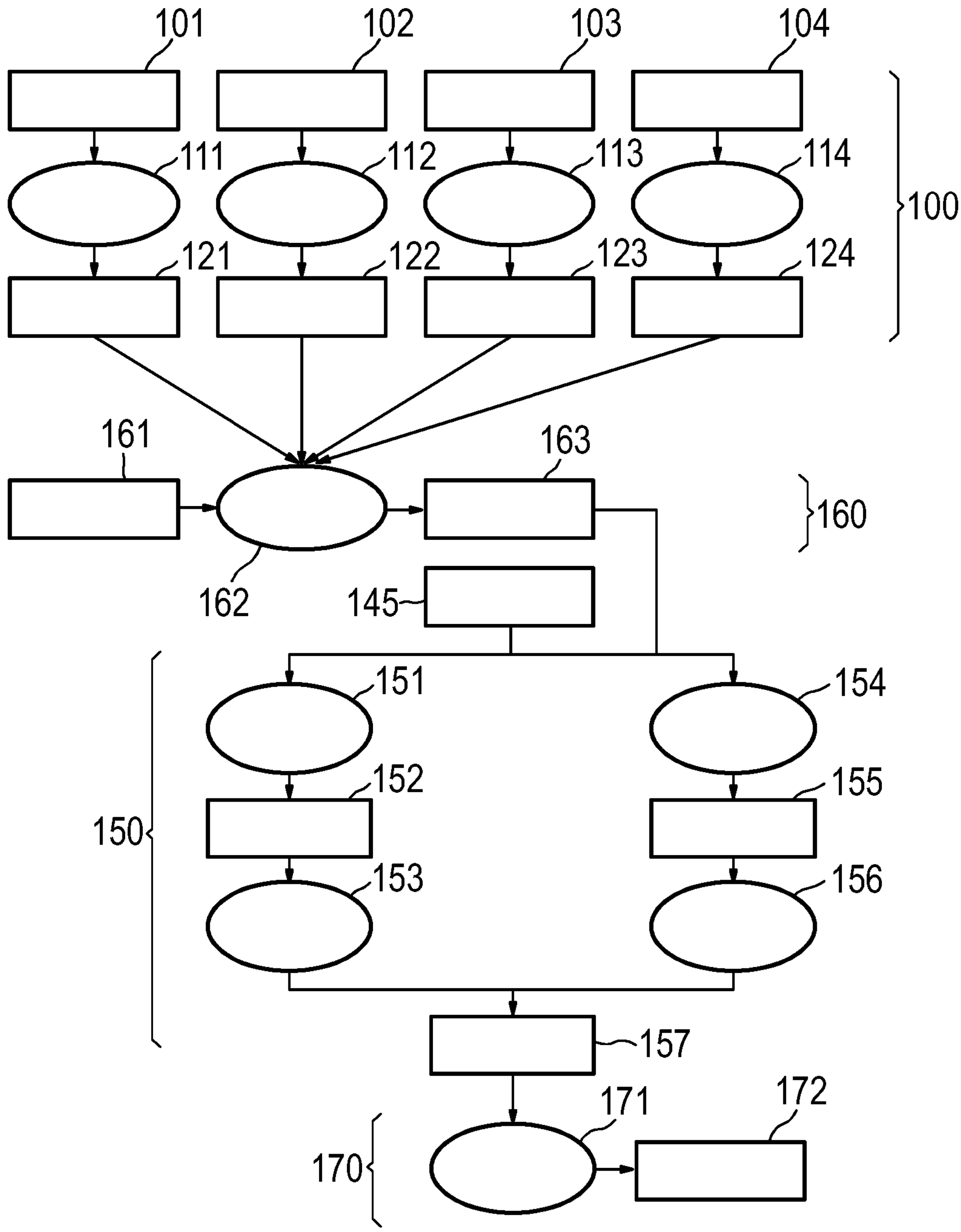
101
102
103
104
111
112
113
114
100
121
122
123
124
161
163
160
162
145
151
154
152
155
150
153
156
157
171
172
170

FIG. 7

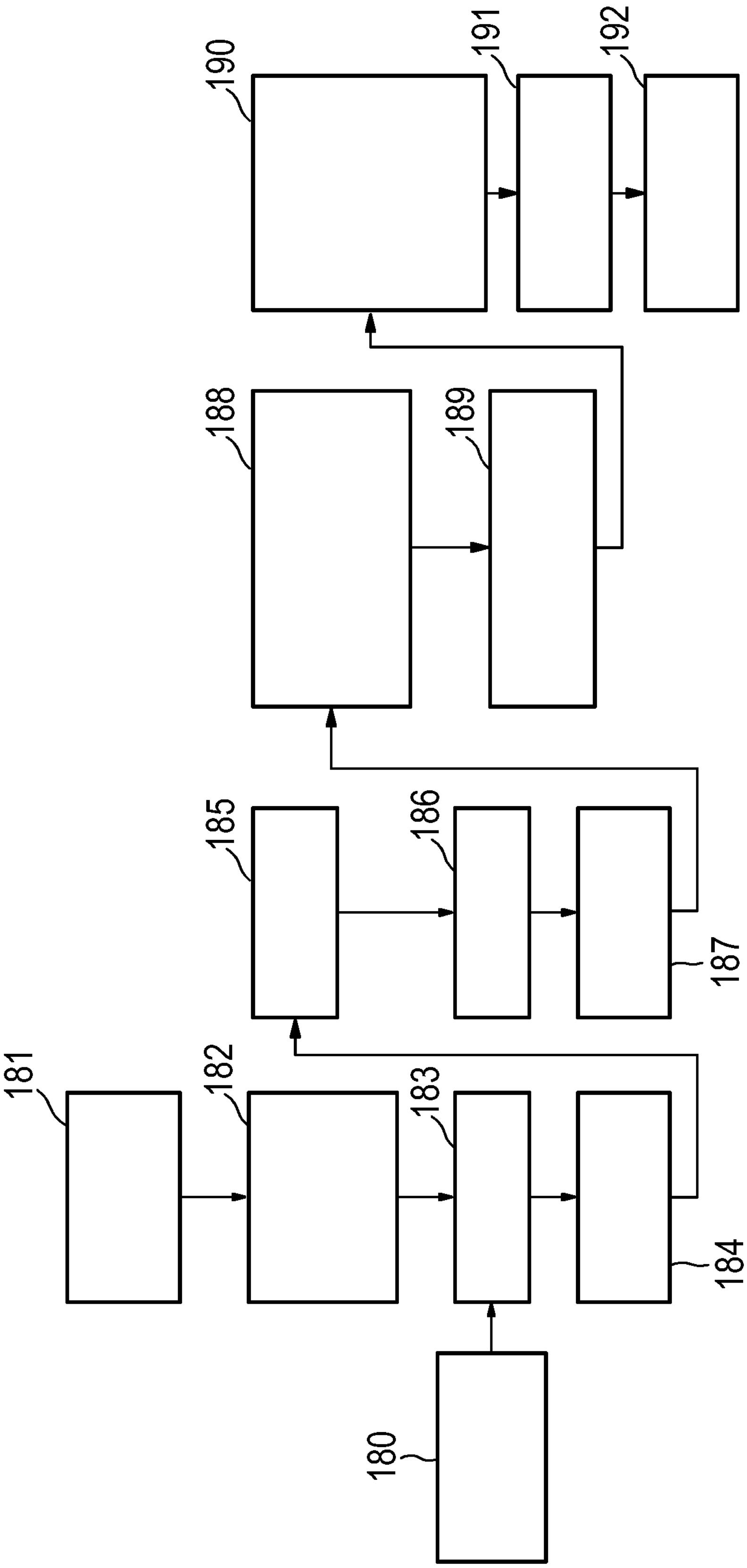


FIG. 8

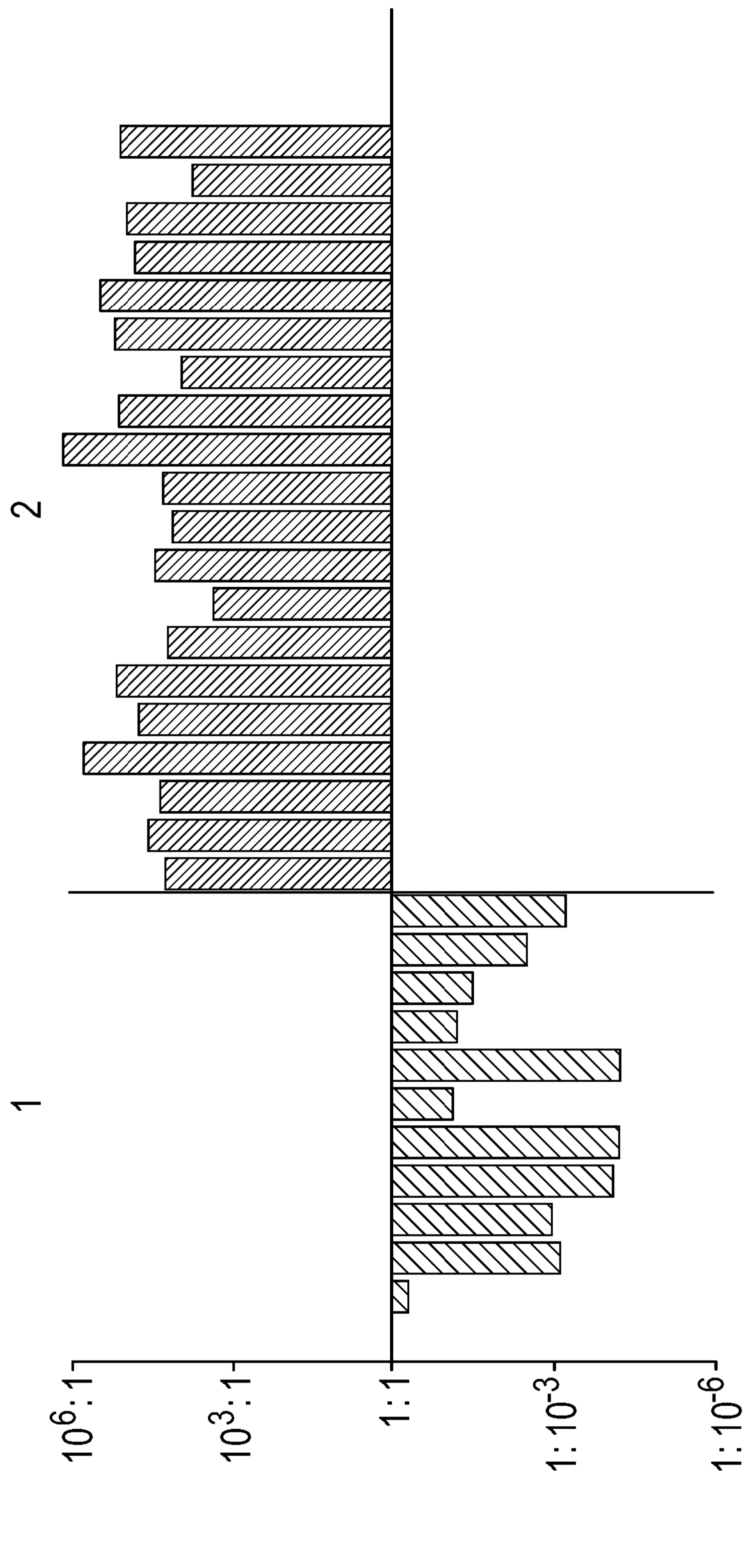


FIG. 9

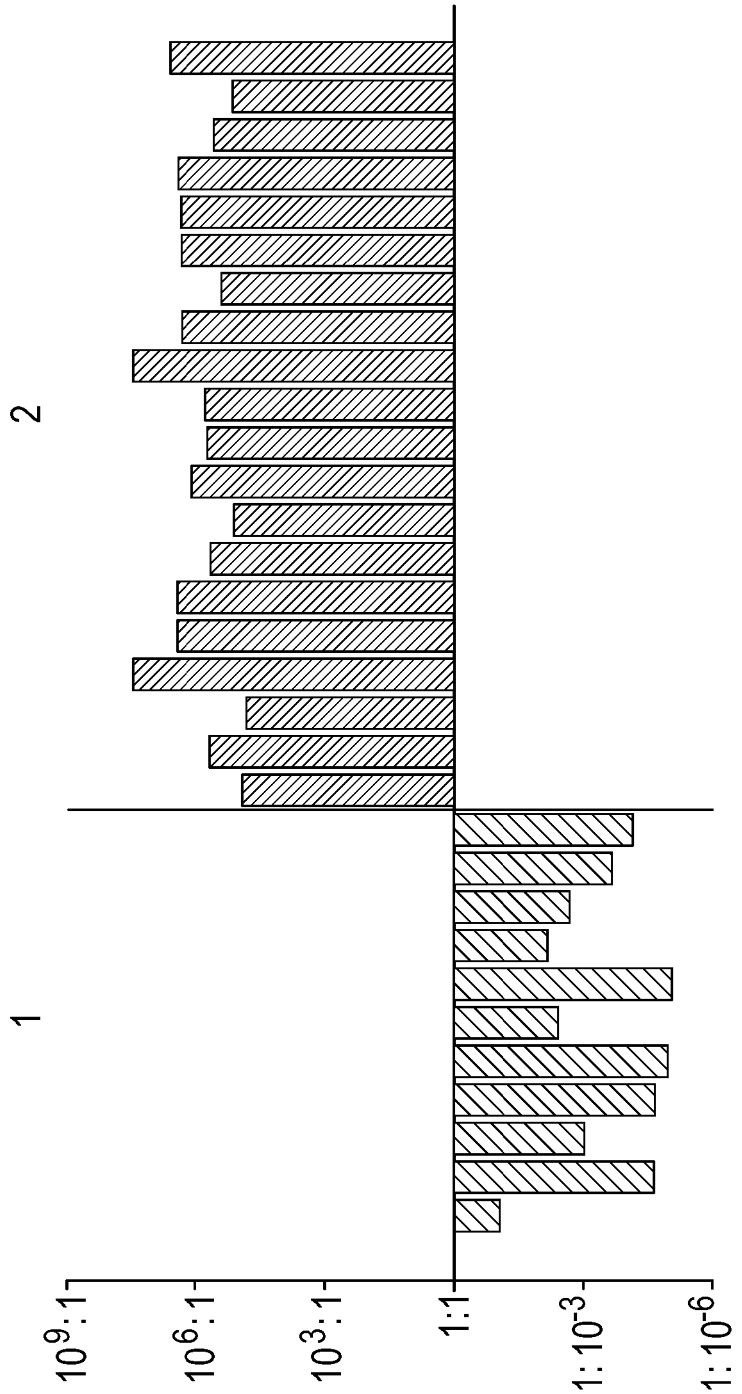


FIG. 10

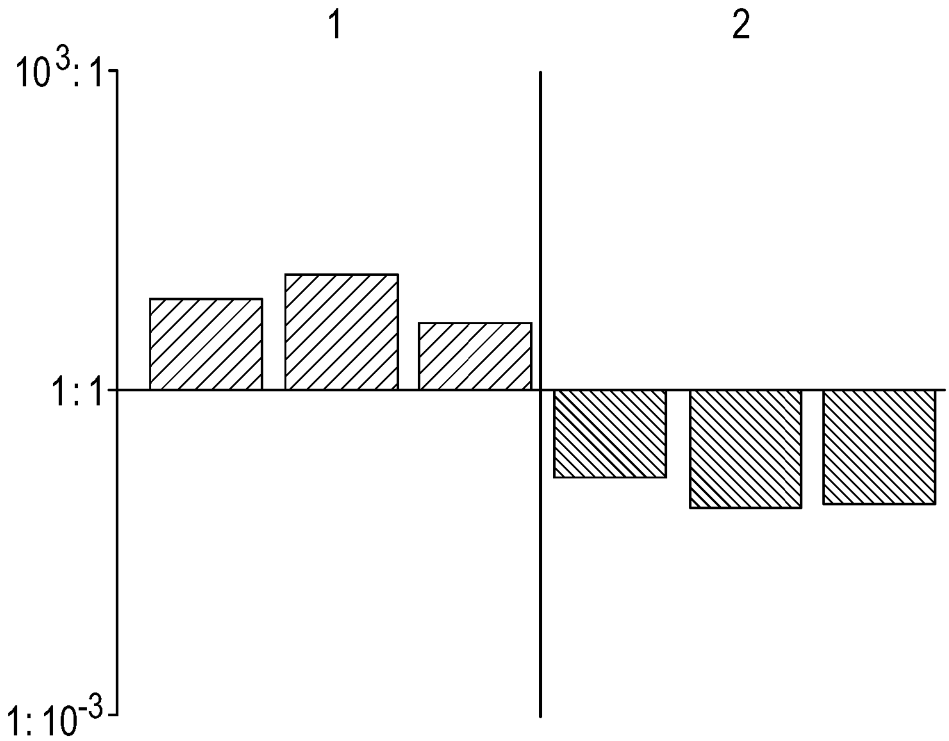


FIG. 11

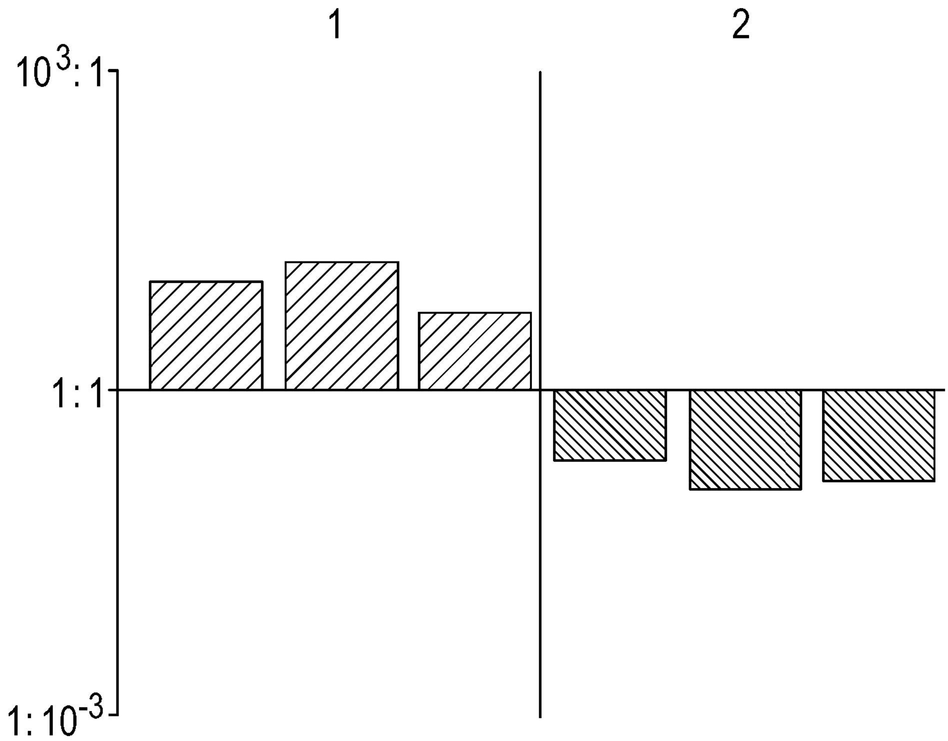


FIG. 12

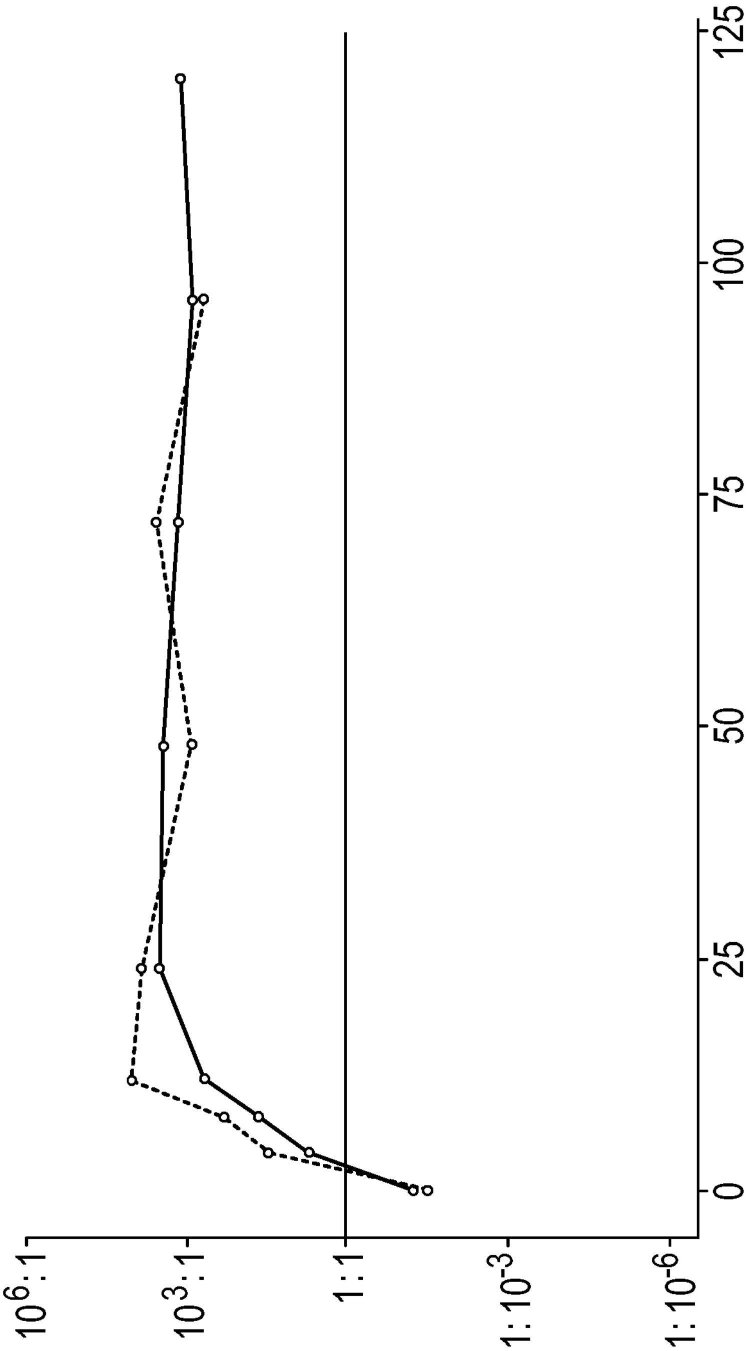


FIG. 13

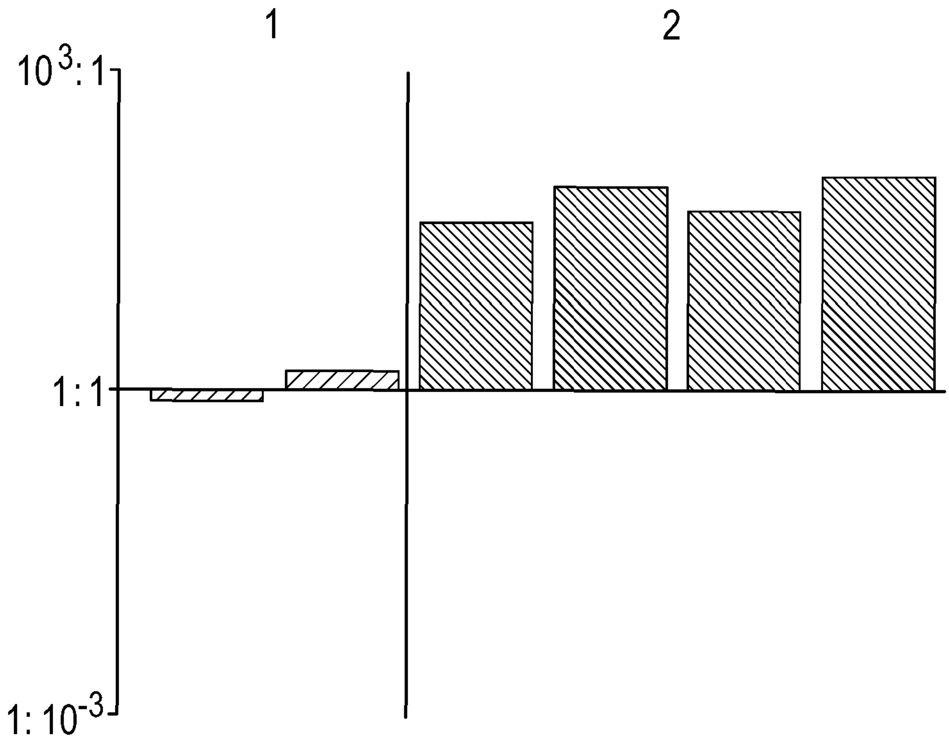


FIG. 14

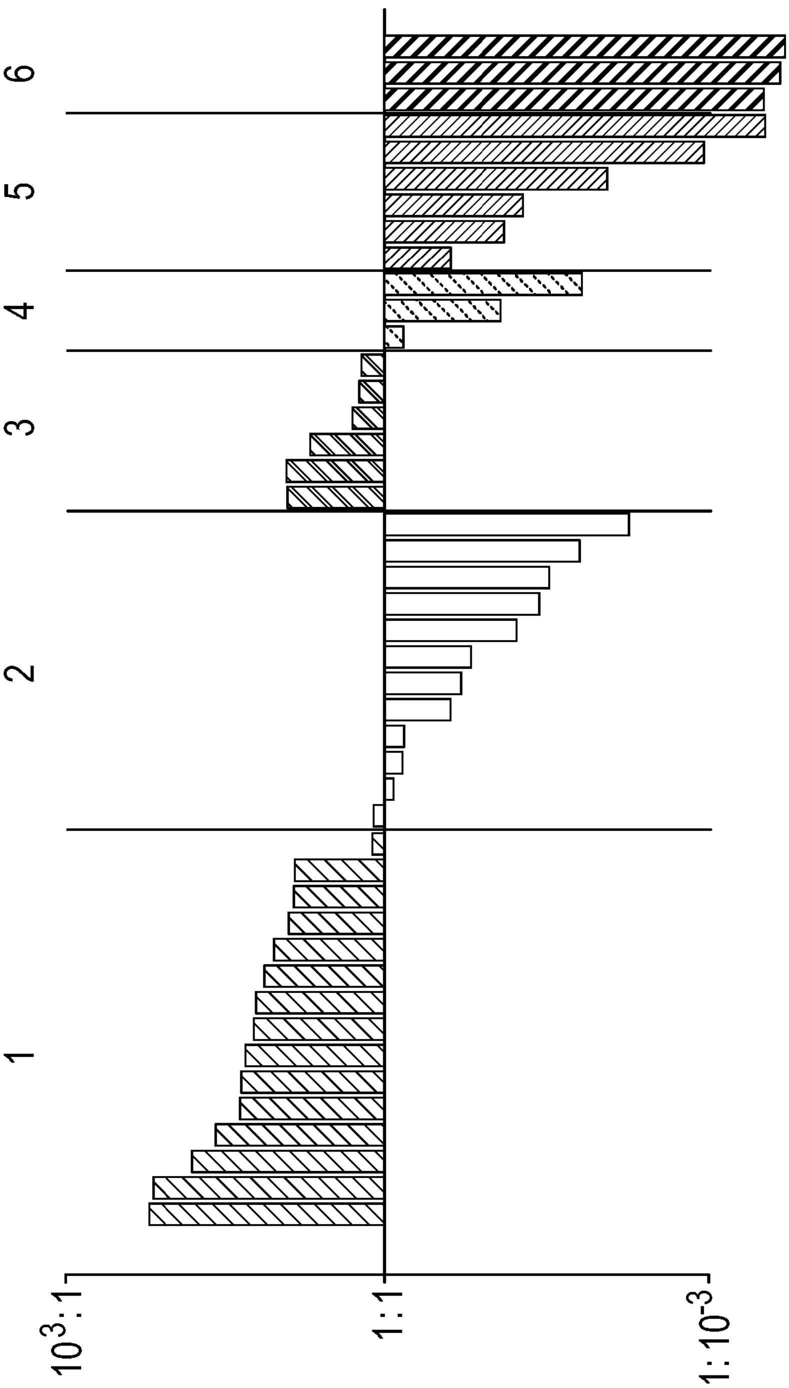


FIG. 15

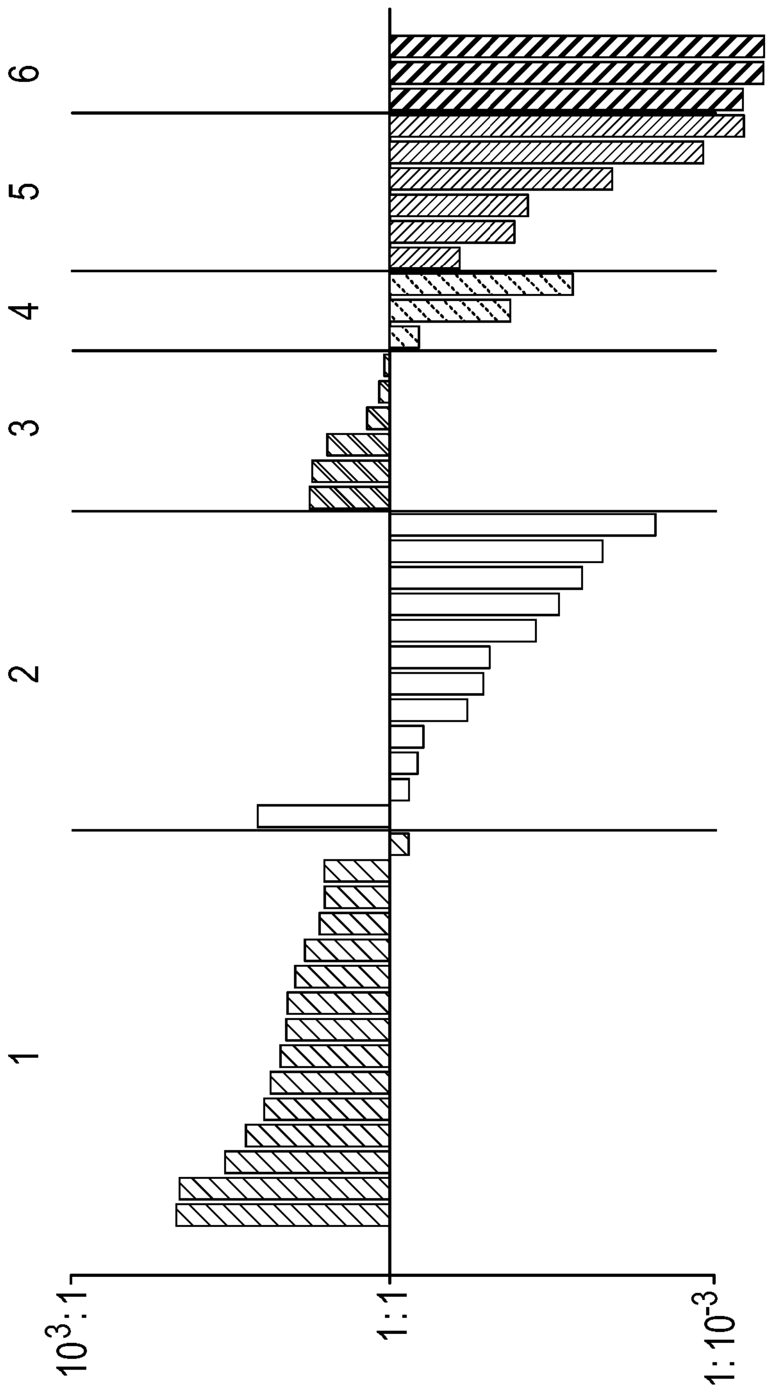


FIG. 16

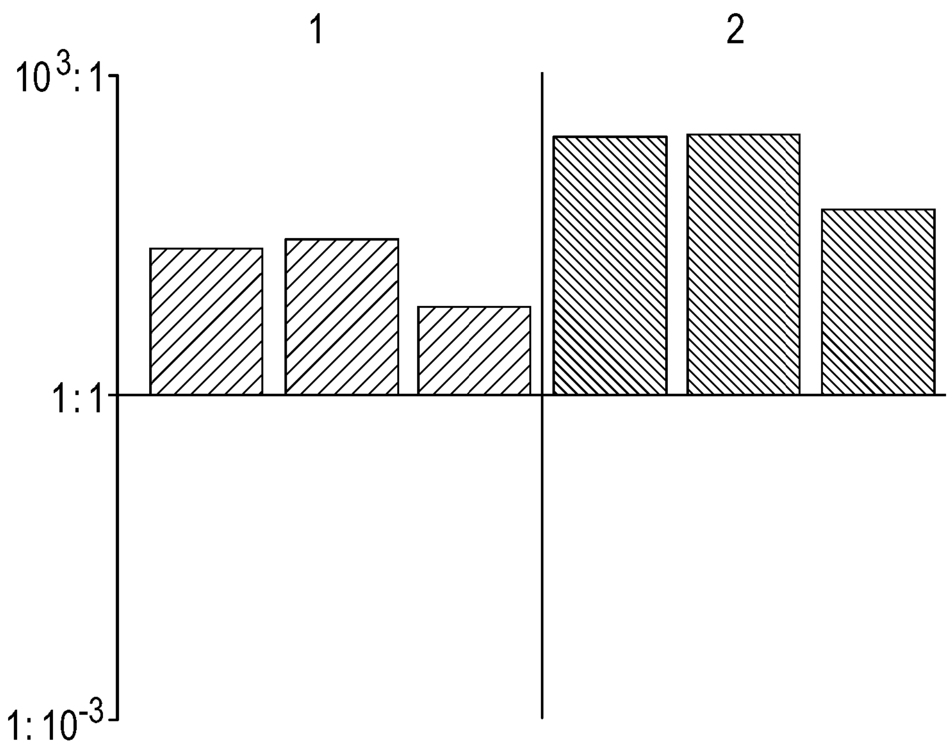


FIG. 17

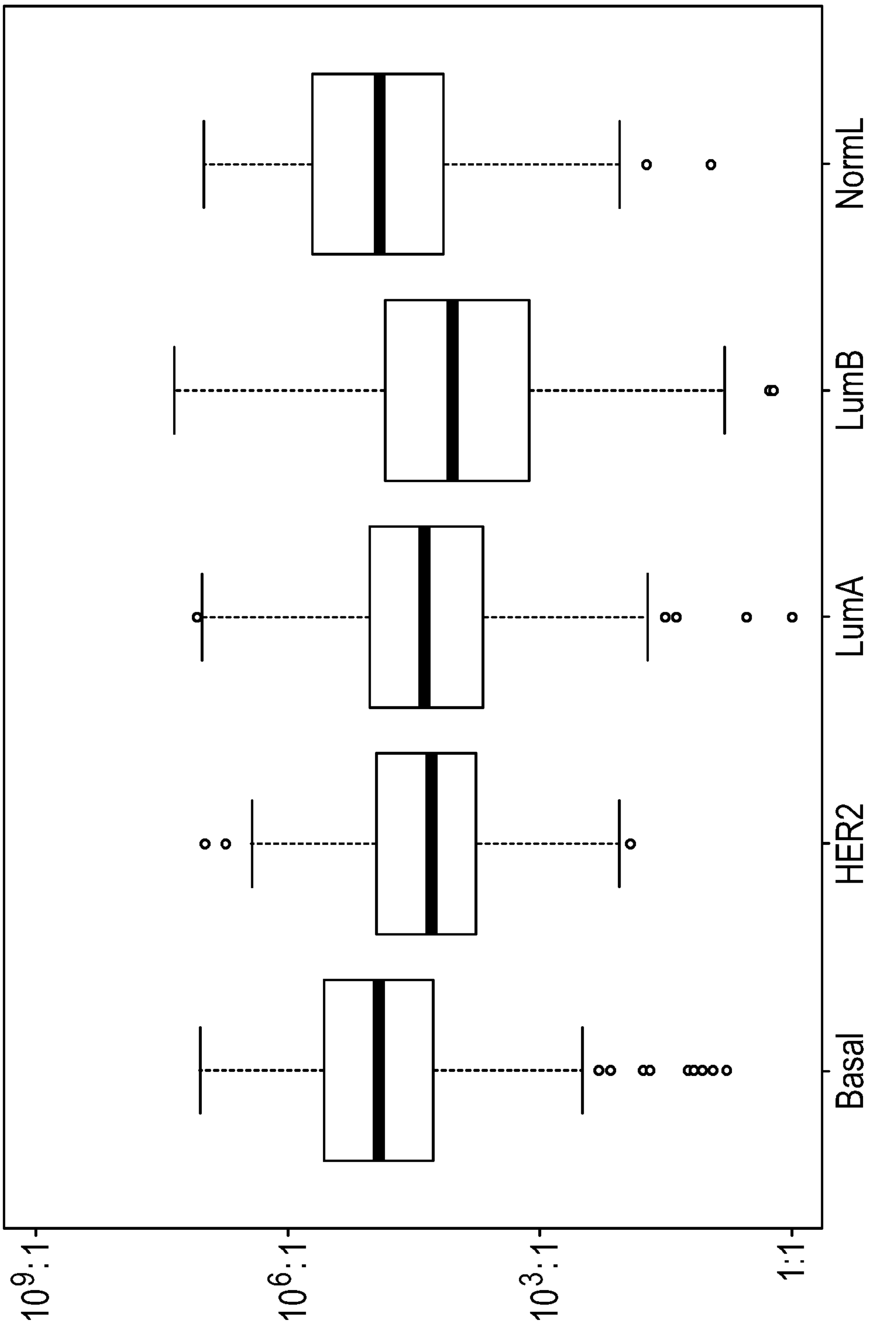


FIG. 18

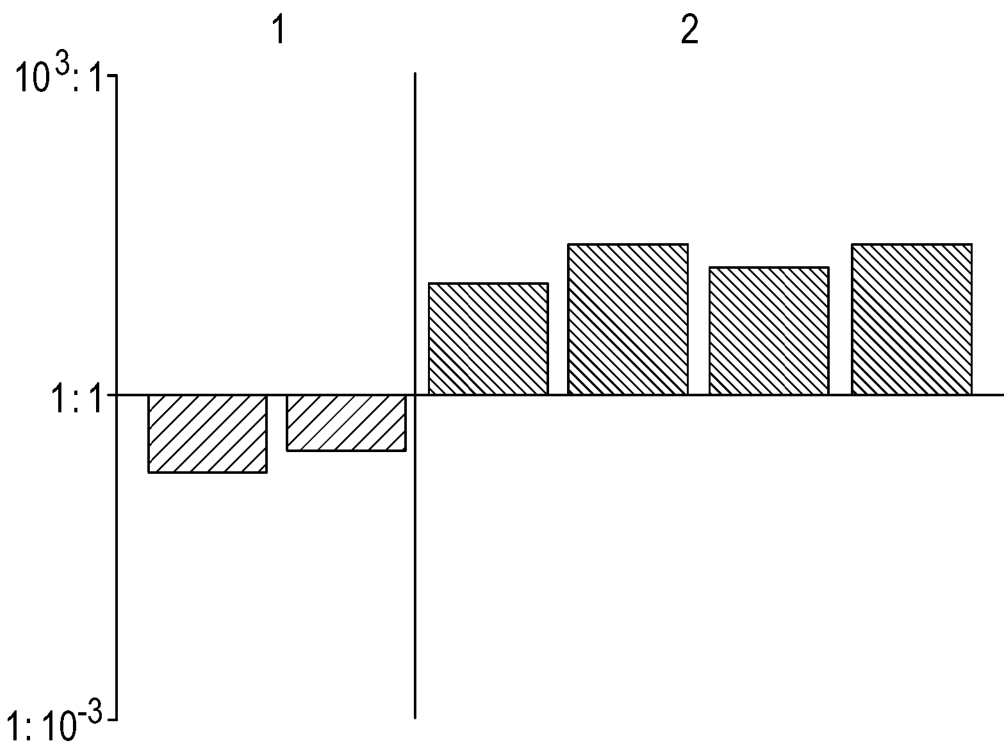


FIG. 19

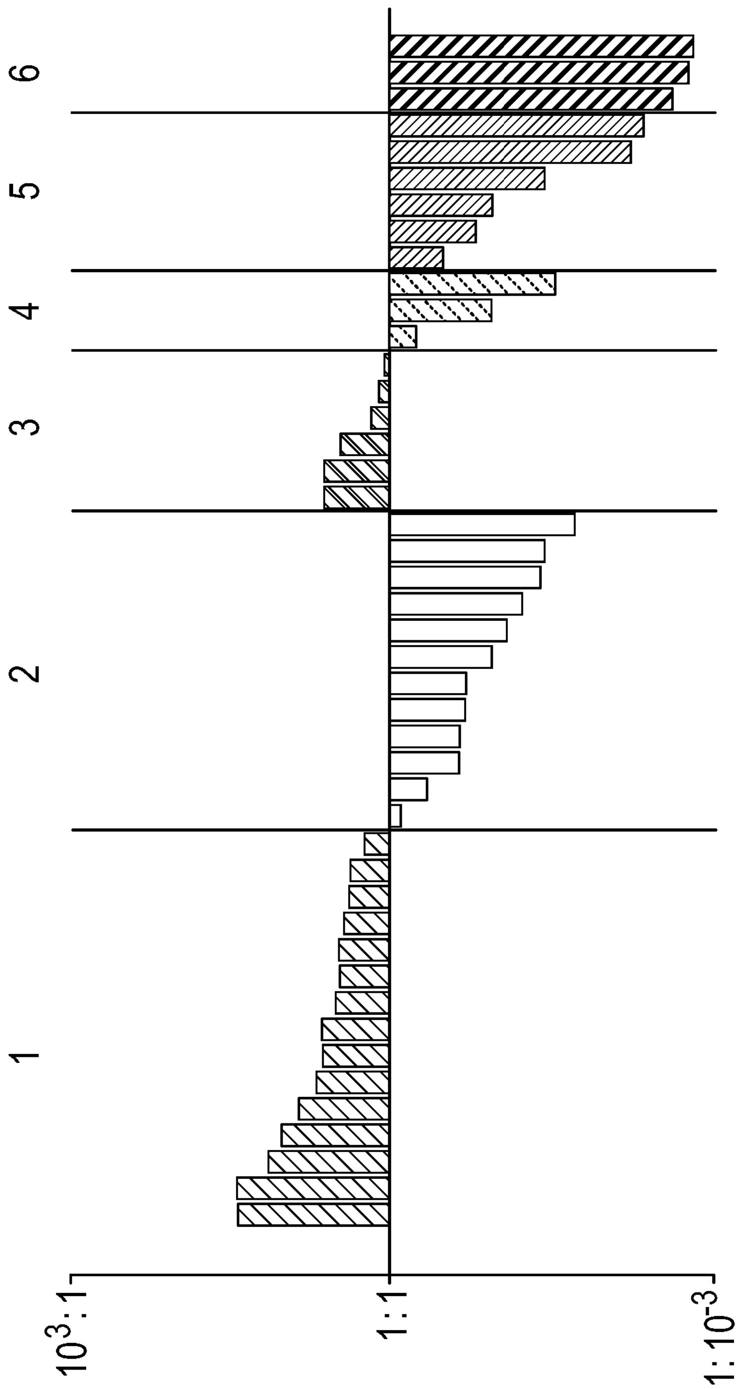


FIG. 20

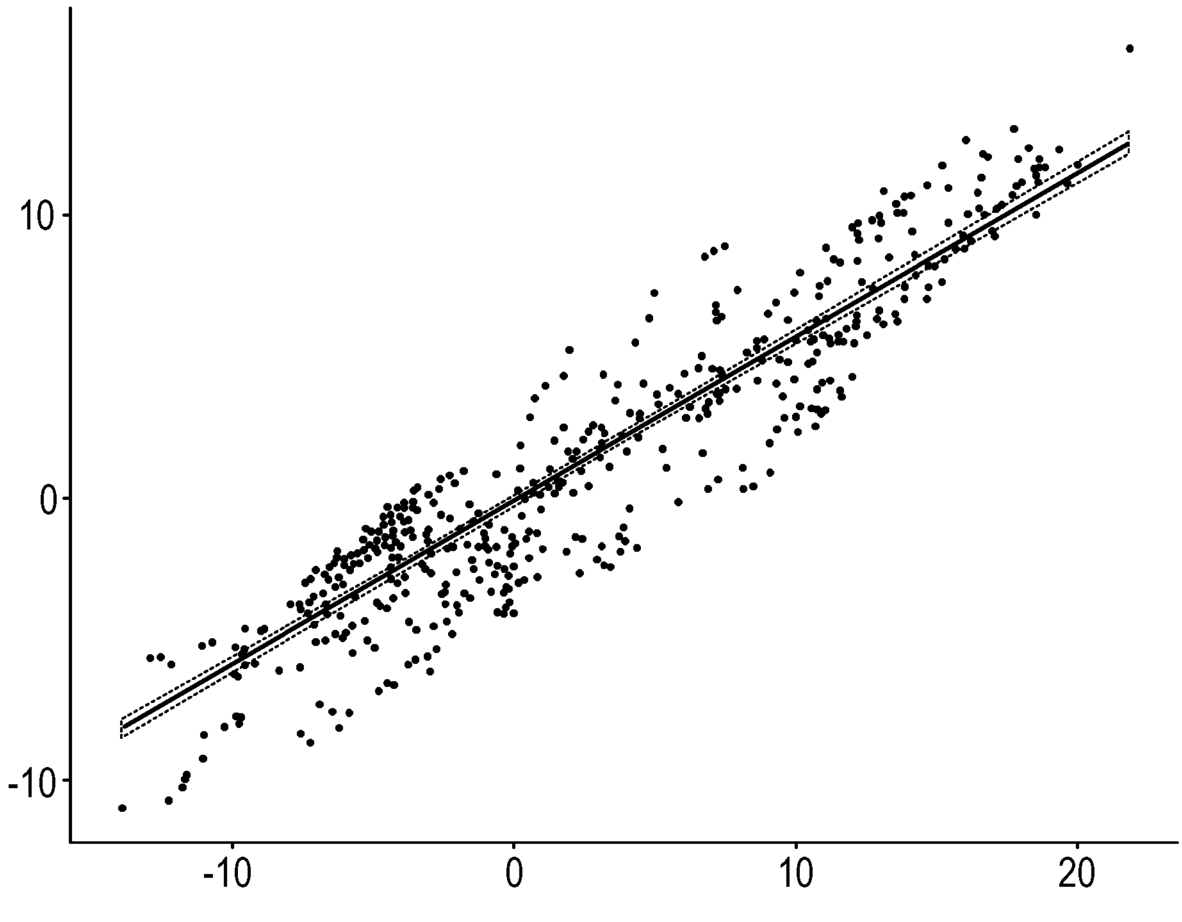


FIG. 21

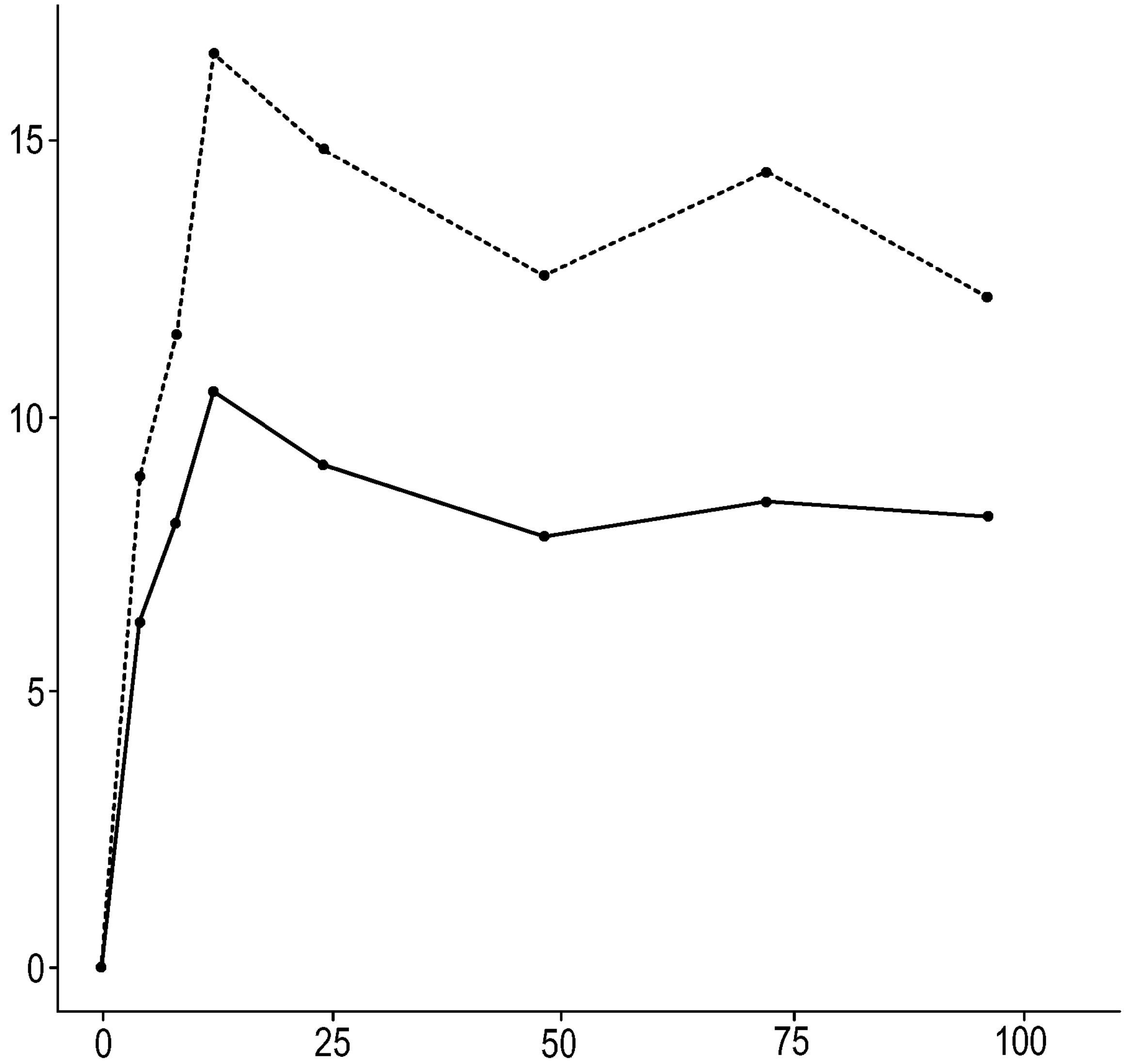


FIG. 22

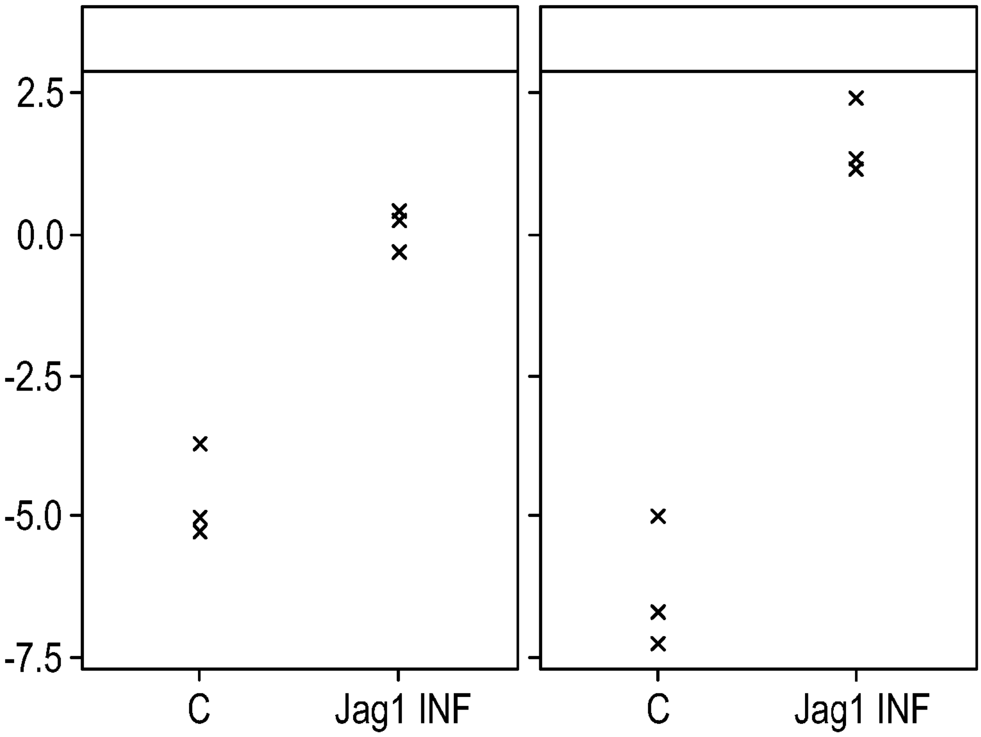


FIG. 23

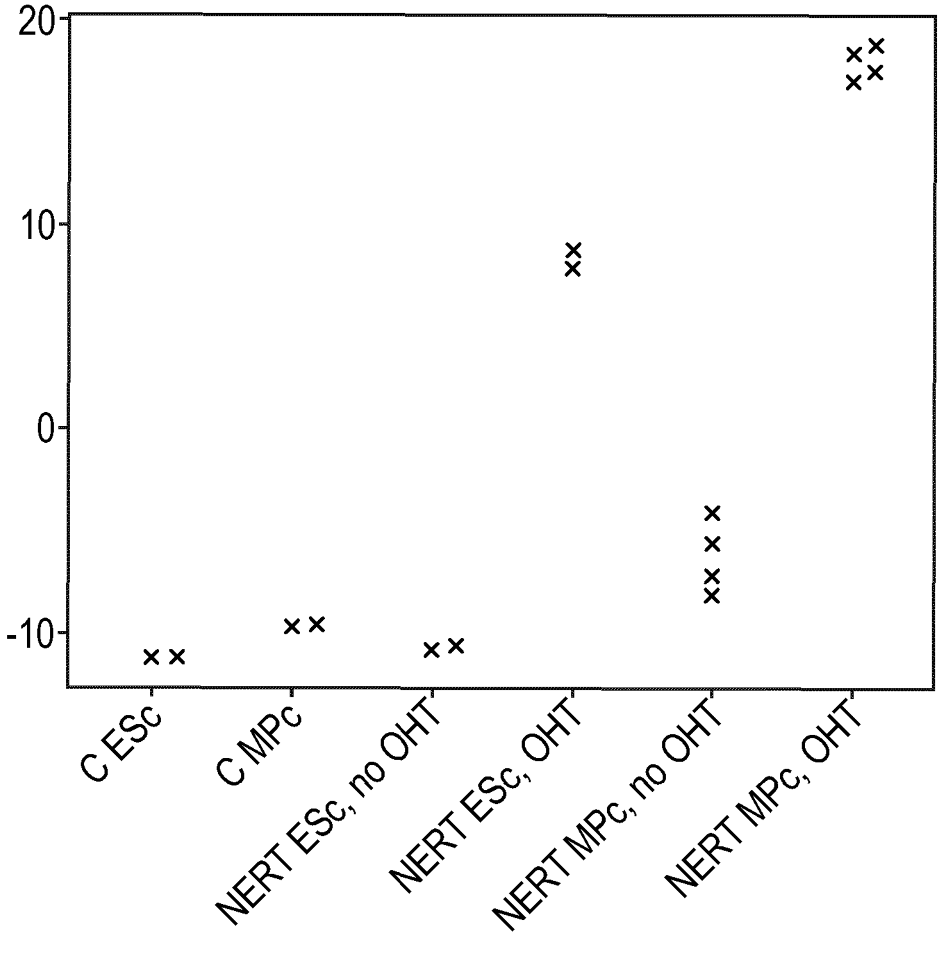
20
10
0
-10
C ESc
C MPc
NERT ESc, no OHT
NERT ESc, OHT
NERT MPc, no OHT
NERT MPc, OHT

FIG. 24

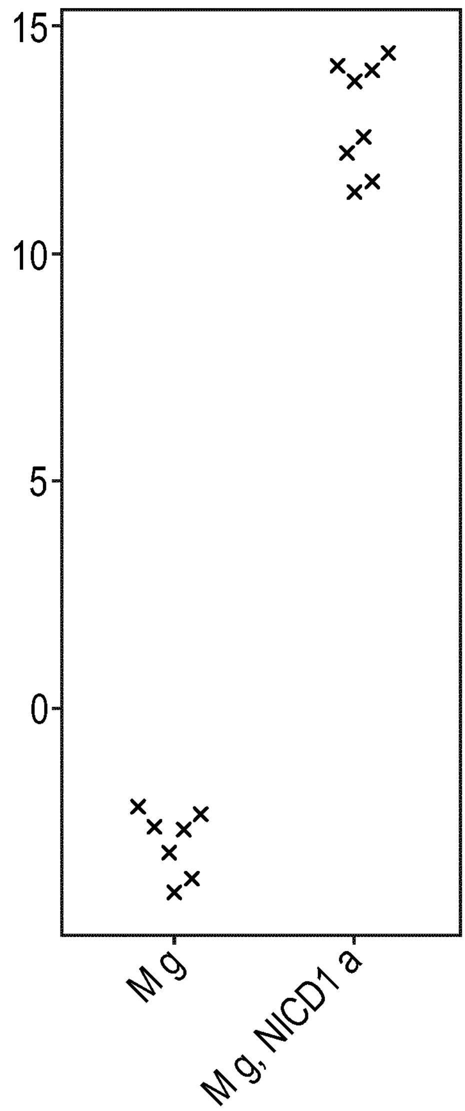


FIG. 25

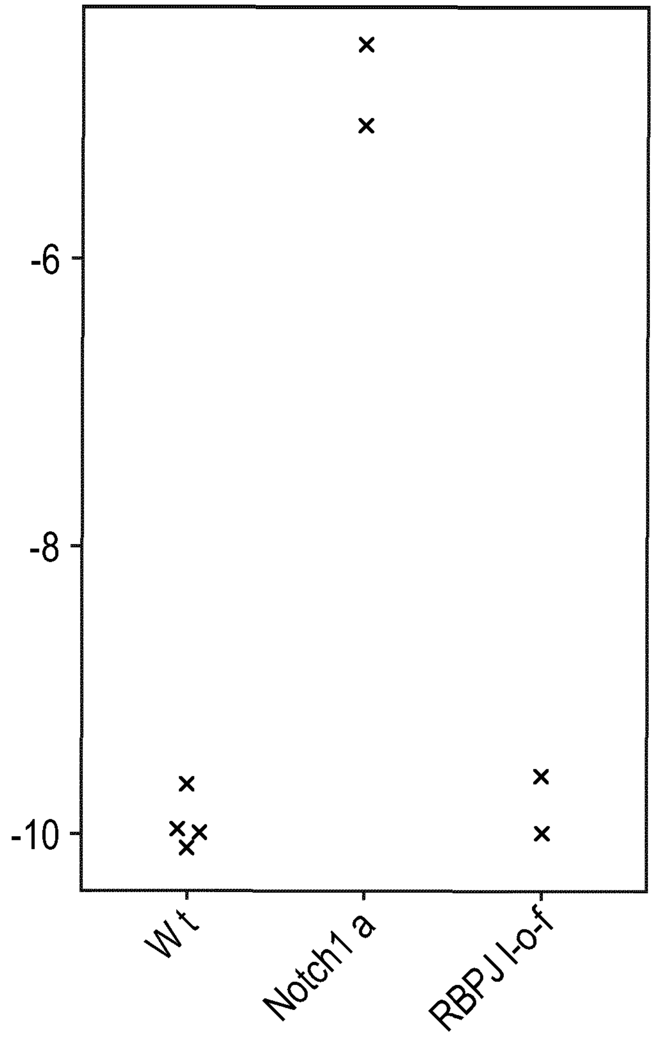


FIG. 26

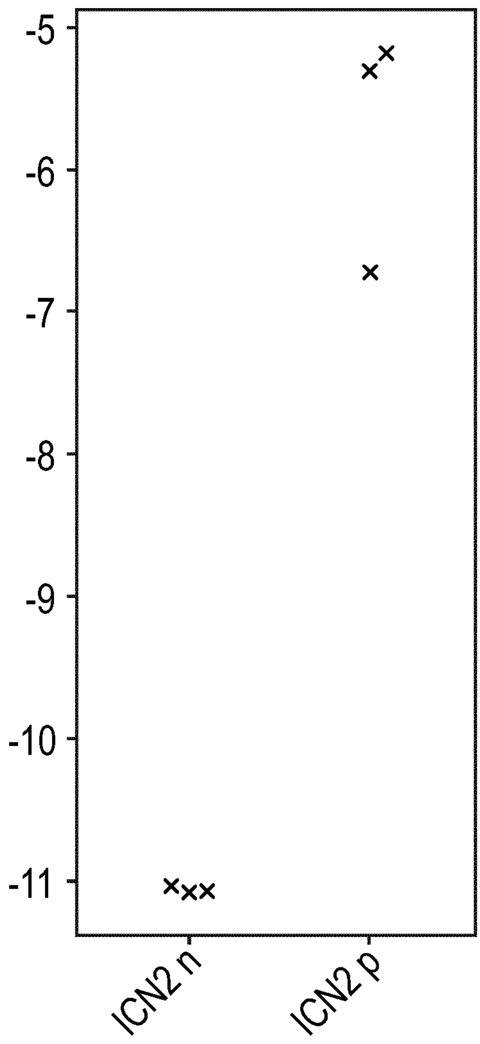


FIG. 27

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2016117439 A1
- WO 2013011479 A
- WO 2016062891 A
- US 7544476 B1
- KR 20160086775 A
- US 2016266095 A1
- US 2016125127 A1
- WO 2013011479 A2
- WO 2014102668 A2
- US 6713297 B
- US 5476928 A
- US 5958691 A
- US 5436134 A
- US 5658751 A


### Non-patent literature cited in the description


- **ASTER J.C. et al.** The varied roles of Notch in cancer. *Annual Review of Pathology,* 01 December 2016, vol. 12, 245-275
- **GURUHARSHA K.G. et al.** The Notch signaling system: recent insights into the complexity of a conserved pathway. *Nature Reviews Genetics,* 13 September 2012, 654-666
- **ESPINOZA I. ; MIELE L.** Notch inhibitors for cancer treatment. *Pharmacology & Therapeutics,* August 2013, vol. 139 (2), 95-110
- **S. S. RAO et al.** *CANCER RESEARCH,* 24 March 2009, vol. 69, 3060-3068
- **WEIWEI LIU et al.** *THE SCIENTIFIC WORLD JOURNAL,* 01 January 2013, vol. 2013, 1-6
- **VERHAEGH W. et al.** Selection of personalized patient therapy through the use of knowledge-based computational models that identify tumor-driving signal transduction pathways. *Cancer Research,* 2014, vol. 74 (11), 2936-2945
- **GURUHARSHA K.G. et al.** The Notch signaling system: recent insights into the complexity of a conserved pathway. *Nature Reviews Genetics,* September 2012, vol. 13, 654-666
- **MEIER-STIEGEN F. et al.** Activated Notch1 target genes during embryonic cell differentiation depend on the cellular context and include lineage determinants and inhibitors. *PLoS One,* July 2010, vol. 5 (7
- **DOHDA T. et al.** Notch signaling induces SKP2 expression and promotes reduction of p27Kip1 in T-cell acute lymphoblastic leukemia cell. *Experimental Cell Research,* August 2007, vol. 313 (14), 3141-3152
- **VAN NES J. et al.** A NOTCH3 Transcriptional Module Induces Cell Motility in Neuroblastoma. *Clinical Cancer Research,* July 2013, vol. 19 (13), 3485-3494
- **WANG H. et al.** Genome-wide analysis reveals conserved and divergent features of Notch1/RBPJ binding in human and murine T-lymphoblastic leukemia cells. *Proceedings of the National Academy of Sciences of the USA,* 2011, vol. 108 (36), 14908-14913
- **WANG H. et al.** Genome-wide analysis reveals conserved and divergent features of Notch1/RBPJ binding in human and murine T-lymphoblastic leukemia cells. *Proceedings of the National Academy of Sciences of the USA,* 2011, vol. 108 (36), 14908-14913
- **YOU L.R. et al.** *Suppression of Notch signaling by the COUP-TFII transcription factor regulates vein identity,* May 2005, vol. 435 (7038), 98-104
- **CHEN X. et al.** COUP-TFII is a major regulator of cell cycle and Notch signaling pathways. *Molecular Endocrinology,* August 2012, vol. 26 (8), 1268-1277
- **ZHONG Y. et al.** NOTCH1 is a Poor Prognostic Factor for Breast Cancer and Is Associated With Breast Cancer Stem Cells. *Oncotargets and Therapy,* November 2016, vol. 9, 6865-6871
- **MIKHAILIK A. et al.** Notch ligand-dependent gene expression in human endometrial stromal cells. *Biochemical and Biophysical Research Communications,* October 2009, vol. 388 (3), 479-482
- **STIEGEN F. et al.** Activated Notch1 Target Genes during Embryonic Cell Differentiation Depend on the Cellular Context and Include Lineage Determinants and Inhibitors. *PLoS One,* July 2010, vol. 5 (7

- **ABRAVANEL D.L. et al.** Notch promotes recurrence of dormant tumor cells following HER2/neu-targeted therapy. *Journal of Clinical Investigation,* June 2015, vol. 125 (6), 2484-2496
- **COPELAND J.N. et al.** Notch signaling regulates remodeling and vessel diameter in the extraembryonic yolk sac. *BMC Developmental Biology,* February 2011
- **OH P. et al.** In vivo mapping of notch pathway activity in normal and stress hematopoiesis. *Cell Stem Cell,* August 2013, vol. 13 (1), 190-204